(19) Europäisches Patentamt
European Patent Office
Office européen des brevets

(11) EP 2 988 732 B1

# (12) EUROPEAN PATENT SPECIFICATION

(45) Date of publication and mention of the grant of the patent:
**03.05.2023 Bulletin 2023/18**

(21) Application number: **14721238.5**

(22) Date of filing: **16.04.2014**

(51) International Patent Classification (IPC):
***A61K 9/10*** (2006.01) ***A61K 47/10*** (2017.01)
***A61K 47/60*** (2017.01) ***C08G 83/00*** (2006.01)
***C08J 3/075*** (2006.01) ***A61K 38/20*** (2006.01)
***A61K 38/28*** (2006.01) ***A61K 47/69*** (2017.01)

(52) Cooperative Patent Classification (CPC):
**C08J 3/075; A61K 38/2006; A61K 38/28; A61K 47/60; A61K 47/6903;** A61K 9/06; A61K 47/10

(86) International application number:
**PCT/EP2014/057760**

(87) International publication number:
**WO 2014/173762 (30.10.2014 Gazette 2014/44)**

## (54) MODIFIED HYDROGELS

MODIFIZIERTE HYDROGELE

HYDROGELS MODIFIÉS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.04.2013 EP 13164674**
**08.10.2013 EP 13187779**

(43) Date of publication of application:
**02.03.2016 Bulletin 2016/09**

(73) Proprietor: **Ascendis Pharma A/S**
**2900 Hellerup (DK)**

(72) Inventors:
- **HERSEL, Ulrich**
  **69120 Heidelberg (DE)**
- **RAU, Harald**
  **69120 Heidelberg (DE)**
- **LAUFER, Burkhardt**
  **69120 Heiderberg (DE)**
- **ZETTLER, Joachim**
  **69120 Heidelberg (DE)**
- **REIMANN, Romy**
  **69120 Heidelberg (DE)**

(74) Representative: **Büchel, Edwin**
**Patentanwälte**
**Isenbruck Bösl Hörschler PartG mbB**
**Eastsite One**
**Seckenheimer Landstraße 4**
**68163 Mannheim (DE)**

(56) References cited:
**WO-A1-2011/012715 WO-A1-2011/089215**
**WO-A1-2013/024053 WO-A1-2014/056915**
**WO-A1-2014/056923 WO-A2-2006/003014**
**US-A- 4 338 419**

- **SHI-BO ZHANG ET AL: "Poly( N -isopropylacrylamide)-based comb-type grafted hydrogel with rapid response to blood glucose concentration change at physiological temperature", POLYMERS FOR ADVANCED TECHNOLOGIES, vol. 19, no. 8, 1 August 2008 (2008-08-01) , pages 937-943, XP055074305, ISSN: 1042-7147, DOI: 10.1002/pat.1079**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### Field of the Invention

The present invention relates to a process for the preparation of a hydrogel suitable as a carrier in a hydrogel-linked prodrug, to hydrogels obtainable from said process, the use of such hydrogel as a carrier in a hydrogel-linked prodrug and to hydrogel-linked prodrugs comprising a covalently conjugated hydrogel of the present invention.

### Background of the Invention

Hydrogels are versatile carriers for carrier-linked prodrugs, see for example WO2006003014A2 and WO2011012715A1. As most of the drugs are connected to the inside of the hydrogel, they are protected from modifying and/or degrading enzymes present in a patient's body which extends the time period over which active drugs are released from such prodrugs.

However, part of the drug load of a hydrogel carrier is also connected to the outside of the hydrogel carrier, which in selected cases may potentially be disadvantageous. One disadvantage may be that drug molecules attached to the outside of the hydrogel may be exposed to modifying and/or degrading enzymes present in a patient's body upon administration of the hydrogel-linked prodrug to a patient.

Another disadvantage may be that drugs attached to the outside of the hydrogel may potentially have a certain level of residual activity or immunogenicity which in rare cases may cause undesired effects, such as immune reactions and/or inflammations.

### Detailed Description of the Invention

Accordingly, there is a need for hydrogel prodrug carriers which at least partially overcome the above shortcomings.

It is therefore an object of the present invention to overcome at least some of the above-mentioned shortcomings and to provide hydrogel prodrug carriers which have a reduced drug loading on the outside of the hydrogel carrier. This is achieved by reducing the number of functional groups of the hydrogel, in particular those at its surface.

In one aspect, the present invention relates to a process for the preparation of a hydrogel suitable as a carrier in a hydrogel-linked prodrug comprising the steps of

(a) providing a hydrogel having groups $A^{x0}$, wherein the groups $A^{x0}$ represent the same or different functional groups;

(b) optionally covalently conjugating a spacer reagent of formula (I)

$$A^{x1}\text{-}SP^2\text{-}A^{x2} \qquad \text{(I)},$$

wherein

$SP^2$ is $C_{1-50}$ alkyl, $C_{2-50}$ alkenyl or $C_{2-50}$ alkynyl, which $C_{1-50}$ alkyl, $C_{2-50}$ alkenyl and $C_{2-50}$ alkynyl is optionally interrupted by one or more group(s) selected from the group consisting of -NH-, -N($C_{1-4}$ alkyl)-, -O-, -S, -C(O)-, -C(O)NH, -C(O)N($C_{1-4}$ alkyl)-, -O-C(O)-, -S(O)-, -S(O)$_2$-, 4- to 7-membered heterocyclyl, phenyl and naphthyl;
$A^{x1}$ is a functional group for reaction with $A^{x0}$ of the hydrogel; and
$A^{x2}$ is a functional group;

to $A^{x0}$ of the hydrogel from step (a); and

(c) reacting the hydrogel of step (a) or step (b) with a reagent of formula (II)

$$A^{x3}\text{-}Z \qquad \text{(II)},$$

wherein

$A^{x3}$ is a functional group; and
Z is an inert linear or branched PEG-based polymer comprising at least 70% PEG, wherein Z has a molecular weight ranging from 10 kDa to 250 kDa, and does not react with $A^{x0}$ or $A^{x2}$ or with other functional groups present;

such that at most 80 mol-% of $A^{x0}$ or $A^{x2}$ react with $A^{x3}$.

It was now surprisingly found that such modified hydrogels have a reduced number of functional groups $A^{x0}$ available on the surface of the hydrogel compared to unmodified hydrogels and thus when such hydrogel is used as a carrier for a hydrogel-linked prodrug has fewer biologically active moieties attached to its surface.

Within the present invention the terms are used with the meaning as follows.

As used herein, the term "hydrogel" means a hydrophilic or amphiphilic polymeric network composed of homopolymers or copolymers, which is insoluble due to the presence of covalent chemical crosslinks. The crosslinks provide the network structure and physical integrity. Hydrogels exhibit a thermodynamic compatibility with water which allows them to swell in aqueous media.

As used herein, the term "reagent" means a chemical compound which comprises at least one functional group for reaction with the functional group of another reagent or moiety.

As used herein, the term "backbone reagent" means a reagent, which is suitable as a starting material for forming hydrogels. As used herein, a backbone reagent preferably does not comprise biodegradable linkages. A backbone reagent may comprise a "branching core" which refers to an atom or moiety to which more than one other moiety is attached.

As used herein, the term "crosslinker reagent" means a linear or branched reagent, which is suitable as a starting material for crosslinking backbone reagents. Preferably, the crosslinker reagent is a linear chemical compound. A crosslinker reagent comprises at least one biodegradable linkage.

As used herein, the term "moiety" means a part of a molecule, which lacks one or more atom(s) compared to the corresponding reagent. If, for example, a reagent of the formula "H-X-H" reacts with another reagent and becomes part of the reaction product, the corresponding moiety of the reaction product has the structure "H-X-" or "-X-", whereas each "-"indicates attachment to another moiety.

Accordingly, the phrase "in bound form" is used to refer to the corresponding moiety of a reagent, i.e. "lysine in bound form" refers to a lysine moiety which lacks one or more atom(s) of the lysine reagent and is part of a molecule.

The term "drug" means any substance which can affect one or more physical or biochemical properties of a biological organism, including viruses, bacteria, fungi, plants, animals, and humans. In particular, as used herein, the term includes any substance intended for diagnosis, cure, mitigation, treatment, or prevention of disease in organisms, in particular humans or animals, or to otherwise enhance physical or mental well-being of organisms, in particular humans or animals.

The term "biologically active moiety" refers to the moiety which results after covalently conjugating a drug to one or more other moieties wherein one or more functional groups of the drug were conjugated to functional groups of said one or more other moieties which subsequently form linkages.

The term "spacer moiety" as used herein refers to any moiety suitable for connecting two moieties and suitable spacer moieties are known to the person skilled in the art.

As used herein, the term "functional group" means a group of atoms which can react with other functional groups. Functional groups include the following groups: carboxylic acid (-(C=O)OH), primary or secondary amine ($-NH_2$, -NH-), maleimide, thiol (-SH), sulfonic acid (-(O=S=O)OH), carbonate, carbamate (-O(C=O)N<), hydroxy (-OH), aldehyde (-(C=O)H), ketone (-(C=O)-), hydrazine (>N-N<), isocyanate, isothiocyanate, phosphoric acid (-O(P=O)OHOH), phosphonic acid (-O(P=O)OHH), haloacetyl, alkyl halide, acryloyl, aryl fluoride, hydroxylamine, disulfide, vinyl sulfone, vinyl ketone, diazoalkane, oxirane, and aziridine.

As used herein, the term "activated functional group" means a functional group, which is connected to an activating group, i.e. a functional group was reacted with an activating reagent. Preferred activated functional groups include activated ester groups, activated carbamate groups, activated carbonate groups and activated thiocarbonate groups. Preferred activating groups are selected from the group consisting of formulas (f-i) to (f-vi):

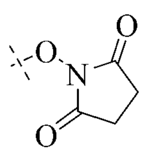 (f-i), 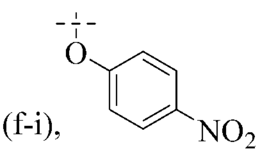 (f-ii), 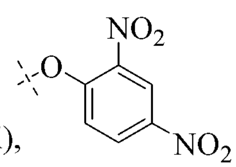 (f-iii),

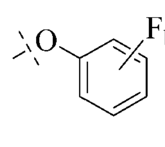 (f-iv), 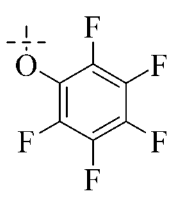 (f-v) and 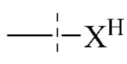 (f-vi),

wherein
the dashed lines indicate attachment to the rest of the molecule;

b is 1, 2, 3 or 4; and
$X^H$ is Cl, Br, I, or F.

Accordingly, a preferred activated ester has the formula

-(C=O)-$Y^1$,

wherein
$Y^1$ is selected from the group consisting of formulas (f-i), (f-ii), (f-iii), (f-iv), (f-v) and (f-vi).
Accordingly, a preferred activated carbamate has the formula

-N-(C=O)-$Y^1$,

wherein
$Y^1$ is selected from the group consisting of formulas (f-i), (f-ii), (f-iii), (f-iv), (f-v) and (f-vi).
Accordingly, a preferred activated carbonate has the formula

-O-(C=O)-$Y^1$,

wherein
$Y^1$ is selected from the group consisting of formulas (f-i), (f-ii), (f-iii), (f-iv), (f-v) and (f-vi).
Accordingly, a preferred activated thiocarbonate has the formula

-S-(C=O)-$Y^1$,

wherein
$Y^1$ is selected from the group consisting of formulas (f-i), (f-ii), (f-iii), (f-iv), (f-v) and (f-vi).
Accordingly, a "functional end group" is a functional group which is localized at the end of a moiety or molecule, i.e. is a terminal functional group.
If a chemical functional group is coupled to another functional group, the resulting chemical structure is referred to as "linkage". For example, the reaction of an amine group with a carboxyl group results in an amide linkage.
As used herein, the term "protecting group" means a moiety which is reversibly connected to a functional group to render it incapable of reacting with, for example, another functional group. Suitable alcohol (-OH) protecting groups are, for example, acetyl, benzoyl, benzyl, *β*-methoxyethoxymethyl ether, dimethoxytrityl, methoxymethyl ether, methoxytrityl, *p*-methoxybenzyl ether, methylthiomethyl ether, pivaloyl, tetrahydropyranyl, trityl, trimethylsilyl, *tert*-butyldimethylsilyl, tri-iso-propylsilyloxymethyl, triisopropylsilyl ether, methyl ether, and ethoxyethyl ether. Suitable amine protecting groups are, for example, carbobenzyloxy, *p*-methoxybenzyl carbonyl, tert-butyloxycarbonyl, 9-fluorenylmethyloxyarbonyl, acetyl, benzoyl, benzyl, carbamate, *p*-methoxybenzyl, 3,4-dimethoxybenzyl, *p*-methoxyphenyl, and tosyl. Suitable carbonyl protecting groups are, for example, acetals and ketals, acylals and dithianes. Suitable carboxylic acid protecting groups are, for example, methyl esters, benzyl esters, *tert*-butyl esters, 2,6-dimethylphenol, 2,6-diisopropylphenol, 2,6.-di-*tert*-butylphenol, silyl esters, orthoesters, and oxazoline. Suitable phosphate protecting groups are, for example, 2-cyanoethyl and methyl.
As used herein, the terms "work-up" and "working-up" refer to the series of manipulations useful and/or required to isolate and purify the product(s) of a chemical reaction, in particular of a polymerization.
As used herein, the term "polymer" means a molecule comprising repeating structural units, i.e. monomers, connected by chemical bonds in a linear, circular, branched, crosslinked or dendrimeric way or a combination thereof, which may be of synthetic or biological origin or a combination of both. It is understood that a polymer may for example also comprise functional groups. Preferably, a polymer has a molecular weight of at least 0.5 kDa, e.g. a molecular weight of at least 1 kDa, a molecular weight of at least 2 kDa, a molecular weight of at least 3 kDa or a molecular weight of at least 5 kDa. At most, a polymer has preferably a molecular weight of 1 million Da.
As used herein, the term "polymeric" means a reagent or a moiety comprising one or more polymer(s).
The person skilled in the art understands that the polymerization products obtained from a polymerization reaction do not all have the same molecular weight, but rather exhibit a molecular weight distribution. Consequently, the molecular weight ranges, molecular weights, ranges of numbers of monomers in a polymer and numbers of monomers in a polymer as used herein, refer to the number average molecular weight and number average of monomers. As used

herein, the term "number average molecular weight" means the ordinary arithmetic means of the molecular weights of the individual polymers.

As used herein, the term "polymerization" or "polymerizing" means the process of reacting monomer or macromonomer reagents in a chemical reaction to form polymer chains or networks, including hydrogels.

As used herein, the term "macromonomer" means a molecule that was obtained from the polymerization of monomer reagents.

As used herein, the term "condensation polymerization" or "condensation reaction" means a chemical reaction, in which the functional groups of two reagents react to form one single molecule, i.e. the reaction product, and a low molecular weight molecule, for example water, is released.

As used herein, the term "suspension polymerization" means a heterogeneous and/or biphasic polymerization reaction, wherein the monomer reagents are dissolved in a first solvent, forming the disperse phase which is emulsified in a second solvent, forming the continuous phase. In the present invention, the monomer reagents are the at least one backbone reagent and the at least one crosslinker reagent. Both the first solvent and the monomer reagents are not soluble in the second solvent. Such emulsion is formed by stirring, shaking, exposure to ultrasound or Microsieve™ emulsification, more preferably by stirring or Microsieve™ emulsification and more preferably by stirring. This emulsion is stabilized by an appropriate emulsifier. The polymerization is initiated by addition of a base as initiator which is soluble in the first solvent. A suitable commonly known base suitable as initiator may be a tertiary base, such as tetramethylethylenediamine (TMEDA).

As used herein, the term "inert" refers to a moiety which is not chemically reactive, i.e. it does not react with other moieties or reagents. The person skilled in the art understands that the term "inert" does not per se exclude the presence of functional groups, but understands that the functional groups potentially present in an inert moiety are not reactive with functional groups of moieties/reagents brought in contact with the inert moiety in, for example, subsequent reactions. In particular, the inert moiety Z does not react with $A^{x0}$ or $A^{x2}$ or with functional groups present, for example, in reversible prodrug linker reagents, drugs, reversible prodrug linker moiety-biologically active moiety conjugate reagents or spacer reagents which may be covalently conjugated to the hydrogel of the present invention to obtain the hydrogel-linked prodrug of the present invention.

As used herein, the term "immiscible" means the property where two substances are not capable of combining to form a homogeneous mixture at ambient temperature and pressure, i.e. at temperature and pressure conditions typically present in a typical laboratory environment.

As used herein, the term "polyamine" means a reagent or moiety comprising more than one amine (-NH- and/or $-NH_2$), e.g. from 2 to 64 amines, from 4 to 48 amines, from 6 to 32 amines, from 8 to 24 amines, or from 10 to 16 amines. Particularly preferred polyamines comprise from 2 to 32 amines.

As used herein, the term "PEG-based comprising at least X% PEG" in relation to a moiety or reagent means that said moiety or reagent comprises at least X% (w/w) ethylene glycol units ($-CH_2CH_2O-$), wherein the ethylene glycol units may be arranged blockwise, alternating or may be randomly distributed within the moiety or reagent and preferably all ethylene glycol units of said moiety or reagent are present in one block; the remaining weight percentage of the PEG-based moiety or reagent are other moieties especially selected from the group consisting of:

- $C_{1-50}$ alkyl, $C_{2-50}$ alkenyl, $C_{2-50}$ alkynyl, $C_{3-10}$ cycloalkyl, 4- to 7-membered heterocyclyl, 8- to 11-membered heterobicyclyl, phenyl; naphthyl; indenyl; indanyl; and tetralinyl; and
- linkages selected from the group of linkages consisting of

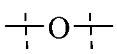, 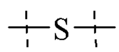, 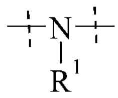, 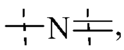, 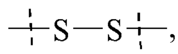, 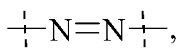,

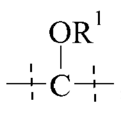, 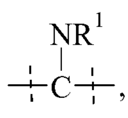, 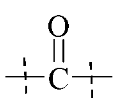, 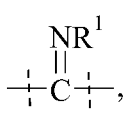, 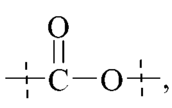, 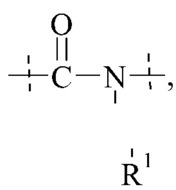,

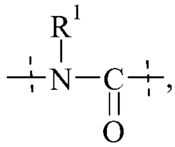, 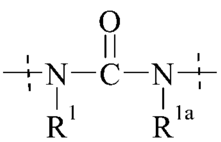, 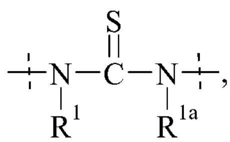, 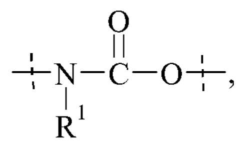,

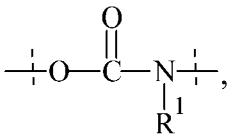, 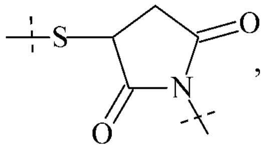, and 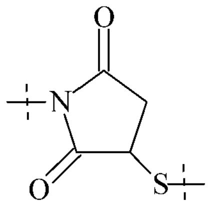,

wherein

dashed lines indicate attachment to the remainder of the moiety or reagent, and
$R^1$ and $R^{1a}$ are independently of each other H or $C_{1-6}$ alkyl.

The term "hyaluronic acid-based comprising at least X% hyaluronic acid" is used accordingly.

As used herein, the term "$C_{1-4}$ alkyl" alone or in combination means a straight-chain or branched alkyl group having 1 to 4 carbon atoms. If present at the end of a molecule, examples of straight-chain and branched $C_{1-4}$ alkyl groups are methyl, ethyl, n-propyl, isopropyl, n-butyl, isobutyl, sec-butyl and tert-butyl. When two moieties of a molecule are linked by the $C_{1-4}$ alkyl group, then examples for such $C_{1-4}$ alkyl groups are $-CH_2-$, $-CH_2-CH_2-$, $-CH(CH_3)-$, $-CH_2-CH_2-CH_2-$, $-CH(C_2H_5)-$, $-C(CH_3)_2-$, $-CH_2-CH_2-CH_2-CH_2-$, and $-CH_2-CH_2-CH_2(CH_3)-$. Each hydrogen atom of a $C_{1-4}$ alkyl group may be replaced by a substituent as defined below.

As used herein, the term "$C_{1-6}$ alkyl" alone or in combination means a straight-chain or branched alkyl group having 1 to 6 carbon atoms. If present at the end of a molecule, examples of straight-chain and branched $C_{1-6}$ alkyl groups are methyl, ethyl, n-propyl, isopropyl, n-butyl, isobutyl, sec-butyl, tert-butyl, n-pentyl, 2-methylbutyl, 2,2-dimethylpropyl, n-hexyl, 2-methylpentyl, 3-methylpentyl, 2,2-dimethylbutyl, 2,3-dimethylbutyl and 3,3-dimethylpropyl. When two moieties of a molecule are linked by the $C_{1-6}$ alkyl group, then examples for such $C_{1-6}$ alkyl groups are $-CH_2-$, $-CH_2-CH_2-$, $-CH(CH_3)-$, $-CH_2-CH_2-CH_2-$, $-CH(C_2H_5)-$ and $-C(CH_3)_2-$. Each hydrogen atom of a $C_{1-6}$ alkyl group may be replaced by a substituent as defined below.

Accordingly, as used herein, the term "$C_{1-20}$ alkyl" alone or in combination means a straight-chain or branched alkyl group having 1 to 20 carbon atoms. The term "$C_{8-18}$ alkyl" alone or in combination means a straight-chain or branched alkyl group having 8 to 18 carbon atoms. Accordingly, as used herein, the term "$C_{1-50}$ alkyl" alone or in combination means a straight-chain or branched alkyl group having 1 to 50 carbon atoms. Each hydrogen atom of a $C_{1-20}$ alkyl group, a $C_{8-18}$ alkyl group and $C_{1-50}$ alkyl group may be replaced by a substituent. In each case the alkyl group may be present at the end of a molecule or two moieties of a molecule may be linked by the alkyl group.

As used herein, the term "$C_{2-6}$ alkenyl" alone or in combination means a straight-chain or branched hydrocarbon moiety comprising at least one carbon-carbon double bond having 2 to 6 carbon atoms. If present at the end of a molecule, examples are $-CH=CH_2$, $-CH=CH-CH_3$, $-CH_2-CH=CH_2$, $-CH=CHCH_2-CH_3$ and $-CH=CH-CH=CH_2$. When two moieties of a molecule are linked by the $C_{2-6}$ alkenyl group, then an example for such $C_{2-6}$ alkenyl is -CH=CH-. Each hydrogen atom of a $C_{2-6}$ alkenyl group may be replaced by a substituent as defined below. Optionally, one or more triple bond(s) may occur.

Accordingly, as used herein, the term "$C_{2-20}$ alkenyl" alone or in combination means a straight-chain or branched hydrocarbon residue comprising at least one carbon-carbon double bond having 2 to 20 carbon atoms. The term "$C_{2-50}$ alkenyl" alone or in combination means a straight-chain or branched hydrocarbon residue comprising at least one carbon-carbon double bond having 2 to 50 carbon atoms. If present at the end of a molecule, examples are $-CH=CH_2$, $-CH=CH-CH_3$, $-CH_2-CH=CH_2$, $-CH=CHCH_2-CH_3$ and $-CH=CH-CH=CH_2$. When two moieties of a molecule are linked by the alkenyl group, then an example is e.g. -CH=CH-.

Each hydrogen atom of a $C_{2-20}$ alkenyl or $C_{2-50}$ alkenyl group may be replaced by a substituent as defined below. Optionally, one or more triple bond(s) may occur.

As used herein, the term "$C_{2-6}$ alkynyl" alone or in combination means straight-chain or branched hydrocarbon residue comprising at least one carbon-carbon triple bond having 2 to 6 carbon atoms. If present at the end of a molecule, examples are -C=CH, $-CH_2-C\equiv CH$, $CH_2-CH_2-C\equiv CH$ and $CH_2-C\equiv C-CH_3$. When two moieties of a molecule are linked by the alkynyl group, then an example is: -C=C-. Each hydrogen atom of a $C_{2-6}$ alkynyl group may be replaced by a substituent as defined below. Optionally, one or more double bond(s) may occur.

Accordingly, as used herein, the term "$C_{2-20}$ alkynyl" alone or in combination means a straight-chain or branched hydrocarbon residue comprising at least one carbon-carbon triple bond having 2 to 20 carbon atoms and "$C_{2-50}$ alkynyl" alone or in combination means a straight-chain or branched hydrocarbon residue comprising at least one carbon-carbon triple bond having 2 to 50 carbon atoms. If present at the end of a molecule, examples are -C=CH, $-CH_2-C\equiv CH$, $CH_2-CH_2-C\equiv CH$ and $CH_2-C\equiv C-CH_3$. When two moieties of a molecule are linked by the alkynyl group, then an example is -C=C-. Each hydrogen atom of a $C_{2-20}$ alkynyl or $C_{2-50}$ alkynyl group may be replaced by a substituent as defined

below. Optionally, one or more double bond(s) may occur.

As used herein, the terms "$C_{3-8}$ cycloalkyl" or "$C_{3-8}$ cycloalkyl ring" means a cyclic alkyl chain having 3 to 8 carbon atoms, which may be saturated or unsaturated, e.g. cyclopropyl, cyclobutyl, cyclopentyl, cyclohexyl, cyclohexenyl, cycloheptyl, cyclooctyl. Each hydrogen atom of a cycloalkyl carbon may be replaced by a substituent as defined below. The term "$C_{3-8}$ cycloalkyl" or "$C_{3-8}$ cycloalkyl ring" also includes bridged bicycles like norbonane or norbonene. Accordingly, "$C_{3-5}$ cycloalkyl" means a cycloalkyl having 3 to 5 carbon atoms and $C_{3-10}$ cycloalkyl having 3 to 10 carbon atoms.

Accordingly, as used herein, the term "$C_{3-10}$ cycloalkyl" means a carbocyclic ring system having 3 to 10 carbon atoms, which may be saturated or unsaturated, e.g. cyclopropyl, cyclobutyl, cyclopentyl, cyclohexyl, cyclohexenyl, cycloheptyl, cyclooctyl, cyclononyl, cyclodecyl. The term "$C_{3-10}$ cycloalkyl" also includes at least partially saturated carbomono- and -bicycles.

As used herein, the term "halogen" means fluoro, chloro, bromo or iodo. Particulary preferred is fluoro or chloro.

As used herein, the term "4- to 7-membered heterocyclyl" or "4- to 7-membered heterocycle" means a ring with 4, 5, 6 or 7 ring atoms that may contain up to the maximum number of double bonds (aromatic or non-aromatic ring which is fully, partially or un-saturated) wherein at least one ring atom up to 4 ring atoms are replaced by a heteroatom selected from the group consisting of sulfur (including $-S(O)-$, $-S(O)_2-$), oxygen and nitrogen (including $=N(O)-$) and wherein the ring is linked to the rest of the molecule via a carbon or nitrogen atom. Examples for 4- to 7-membered heterocycles include azetidine, oxetane, thietane, furan, thiophene, pyrrole, pyrroline, imidazole, imidazoline, pyrazole, pyrazoline, oxazole, oxazoline, isoxazole, isoxazoline, thiazole, thiazoline, isothiazole, isothiazoline, thiadiazole, thiadiazoline, tetrahydrofuran, tetrahydrothiophene, pyrrolidine, imidazolidine, pyrazolidine, oxazolidine, isoxazolidine, thiazolidine, isothiazolidine, thiadiazolidine, sulfolane, pyran, dihydropyran, tetrahydropyran, imidazolidine, pyridine, pyridazine, pyrazine, pyrimidine, piperazine, piperidine, morpholine, tetrazole, triazole, triazolidine, tetrazolidine, diazepane, azepine and homopiperazine. Each hydrogen atom of a 4- to 7-membered heterocyclyl or 4- to 7-membered heterocyclic group may be replaced by a substituent as defined below.

As used herein, the term "8- to 11-membered heterobicyclyl" or "8- to 11-membered heterobicycle" means a heterocyclic system of two rings with 8 to 11 ring atoms, where at least one ring atom is shared by both rings and that may contain up to the maximum number of double bonds (aromatic or non-aromatic ring which is fully, partially or unsaturated) wherein at least one ring atom up to 6 ring atoms are replaced by a heteroatom selected from the group consisting of sulfur (including $-S(O)-$, $-S(O)_2-$), oxygen and nitrogen (including $=N(O)-$) and wherein the ring is linked to the rest of the molecule via a carbon or nitrogen atom. Examples for a 8- to 11-membered heterobicycle are indole, indoline, benzofuran, benzothiophene, benzoxazole, benzisoxazole, benzothiazole, benzisothiazole, benzimidazole, benzimidazoline, quinoline, quinazoline, dihydroquinazoline, quinoline, dihydroquinoline, tetrahydroquinoline, decahydroquinoline, isoquinoline, decahydroisoquinoline, tetrahydroisoquinoline, dihydroisoquinoline, benzazepine, purine and pteridine. The term 8-to 11-membered heterobicycle also includes spiro structures of two rings like 1,4-dioxa-8-azaspiro[4.5]decane or bridged heterocycles like 8-aza-bicyclo[3.2.1]octane. Each hydrogen atom of an 8- to 11-membered heterobicyclyl or 8- to 11-membered heterobicycle carbon may be replaced by a substituent as defined below.

The term "substituted" means that one or more -H atom(s) of a molecule or moiety are replaced by a different atom or a group of atoms, which are referred to as "substituent". Suitable substituents are halogen; CN; $COOR^9$; $OR^9$; $C(O)R^9$; $C(O)N(R^9R^{9a})$; $S(O)_2N(R^9R^{9a})$, $S(O)N(R^9R^{9a})$; $S(O)_2R^9$; $S(O)R^9$; $N(R^9)S(O)_2N(R^{9a}R^{9b})$; $SR^9$; $N(R^9R^{9a})$; $NO_2$; $OC(O)R^9$; $N(R^9)C(O)R^{9a}$; $N(R^9)S(O)_2R^{9a}$; $N(R^9)S(O)R^{9a}$; $N(R^9)C(O)OR^{9a}$; $N(R^9)C(O)N(R^{9a}R^{9b})$; $OC(O)N(R^9R^{9a})$; T; $C_{1-50}$ alkyl; $C_{2-50}$ alkenyl; or $C_{2-50}$ alkynyl, which T; $C_{1-50}$ alkyl; $C_{2-50}$ alkenyl; and $C_{2-50}$ alkynyl are optionally substituted with one or more $R^{10}$, which are the same or different and wherein $C_{1-50}$ alkyl; $C_{2-50}$ alkenyl; and $C_{2-50}$ alkynyl are optionally interrupted by one or more group(s) selected from the group consisting of T, $-C(O)O-$; $-O-$; $-C(O)-$; $-C(O)N(R^{11})-$; $-S(O)_2N(R^{11})-$; $-S(O)N(R^{11})-$; $-S(O)_2-$; $-S(O)-$; $-N(R^{11})S(O)_2N(R^{11a})-$; $-S-$; $-N(R^{11})-$; $-OC(O)R^{11}$; $-N(R^{11})C(O)-$; $-N(R^{11})S(O)_2-$; $-N(R^{11})S(O)-$; $-N(R^{11})C(O)O-$; $-N(R^{11})C(O)N(R^{11a})-$; and $-OC(O)N(R^{11}R^{11a})$;

wherein

| | |
|---|---|
| $R^9$, $R^{9a}$, $R^{9b}$ | are independently selected from the group consisting of H; T; $C_{1-50}$ alkyl; $C_{2-50}$ alkenyl; and $C_{2-50}$ alkynyl, which T; $C_{1-50}$ alkyl; $C_{2-50}$ alkenyl; and $C_{2-50}$ alkynyl are optionally substituted with one or more $R^{10}$, which are the same or different and which $C_{1-50}$ alkyl; $C_{2-50}$ alkenyl; and $C_{2-50}$ alkynyl are optionally interrupted by one or more group(s) selected from the group consisting of T, $-C(O)O-$; $-O-$; $-C(O)-$; $-C(O)N(R^{11})-$; $-S(O)_2N(R^{11})-$; $-S(O)N(R^{11})-$; $-S(O)_2-$; $-S(O)-$; $-N(R^{11})S(O)_2N(R^{11a})-$; $-S-$; $-N(R^{11})-$; $-OC(O)R^{11}$; $-N(R^{11})C(O)-$; $-N(R^{11})S(O)_2-$; $-N(R^{11})S(O)-$; $-N(R^{11})C(O)O-$; $-N(R^{11})C(O)N(R^{11a})-$; and $-OC(O)N(R^{11}R^{11a})$; |
| T | is selected from the group consisting of phenyl; naphthyl; indenyl; indanyl; tetralinyl; $C_{3-10}$ cycloalkyl; 4- to 7-membered heterocyclyl; and 8- to 11- membered heterobicyclyl, wherein T is optionally substituted with one or more $R^{10}$, which are the same or different; |
| $R^{10}$ | is halogen; CN; oxo (=O); $COOR^{12}$; $OR^{12}$; $C(O)R^{12}$; $C(O)N(R^{12}R^{12a})$; $S(O)_2N(R^{12}R^{12a})$; |

$S(O)N(R^{12}R^{12a})$; $S(O)_2R^{12}$; $S(O)R^{12}$; $N(R^{12})S(O)_2N(R^{12a}R^{12b})$; $SR^{12}$; $N(R^{12}R^{12a})$; $NO_2$; $OC(O)R^{12}$; $N(R^{12})C(O)R^{12a}$; $N(R^{12})S(O)_2R^{12a}$; $N(R^{12})S(O)R^{12a}$; $N(R^{12})C(O)OR^{12a}$; $N(R^{12})C(O)N(R^{12a}R^{12b})$; $OC(O)N(R^{12}R^{12a})$; or $C_{1-6}$ alkyl, which $C_{1-6}$ alkyl is optionally substituted with one or more halogen, which are the same or different;

$R^{11}$, $R^{11a}$, $R^{12}$, $R^{12a}$, $R^{12b}$ are independently of each other selected from the group consisting of H; and $C_{1-6}$ alkyl, which $C_{1-6}$ alkyl is optionally substituted with one or more halogen, which are the same or different.

In one embodiment $R^9$, $R^{9a}$, $R^{9b}$ are independently of each other H.

In one embodiment $R^{10}$ is $C_{1-6}$ alkyl.

In one embodiment T is phenyl.

Preferably, a maximum of 6 -H atoms of a moiety or molecule are independently replaced by a substituent, e.g. 5 -H atoms are independently replaced by a substiuent, 4 -H atoms are independently replaced by a substituent, 3 -H atoms are independently replaced by a substituent, 2 -H atoms are independently replaced by a substituent, or 1 -H atom is replaced by a substituent.

As used herein, the term "interrupted" means that between two carbon atoms or at the end of a carbon chain between the respective carbon atom and the hydrogen atom one or more atom(s) are inserted.

As used herein, the term "prodrug" means a compound that undergoes biotransformation before exhibiting its pharmacological effects. Prodrugs can thus be viewed as biologically active moieties connected to specialized non-toxic protective groups used in a transient manner to alter or to eliminate undesirable properties in the parent molecule. This also includes the enhancement of desirable properties in the drug and the suppression of undesirable properties.

As used herein, the term "carrier-linked prodrug" means a prodrug that contains a reversible linkage of a biologically active moiety with a carrier group and which carrier improves the physicochemical or pharmacokinetic properties of the biologically active moiety and which carrier is removed *in vivo,* usually by a hydrolytic cleavage. Preferably, the carrier is a polymer.

Accordingly, the term "hydrogel-linked prodrug" refers to a carrier-linked prodrug in which the carrier is a hydrogel.

In order for a hydrogel to be "suitable as a carrier in a hydrogel-linked prodrug" such hydrogel needs functional groups for conjugating reversible prodrug linker reagents or reversible prodrug linker moiety-biologically active moiety conjugate reagents to said hydrogel.

As used herein, the term "reversible prodrug linker" means a moiety which on its one end is attached to a backbone moiety of the hydrogel either directly or through a spacer moiety and on another end is attached to a biologically active moiety through a reversible linkage.

A "biodegradable linkage" or "reversible linkage" is is a linkage that is enzymatically and/or non-enzymatically hydrolytically degradable, i.e. cleavable, under physiological conditions (aqueous buffer at pH 7.4, 37°C) with a half-life ranging from one hour to twelve months. Preferably, a biodegradable linkage is non-enzymatically hydrolytically degradable, i.e. degradable independent of enzymatic activity, under physiological conditions with a half-life ranging from one hour to twelve months.

In contrast, a "permanent linkage" is non-enzymatically hydrolytically degradable under physiological conditions (aqueous buffer at pH 7.4, 37°C) with a half-life of more than twelve months.

As used herein, the term "pharmaceutical composition" means one or more active ingredients, i.e. drugs or prodrugs, and one or more inert ingredients, the so-called excipients, as well as any product which results, directly or indirectly, from combination, complexation or aggregation of any two or more of the ingredients, or from dissociation of one or more of the ingredients, or from other types of reactions or interactions of one or more of the ingredients. Accordingly, the pharmaceutical compositions of the present invention encompass any composition made by admixing the hydrogel-linked prodrug releasing tag moiety-biologically active moiety conjugates of the present invention and one or more pharmaceutically acceptable excipient(s).

As used herein, the term "excipient" refers to a diluent, adjuvant, or vehicle with which the active ingredient is administered. Such pharmaceutical excipient can be sterile liquids, such as water and oils, including those of petroleum, animal, vegetable or synthetic origin, including peanut oil, soybean oil, mineral oil and sesame oil. Water is a preferred excipient when the pharmaceutical composition is administered orally. Saline and aqueous dextrose are preferred excipients when the pharmaceutical composition is administered intravenously. Saline solutions and aqueous dextrose and glycerol solutions are preferably employed as liquid excipients for injectable solutions. Suitable pharmaceutical excipients include starch, glucose, lactose, sucrose, mannitol, trehalose, gelatin, malt, rice, flour, chalk, silica gel, sodium stearate, glycerol monostearate, talc, sodium chloride, dried skim milk, glycerol, propylene, glycol, water and ethanol. The pharmaceutical composition, if desired, can also contain minor amounts of wetting or emulsifying agents, pH buffering agents, like, for example, acetate, succinate, tris, carbonate, phosphate, HEPES (4-(2-hydroxyethyl)-1-piperazineethanesulfonic acid), MES (2-(N-morpholino)ethanesulfonic acid), or can contain detergents, like Tween, poloxamers, poloxamines, CHAPS, Igepal, or amino acids like, for example, glycine, lysine, or histidine. These pharmaceutical compositions can take the form of solutions, suspensions, emulsions, tablets, pills, capsules, powders and sustained-release formulations. The pharmaceutical composition can be formulated as a suppository, with traditional binders and excipients such as triglycerides. An oral formulation can include standard excipients such as pharmaceutical grades of mannitol, lactose, starch, magnesium stearate, sodium saccharine, cellulose and magnesium carbonate. Examples of suitable pharmaceutical excipients are described in "Remington's Pharmaceutical Sciences" by E.W. Martin. Such compositions will contain a therapeutically effective amount of the drug or prodrug, together with a suitable amount of excipient, so as to provide the form for proper administration to the patient. The formulation should suit the mode of administration.

In general the term "comprise" or "comprising" also encompasses "consist of" or "consisting of".

**Step (a)**

The hydrogel of step (a) may be any hydrogel known in the art that is suitable as a carrier for hydrogel-linked prodrugs. It is understood that the functional groups $A^{x0}$ and $A^{x2}$ are used to covalently conjugate reversible prodrug linker moieties or reversible prodrug linker moiety-biologically active moiety conjugate reagents to the hydrogel.

In certain embodiments, the groups $A^{x0}$ represent the same functional groups.

Preferably, $A^{x0}$ is selected from the group consisting of maleimide, amine $-NH_2$, hydroxyl -OH, thiol, carboxyl -COOH and activated carboxyl $-COY^1$, wherein $Y^1$ is selected from formulas (f-i) to (f-vi):

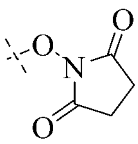 (f-i), 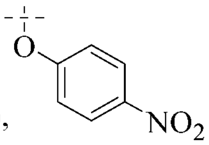 (f-ii), 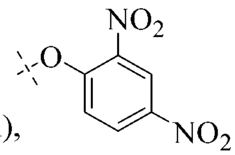 (f-iii),

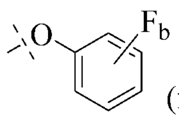 (f-iv), 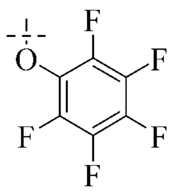 (f-v) and 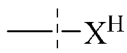 (f-vi),

wherein

the dashed lines indicate attachment to the rest of the molecule,

b is 1, 2, 3 or 4; and

$X^H$ is Cl, Br, I, or F.

More preferably, $A^{x0}$ is selected from the group consisting of maleimide, amine $-NH_2$, hydroxyl -OH, carboxyl -COOH and activated carboxyl $-COY^1$, wherein $Y^1$ is selected from formulas (f-i) to (f-vi):

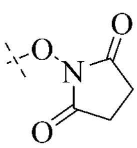 (f-i), 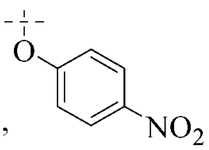 (f-ii), 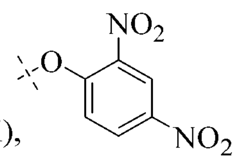 (f-iii),

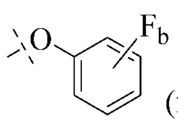 (f-iv), 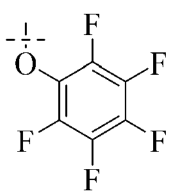 (f-v) and 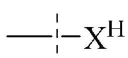 (f-vi),

wherein

the dashed lines indicate attachment to the rest of the molecule,

b is 1, 2, 3 or 4; and
$X^H$ is Cl, Br, I, or F.

More preferably, $A^{x0}$ is an amine or maleimide.

It is equally preferred that $A^{x0}$ is thiol.

Preferably, the hydrogel of step (a) is a shaped article, such as a coating, mesh, stent, nanoparticle or a microparticle. More preferably, the hydrogel is in the form of microparticular beads having a diameter from 1 to 1000 micrometer, more preferably with a diameter from 10 to 300 micrometer, even more preferably with a diameter from 20 and 150 micrometer and most preferably with a diameter from 30 to 130 micrometer. The aforementioned diameters are measured when the hydrogel microparticles are fully hydrated in water.

In one embodiment the hydrogel of step (a) is a hydrogel as disclosed in WO2006003014A2.

In another embodiment the hydrogel of step (a) is a hydrogel as disclosed in WO2011012715A1.

In a preferred embodiment the hydrogel of step (a) is obtainable by a process comprising the steps of:

(a-i) providing a mixture comprising

(a-ia) at least one backbone reagent, wherein the at least one backbone reagent has a molecular weight ranging from 1 to 100 kDa, and comprises at least three functional groups $A^{x0}$, wherein each $A^{x0}$ is a maleimide, amine $-NH_2$, hydroxyl -OH, carboxyl -COOH or activated carboxyl $-COY^1$, wherein $Y^1$ is selected from formulas (f-i) to (f-vi):

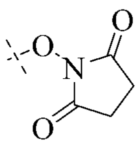 (f-i), 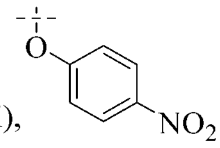 (f-ii), 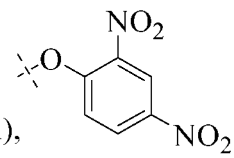 (f-iii),

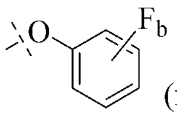 (f-iv), 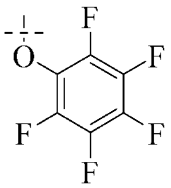 (f-v) and 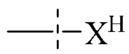 (f-vi),

wherein
the dashed lines indicate attachment to the rest of the molecule,

b is 1, 2, 3 or 4, and
$X^H$ is Cl, Br, I, or F;

(a-ib) at least one crosslinker reagent, wherein the at least one crosslinker reagent has a molecular weight ranging from 0.2 to 40 kDa and comprises at least two functional end groups selected from the group consisting of activated ester groups, activated carbamate groups, activated carbonate groups, activated thiocarbonate groups, amine groups and thiol groups;
in a weight ratio of the at least one backbone reagent to the at least one crosslinker reagent ranging from 1:99 to 99:1 and wherein the molar ratio of $A^{x0}$ to functional end groups is >1;

(a-ii) polymerizing the mixture of step (a-i) to a hydrogel; and

(a-iii) optionally working-up the hydrogel of step (a-ii).

Equally preferably, $A^{x0}$ of step (a-ia) is thiol.

**The Backbone Reagent of Step (a-ia)**

The at least one backbone reagent has a molecular weight ranging from 1 to 100 kDa, preferably from 2 to 50 kDa, more preferably from 5 and 30 kDa, even more preferably from 5 to 25 kDa and most preferably from 5 to 15 kDa.

In one embodiment the at least one backbone reagent of step (a-ia) is present in the form of its acidic salt, preferably in the form of an acid addition salt, if $A^{x0}$ is amine. Suitable acid addition salts are formed from acids which form non-toxic salts. Examples include acetate, aspartate, benzoate, besylate, bicarbonate, carbonate, bisulphate, sulphate, borate, camsylate, citrate, edisylate, esylate, formate, fumarate, gluceptate, gluconate, glucuronate, hexafluorophosphate, hibenzate, hydrochloride, hydrobromide, hydroiodide, isethionate, lactate, malate, maleate, malonate, mesylate, methylsulphate, naphthylate, nicotinate, nitrate, orotate, oxalate, palmitate, pamoate, phosphate, hydrogen phosphate, dihydrogen phosphate, sacharate, stearate, succinate, tartrate and tosylate. Particularly preferred, the backbone reagent is present in the form of its hydrochloride salt.

The at least one backbone reagent of step (a-ia) comprises one or more polymer(s) selected from the group consisting of 2-methacryloyl-oxyethyl phosphoyl cholins, poly(acrylic acids), poly(acrylates), poly(acrylamides), poly(alkyloxy) polymers, poly(amides), poly(amidoamines), poly(amino acids), poly(anhydrides), poly(aspartamides), poly(butyric acids), poly(glycolic acids), polybutylene terephthalates, poly(caprolactones), poly(carbonates), poly(cyanoacrylates), poly(dimethylacrylamides), poly(esters), poly(ethylenes), poly(ethyleneglycols), poly(ethylene oxides), poly(ethyl phosphates), poly(ethyloxazolines), poly(glycolic acids), poly(hydroxyethyl acrylates), poly(hydroxyethyl-oxazolines), poly(hydroxymethacrylates), poly(hydroxypropylmethacrylamides), poly(hydroxypropyl methacrylates), poly(hydroxypropyloxazolines), poly(iminocarbonates), poly(lactic acids), poly(lactic-co-glycolic acids), poly(methacrylamides), poly(methacrylates), poly(methyloxazolines), poly(organophosphazenes), poly(ortho esters), poly(oxazolines), polypropylene glycols), poly(siloxanes), poly(urethanes), poly(vinyl alcohols), poly(vinyl amines), poly(vinylmethylethers), poly(vinylpyrrolidones), silicones, celluloses, carbomethyl celluloses, hydroxypropyl methylcelluloses, chitins, chitosans, dextrans, dextrins, gelatins, hyaluronic acids and derivatives, functionalized hyaluronic acids, mannans, pectins, rhamnogalacturonans, starches, hydroxyalkyl starches, hydroxyethyl starches and other carbohydrate-based polymers, xylans, and copolymers thereof.

Preferably, the at least one backbone reagent of step (a-ia) is PEG-based comprising at least 10% PEG or is hyaluronic acid-based comprising at least 20% hyaluronic acid.

In a preferred embodiment, the at least one backbone reagent of step (a-ia) is hyaluronic acid-based comprising at least 20% hyaluronic acid, more preferably, comprising at least 40% hyaluronic acid, even more preferably, at least 60% hyaluronic acid, even more preferred at least 80% hyaluronic acid.

Preferably, in such hyaluronic acid-comprising backbone reagent of step (a-ia) each $A^{x0}$ is an amine.

In another preferred embodiment, the at least one backbone reagent of step (a-ia) is PEG-based comprising at least 10% PEG, preferably at least 20% PEG, even more preferably at least 30%, even more preferably at least 40% PEG, even more preferably at least 50% PEG, and most preferably at least 60%.

Preferably, in such PEG-based backbone reagent of step (a-ia) each $A^{x0}$ is an amine or maleimide and most preferably each $A^{x0}$ is an amine.

In one embodiment, the at least one backbone reagent of step (a-ia) is selected from the group consisting of

(i) a compound of formula (IIIa)

$$B(-(A^0)_{x1}-(SP^1)_{x2}-A^1-P-A^2-Hyp^1)_x \qquad (IIIa),$$

wherein

| | |
|---|---|
| B | is a branching core, |
| $SP^1$ | is a spacer moiety selected from the group consisting of $C_{1-6}$ alkyl, $C_{2-6}$ alkenyl and $C_{2-6}$ alkynyl, |
| P | is a PEG-based polymeric chain comprising at least 80% PEG, preferably at least 85% PEG, more preferably at least 90% PEG and most preferably at least 95% PEG, |
| $Hyp^1$ | is a moiety comprising an amine ($-NH_2$ and/or -NH-) or a polyamine comprising at least two amines ($-NH_2$ and/or -NH-), |
| x | is an integer from 3 to 16, |
| x1, x2 | are independently of each other 0 or 1, provided that x1 is 0, if x2 is 0, |
| $A^0$, $A^1$, $A^2$ | are independently of each other selected from the group consisting of |

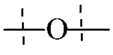, 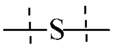, 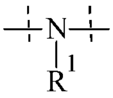, 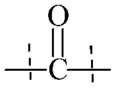, 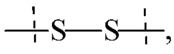, 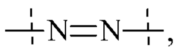,

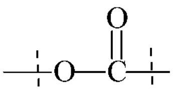, 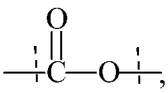, 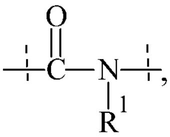, 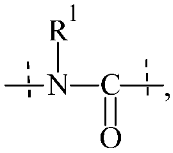, 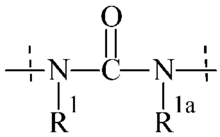,

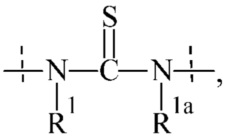, 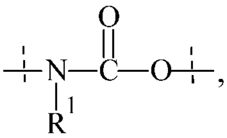, 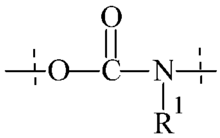,

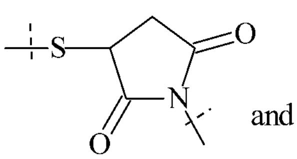 and 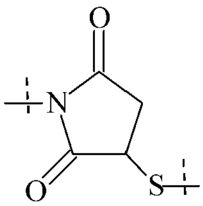 ,

wherein $R^1$ and $R^{1a}$ are independently of each other H or $C_{1-6}$ alkyl;

(ii) a compound of formula (IIIb)

$$Hyp^2\text{-}A^3\text{-}P\text{-}A^4\text{-}Hyp^3 \qquad \text{(IIIb)},$$

wherein

| | |
|---|---|
| P | is defined as above in the compound of formula (IIIa), |
| $Hyp^2$, $Hyp^3$ | are independently of each other a polyamine comprising at least two amines ($-NH_2$ and/or $-NH-$), and |
| $A^3$ and $A^4$ | are independently selected from the group consisting of |

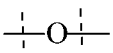, 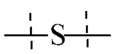, 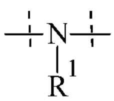, 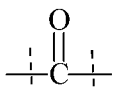, 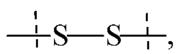, 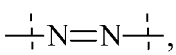,

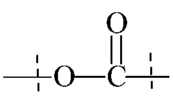, 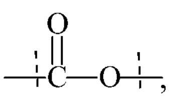, 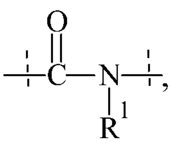, 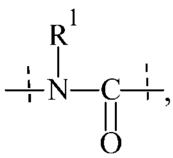, 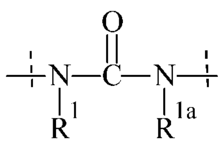,

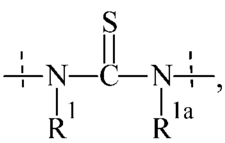, 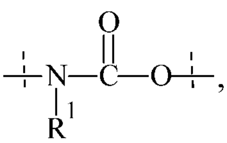, 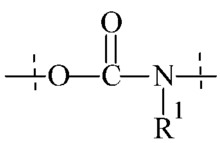,

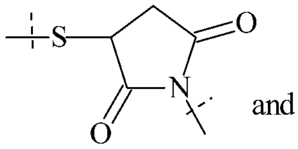 and 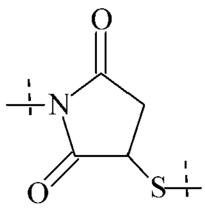 ,

wherein $R^1$ and $R^{1a}$ are independently of each other H or $C_{1-6}$ alkyl;

(iii) a compound of formula (IIIc)

$P^1$-$A^5$-$Hyp^4$ (IIIc),

wherein

| | |
|---|---|
| $P^1$ | is a PEG-based polymeric chain comprising at least 80% PEG, preferably at least 85% PEG, more preferably at least 90% PEG and most preferably at least 95% PEG, |
| $Hyp^4$ | is a polyamine comprising at least three amines ($-NH_2$ and/or -NH), and |
| $A^5$ | is selected from the group consisting of |

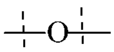, 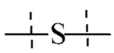, 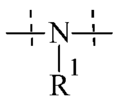, 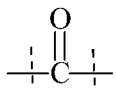, 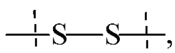, 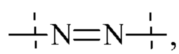,

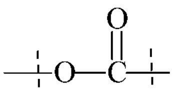, 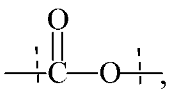, 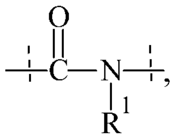, 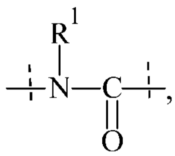, 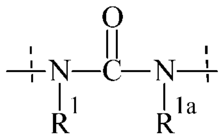,

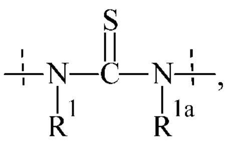, 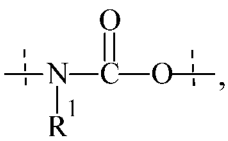, 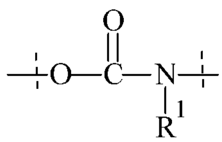,

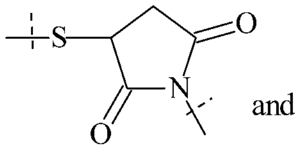 and 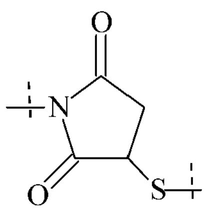 ,

wherein $R^1$ and $R^{1a}$ are independently of each other H or $C_{1-6}$ alkyl;
and

(iv) a compound of formula (IIId)

$T^1$-$A^6$-$Hyp^5$ (IIId),

wherein

| | |
|---|---|
| $Hyp^5$ | is a polyamine comprising at least three amines ($-NH_2$ and/or -NH), and |
| $A^6$ | is selected from the group consisting of |

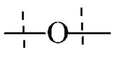, 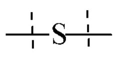, 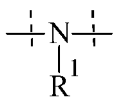, 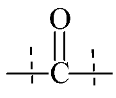, 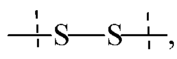, 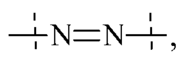,

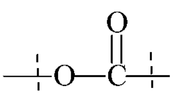, 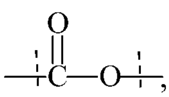, 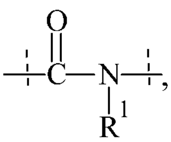, 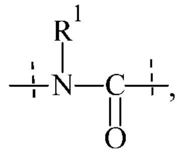, 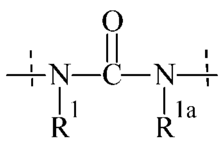,

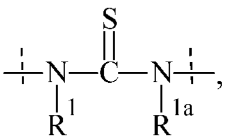, 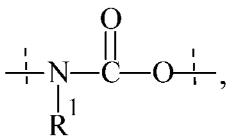, 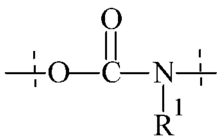,

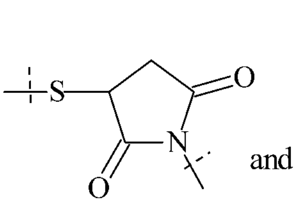 and 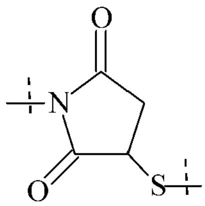,

wherein $R^1$ and $R^{1a}$ are independently of each other H or $C_{1-6}$ alkyl; and

$T^1$ is selected from the group consisting of $C_{1-50}$ alkyl, $C_{2-50}$ alkenyl and $C_{2-50}$ alkynyl, which $C_{1-50}$ alkyl, $C_{2-50}$ alkenyl or $C_{2-50}$ alkynyl are optionally interrupted by one or more group(s) selected from the group consisting of -NH-, -N($C_{1-4}$ alkyl)-, -O-, -S-, -C(O)-, -C(O)NH-, -C(O)N($C_{1-4}$ alkyl)-, -O-C(O)-, -S(O)-, -S(O)$_2$-, 4- to 7-membered heterocyclyl, phenyl and naphthyl.

In the following sections the term "Hyp"" refers to $Hyp^1$, $Hyp^2$, $Hyp^3$, $Hyp^4$ and $Hyp^5$ collectively.

Preferably, the backbone reagent is a compound of formula (IIIa), (IIIb) or (IIIc), more preferably the backbone reagent is a compound of formula (IIIa) or (IIIc), and most preferably the backbone reagent is a compound of formula (IIIa).

In a preferred embodiment, the backbone reagent is of formula (IIIa) and x is 4, 6 or 8, more preferably x is 4 or 8 and most preferably x is 4.

In a preferred embodiment $A^0$, $A^1$, $A^2$, $A^3$, $A^4$, $A^5$ and $A^6$ of formulas (IIIa) to (IIId) are selected from the group consisting of

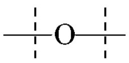, 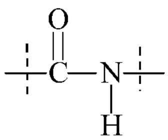, 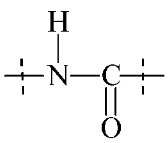, and 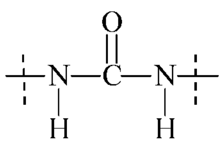.

Preferably, $A^0$ of formula (IIIa) is selected from the group consisting of

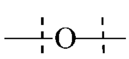, 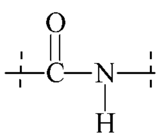, and 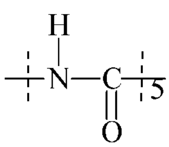.

Preferably, $A^1$ of formula (IIIa) is selected from the group consisting of

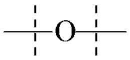 , 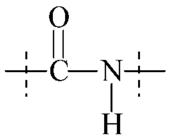 , and 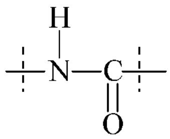 .

Preferably, $A^2$ of formula (IIIa) is selected from the group consisting of

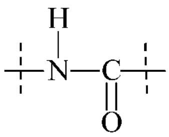 , and 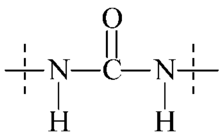 .

Preferably, $A^3$ of formula (IIIb) is selected from the group consisting of

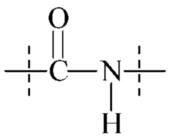 , and 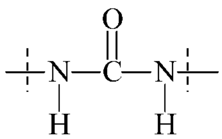 .

Preferably, $A^4$ of formula (IIIb) is selected from the group consisting of

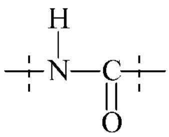 , and 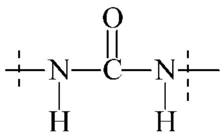 .

Preferably, $A^5$ of formula (IIIc) is selected from the group consisting of

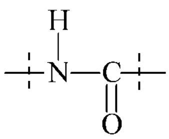 , and 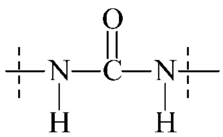 .

Preferably, $A^6$ of formula (IIId) is selected from the group consisting of

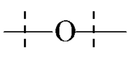 , 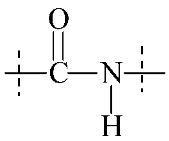 , and 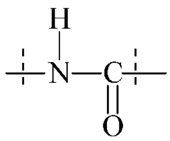 .

Preferably, in a compound of formula (IIId), $T^1$ is H or $C_{1-6}$ alkyl.

$SP^1$ is a spacer moiety selected from the group consisting of $C_{1-6}$ alkyl, $C_{2-6}$ alkenyl and $C_{2-6}$ alkynyl. Preferably, $SP^1$ is $-CH_2-$, $-CH_2-CH_2-$, $-CH(CH_3)-$, $-CH_2-CH_2-CH_2-$, $-CH(C_2H_5)-$, $-C(CH_3)_2-$, -CH=CH- or -CH=CH-, and most preferably $SP^1$ is $-CH_2-$, $-CH_2-CH_2-$ or -CH=CH-.

In one embodiment B of formula (IIIa) is selected from the group consisting of:

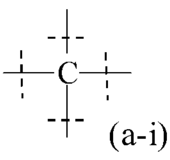 (a-i) 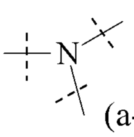 (a-ii) 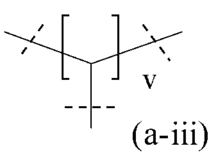 (a-iii) 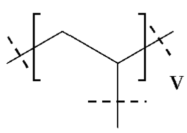 (a-iv)

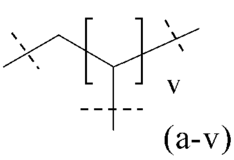
(a-v)
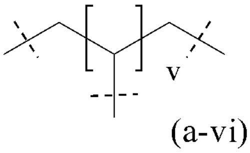
(a-vi)
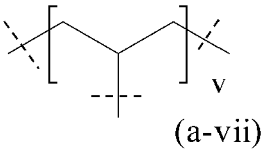
(a-vii)

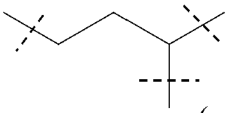
(a-viii)
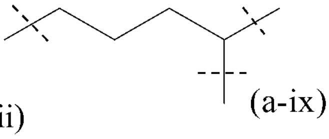
(a-ix)
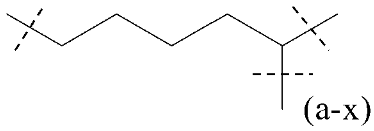
(a-x)

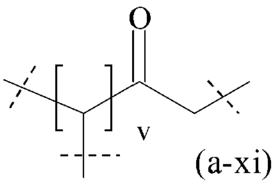
(a-xi)
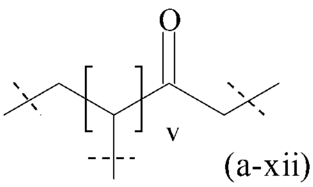
(a-xii)
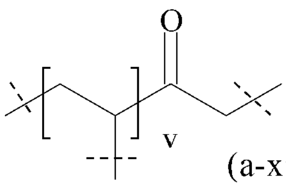
(a-xiii)

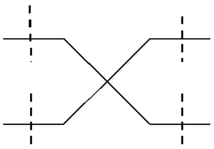
(a-xiv)
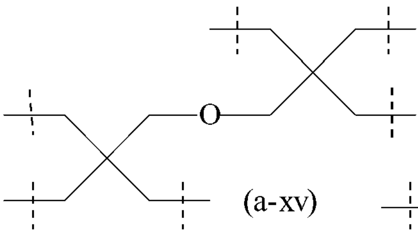
(a-xv)
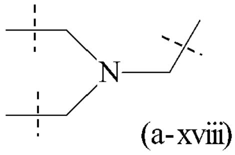
(a-xviii)

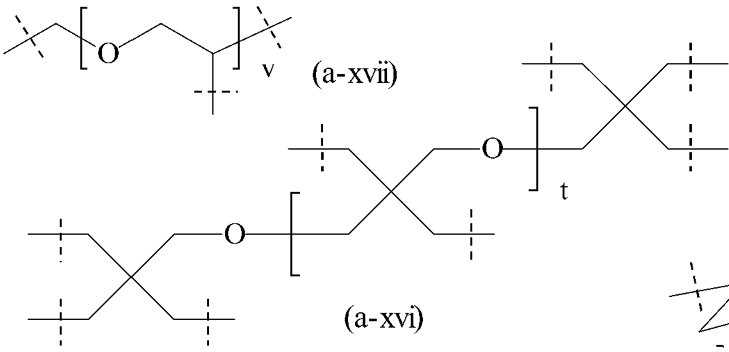
(a-xvii)

(a-xvi)
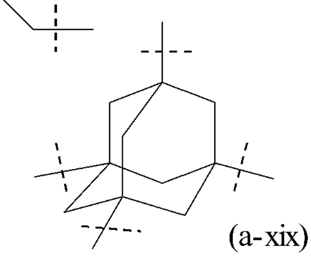
(a-xix)

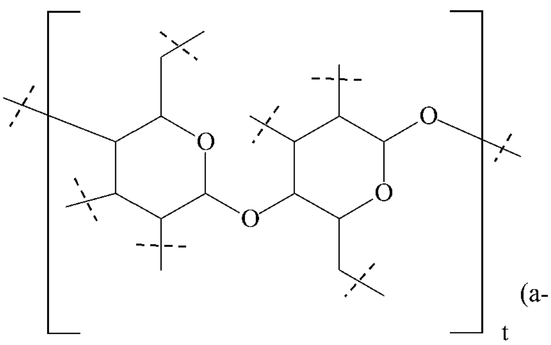
(a-xx)
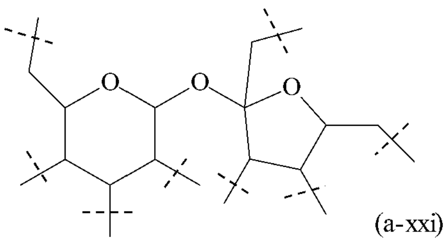
(a-xxi)

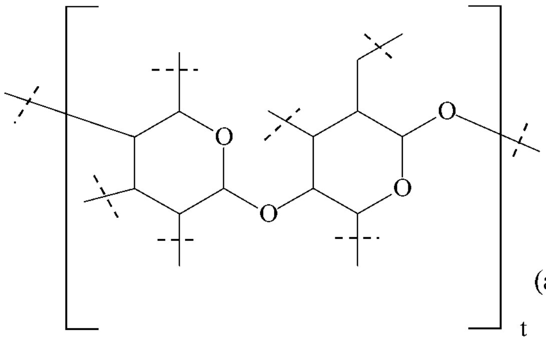

(a-xxii) and

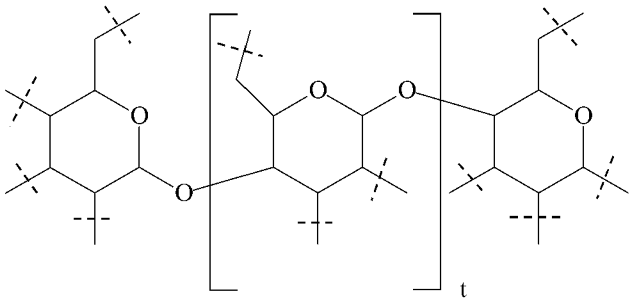

(a-xxiii)

wherein
dashed lines indicate attachment to $A^0$ or, if x1 and x2 are both 0, to $A^1$,

t is 1 or 2; preferably t is 1,

v is 1, 2, 3, 4, 5, 6, 7, 8, 9, 10, 11, 12, 13 or 14; preferably, v is 2, 3, 4, 5, 6; more preferably, v is 2, 4 or 6; most preferably, v is 2.

In a preferred embodiment, B has a structure of formula (a-i), (a-ii), (a-iii), (a-iv), (a-v), (a-vi), (a-vii), (a-viii), (a-ix), (a-x), (a-xiv), (a-xv) or (a-xvi). More preferably, B has a structure of formula (a-iii), (a-iv), (a-v), (a-vi), (a-vii), (a-viii), (a-ix), (a-x) or (a-iv). Most preferably, B has a structure of formula (a-xiv).

A preferred combination of B and $A^0$, or, if x1 and x2 are both 0, of B and $A^1$, is selected from the group consisting of the following structures:

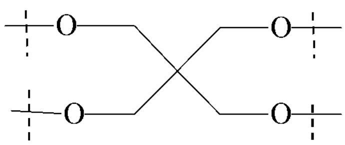

(b-i)

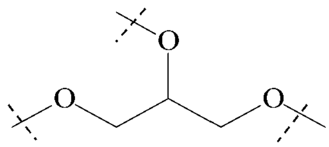

(b-ii)

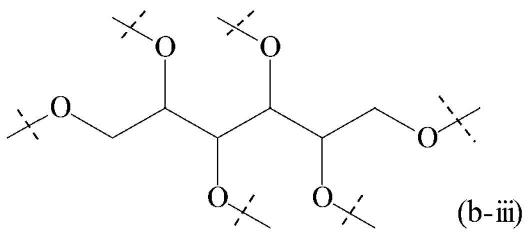

(b-iii)

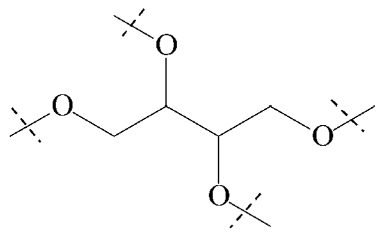

(b-iv)

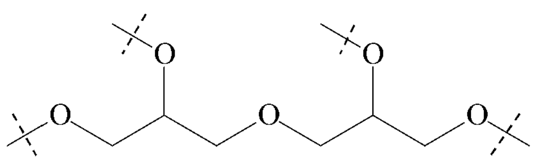

(b-v)

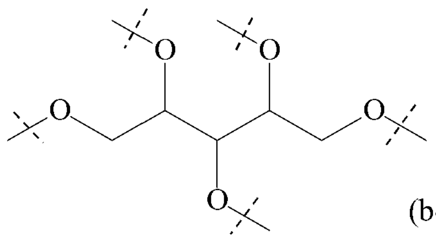

(b-vi)

and

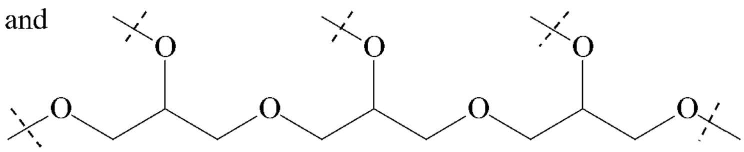

(b-vii)

wherein

dashed lines indicate attachment to $SP^1$ or, if x1 and x2 are both 0, to P.

More preferably, the combination of B and $A^0$ or, if x1 and x2 are both 0, the combination of B and $A^1$, is of formula (b-i), (b-iv), (b-vi) or (b-viii) and most preferably is of formula (b-i).

In one embodiment, x1 and x2 of formula (IIIa) are both 0.

In one embodiment, the PEG-based polymeric chain P has a molecular weight from 0.3 kDa to 40 kDa; e.g. from 0.4 to 35 kDa, from 0.6 to 38 kDa, from 0.8 to 30 kDa, from 1 to 25 kDa, from 1 to 15 kDa or from 1 to 10 kDa. Most preferably, P has a molecular weight from 1 to 10 kDa.

In one embodiment, the PEG-based polymeric chain $P^1$ has a molecular weight from 0.3 kDa to 40 kDa; e.g. from 0.4 to 35 kDa, from 0.6 to 38 kDA, from 0.8 to 30 kDa, from 1 to 25 kDa, from 1 to 15 kDa or from 1 to 10 kDa. Most preferably, $P^1$ has a molecular weight from 1 to 10 kDa.

In one embodiment, P of formula (IIIa) or (IIIb) is of formula (c-i):

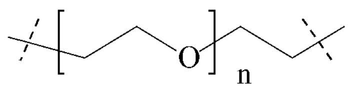

(c-i),

wherein n ranges from 6 to 900, more preferably n ranges from 20 to 700 and most preferably n ranges from 20 to 250.

In one embodiment, $P^1$ of formula (IIIc) has the structure of formula (c-ii):

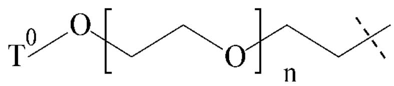

(c-ii),

wherein

- n ranges from 6 to 900, more preferably n ranges from 20 to 700 and most preferably n ranges from 20 to 250;
- $T^0$ is selected from the group consisting of $C_{1-6}$ alkyl, $C_{2-6}$ alkenyl and $C_{2-6}$ alkynyl, which $C_{1-6}$ alkyl, $C_{2-6}$ alkenyl and $C_{2-6}$ alkynyl are optionally interrupted by one or more group(s) selected from the group consisting of -NH-, -N($C_{1-4}$ alkyl)-, -O-, -S-, -C(O)-, -C(O)NH-, -C(O)N($C_{1-4}$ alkyl)-, -O-C(O)-, -S(O)- and $-S(O)_2-$.

In one embodiment, the moiety $Hyp^x$ of formulas (IIIa) to (IIId) is a polyamine and preferably comprises in bound form and, where applicable, in R- and/or S-configuration, a moiety of formulas (d-i), (d-ii), (d-iii) and/or (d-iv):

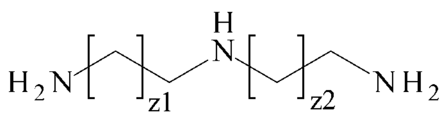

(d-i),

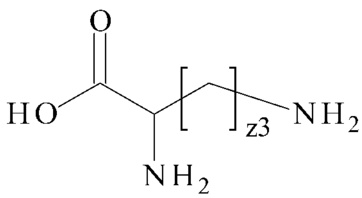

(d-ii),

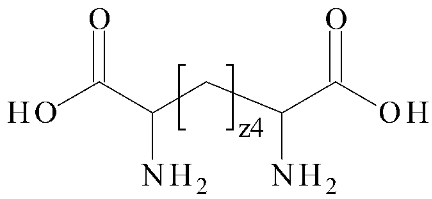

(d-iii),

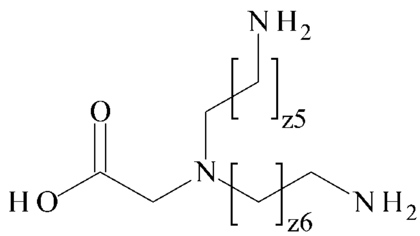

(d-iv),

wherein

z1, z2, z3, z4, z5, z6 are independently of each other 1, 2, 3, 4, 5, 6, 7 or 8.

More preferably, $Hyp^x$ comprises in bound form and in R- and/or S-configuration lysine, ornithine, diaminopropionic acid and/or diaminobutyric acid. $Hyp^x$ comprises in bound form and in R- and/or S-configuation lysine.

Preferably, $Hyp^x$ has a molecular weight from 40 Da to 30 kDa, preferably from 0.3 kDa to 25 kDa, more preferably from 0.5 kDa to 20 kDa, even more preferably from 1 kDa to 20 kDa and most preferably from 2 kDa to 15 kDa.

$Hyp^x$ is preferably selected from the group consisting of:

a moiety of formula (e-i)

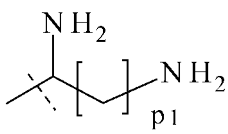

**(e-i)**

wherein

p1 is an integer from 1 to 5, preferably p1 is 4, and

the dashed line indicates attachment to $A^2$ if the backbone reagent is of formula (IIIa) and to $A^3$ or $A^4$ if the backbone reagent is of formula (IIIb);

a moiety of formula (e-ii)

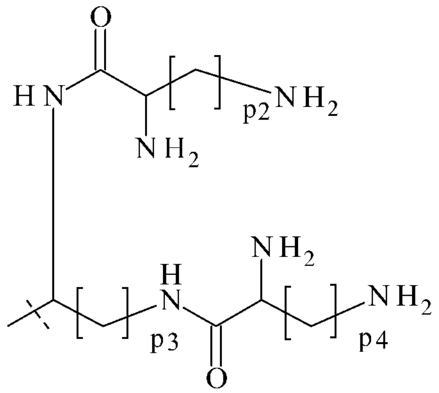

(e-ii)

wherein

p2, p3 and p4 are identical or different and each is independently of the others an integer from 1 to 5, preferably p2, p3 and p4 are 4, and

the dashed line indicates attachment to $A^2$ if the backbone reagent is of formula (IIIa), to $A^3$ or $A^4$ if the backbone reagent is of formula (IIIb), to $A^5$ if the backbone is of formula (IIIc) and to $A^6$ if the backbone reagent is of formula (IIId);

a moiety of formula (e-iii)

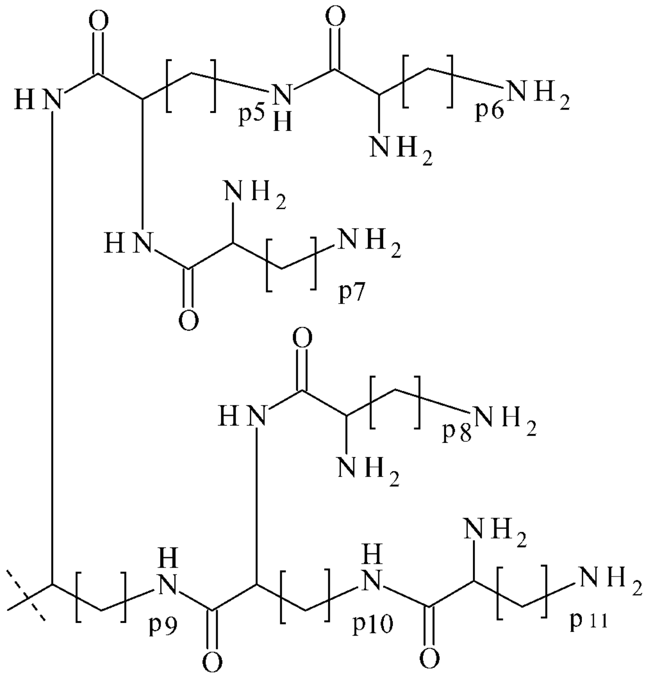

(e-iii)

wherein

p5 to p11 are identical or different and each is independently of the others an integer from 1 to 5, preferably p5 to p11 are 4, and

the dashed line indicates attachment to $A^2$ if the backbone reagent is of formula (IIIa), to $A^3$ or $A^4$ if the backbone reagent is of formula (IIIb), to $A^5$ if the backbone reagent is of formula (IIIc) and to $A^6$ if the backbone reagent is of formula (IIId);

a moiety of formula (e-iv)

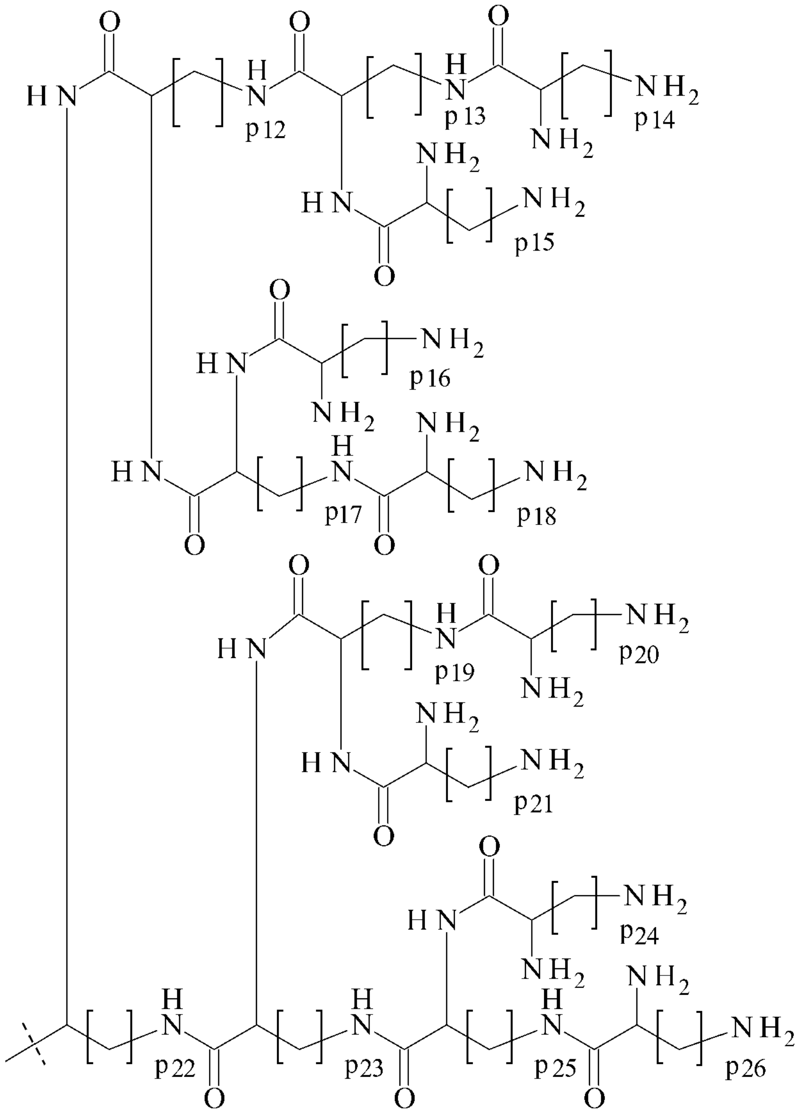

(e-iv)

wherein

p12 to p26 are identical or different and each is independently of the others an integer from 1 to 5, preferably p12 to p26 are 4, and

the dashed line indicates attachment to $A^2$ if the backbone reagent is of formula (IIIa), to $A^3$ or $A^4$ if the backbone reagent is of formula (IIIb), to $A^5$ if the backbone is of formula (IIIc) and to $A^6$ if the backbone reagent is of formula (IIId);

a moiety of formula (e-v)

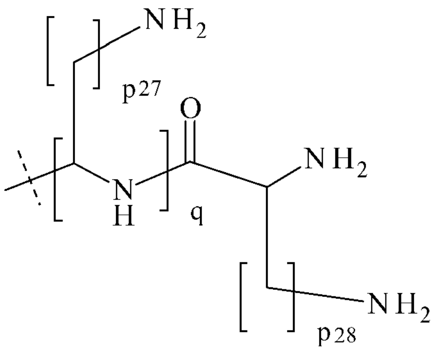

(e-v)

wherein

p27 and p28 are identical or different and each is independently of the other an integer from 1 to 5, preferably p27 and p28 are 4,

q is an integer from 1 to 8, preferably q is 2 or 6 and most preferably q is 6, and

the dashed line indicates attachment to $A^2$ if the backbone reagent is of formula (IIIa), to $A^3$ or $A^4$ if the backbone reagent is of formula (IIIb), to $A^5$ if the backbone reagent is of formula (IIIc) and to $A^6$ if the backbone reagent is of formula (IIId);

a moiety of formula (e-vi)

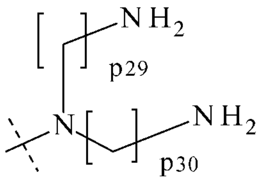

(e-vi)

wherein

p29 and p30 are identical or different and each is independently of the other an integer from 2 to 5, preferably p29 and p30 are 3, and

the dashed line indicates attachment to $A^2$ if the backbone reagent is of formula (IIIa), to $A^3$ or $A^4$ if the backbone reagent is of formula (IIIb), to $A^5$ if the backbone reagent is of formula (IIIc) and to $A^6$ if the backbone reagent is of formula (IIId);

a moiety of formula (e-vii)

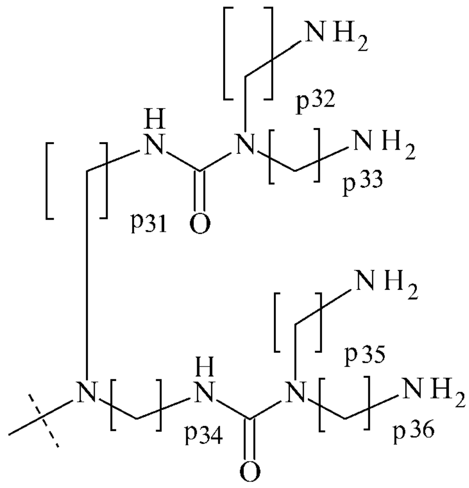

(e-vii)

wherein

p31 to p36 are identical or different and each is independently of the others an integer from 2 to 5, preferably p31 to p36 are 3, and

the dashed line indicates attachment to $A^2$ if the backbone reagent is of formula (IIIa), to $A^3$ or $A^4$ if the backbone reagent is of formula (IIIb), to $A^5$ if the backbone reagent is of formula (IIIc) and to $A^6$ if the backbone reagent is of formula (IIId);

a moiety of formula (e-viii)

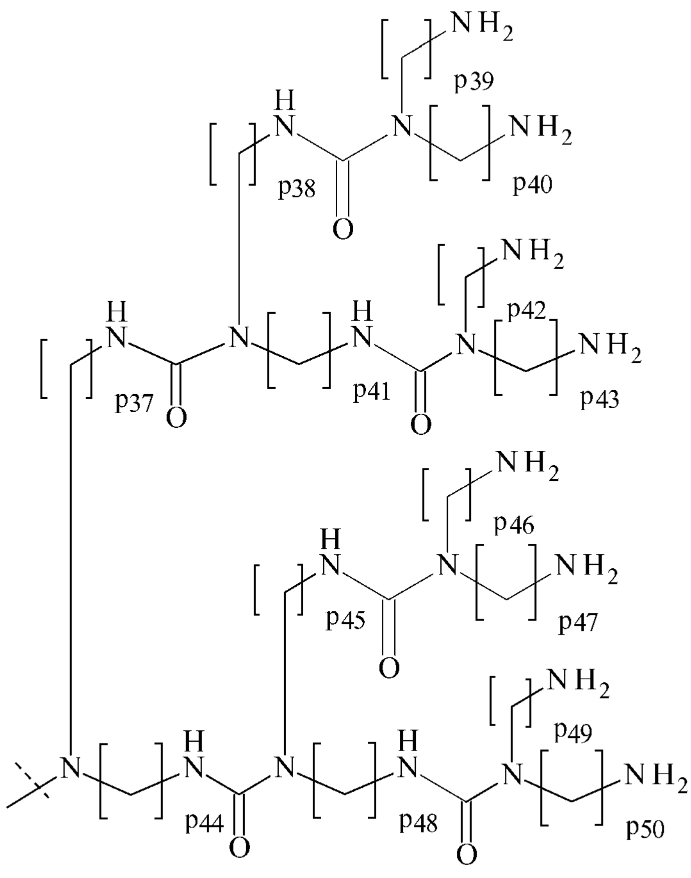

(e-viii)

wherein

p37 to p50 are identical or different and each is independently of the others an integer from 2 to 5, preferably p37 to p50 are 3, and

the dashed line indicates attachment to $A^2$ if the backbone reagent is of formula (IIIa), to $A^3$ or $A^4$ if the backbone reagent is of formula (IIIb), to $A^5$ if the backbone reagent is of formula (IIIc) and to $A^6$ if the backbone reagent is of formula (IIId); and

a moiety of formula (e-ix):

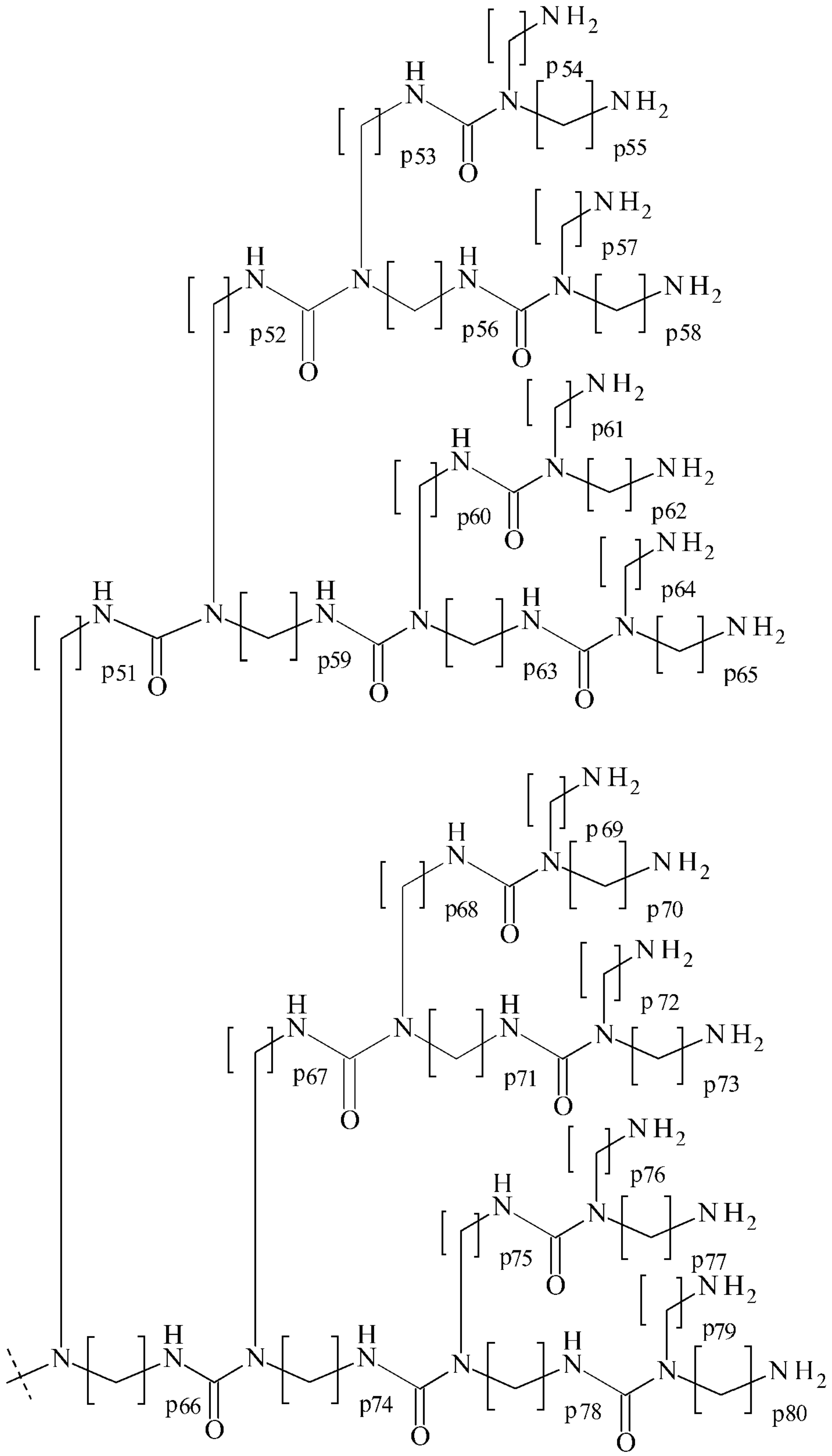

(e-ix)

wherein

p51 to p80 are identical or different and each is independently of the others an integer from 2 to 5, preferably p51 to p80 are 3, and

the dashed line indicates attachment to $A^2$ if the backbone reagent is of formula (IIIa), to $A^3$ or $A^4$ if the backbone reagent is of formula (IIIb), to $A^5$ if the backbone reagent is of formula (IIIc) and to $A^6$ if the backbone reagent is of formula (IIId); and

wherein the moieties (e-i) to (e-v) may at each chiral center be in either R- or S-configuration, preferably, all chiral centers of a moiety (e-i) to (e-v) are in the same configuration.

Preferably, $Hyp^x$ is of formula (e-i), (e-ii), (e-iii), (e-iv), (e-vi), (e-vii), (e-viii) or (e-ix). More preferably, $Hyp^x$ is of formula (e-ii), (e-iii), (e-iv), (e-vii), (e-viii) or (e-ix), even more preferably $Hyp^x$ is of formula (e-ii), (e-iii), (e-vii) or (e-viii) and most preferably $Hyp^x$ is of formula (e-iii).

Preferrably, the moiety - $A^2$ - $Hyp^1$ is

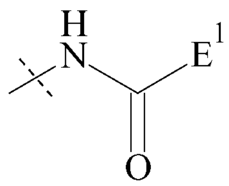

wherein

the dashed line indicates attachment to P; and
$E^1$ is selected from formulas (e-i) to (e-ix).

Preferrably, the moiety $Hyp^2$ - $A^3$ - is

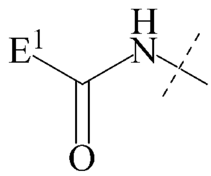

,

wherein

the dashed line indicates attachment to P; and
$E^1$ is selected from formulas (e-i) to (e-ix).

Preferably, the moiety - $A^4$ - $Hyp^3$ is

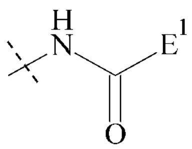

,

wherein

the dashed line indicates attachment to P; and
$E^1$ is selected from formulas (e-i) to (e-ix).

Preferably, the moiety - $A^5$ - $Hyp^4$ is

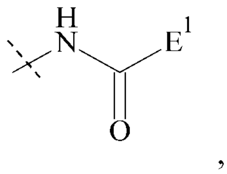

wherein

the dashed line indicates attachment to $P^1$; and
$E^1$ is selected from formulas (e-i) to (e-ix).

More preferably, the backbone reagent is of formula (IIIa) and B is of formula (a-xiv).
Even more preferably, the backbone reagent is of formula (IIIa), B is of formula (a-xiv), x1 and x2 are 0, and $A^1$ is -O-.
Even more preferably, the backbone reagent is of formula (IIIa), B is of formula (a-xiv), $A^1$ is -O-, and P is of formula (c-i).
Even more preferably, the backbone reagent is of formula (IIIa), B is of formula (a-xiv), x1 and x2 are 0, $A^1$ is -O-, and P is of formula (c-i).
Most preferably, the backbone reagent has the following formula:

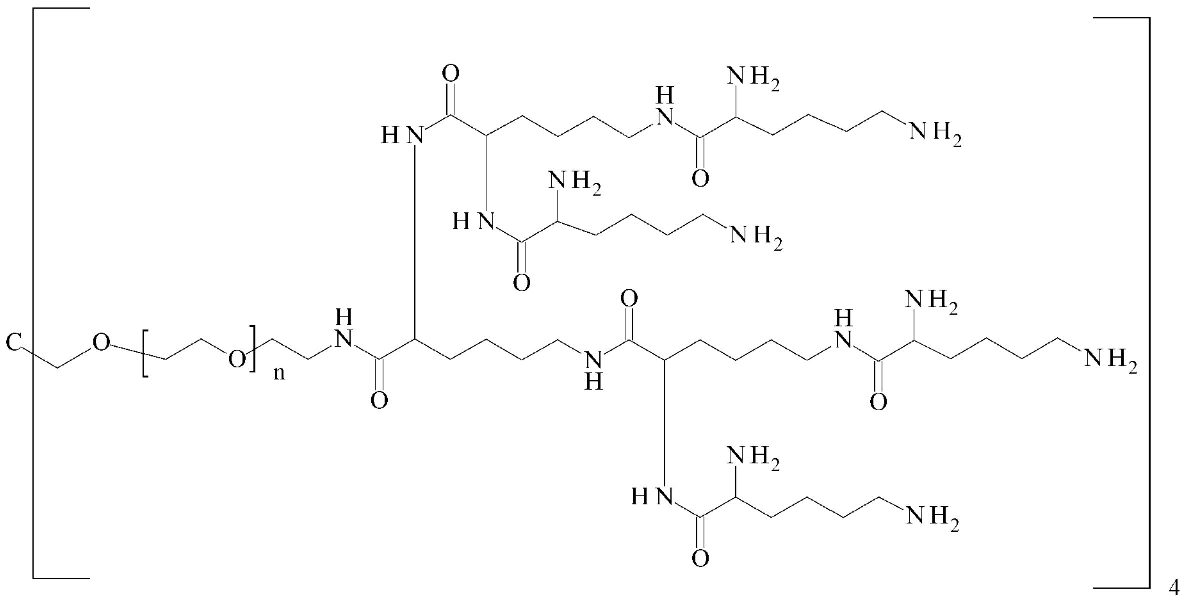

wherein

n ranges from 10 to 40, preferably from 10 to 30, more preferably from 10 to 20.

Equally preferably, n ranges from 20 to 30 and most preferably n is 28.

**The Crosslinker Reagent of Step (a-ib)**

Preferably, the at least one crosslinker reagent of step (a-ib) comprises a polymer.
The at least one crosslinker reagent of step (a-ib) comprises one or more polymer(s) selected from the group consisting of 2-methacryloyl-oxyethyl phosphoyl cholins, poly(acrylic acids), poly(acrylates), poly(acrylamides), poly(alkyloxy) polymers, poly(amides), poly(amidoamines), poly(amino acids), poly(anhydrides), poly(aspartamides), poly(butyric acids), poly(glycolic acids), polybutylene terephthalates, poly(caprolactones), poly(carbonates), poly(cyanoacrylates), poly(dimethylacrylamides), poly(esters), poly(ethylenes), poly(ethyleneglycols), poly(ethylene oxides), poly(ethyl phosphates), poly(ethyloxazolines), poly(glycolic acids), poly(hydroxyethyl acrylates), poly(hydroxyethyl-oxazolines), poly(hydroxymethacrylates), poly(hydroxypropylmethacrylamides), poly(hydroxypropyl methacrylates), poly(hydroxypropyloxazolines), poly(iminocarbonates), poly(lactic acids), poly(lactic-co-glycolic acids), poly(methacrylamides), poly(methacrylates), poly(methyloxazolines), poly(organophosphazenes), poly(ortho esters), poly(oxazolines), polypropylene glycols), poly(siloxanes), poly(urethanes), poly(vinyl alcohols), poly(vinyl amines), poly(vinylmethylethers), poly(vinylpyrrolidones), silicones, celluloses, carbomethyl celluloses, hydroxypropyl methylcelluloses, chitins, chitosans, dextrans, dextrins, gelatins, hyaluronic acids and derivatives, functionalized hyaluronic acids, mannans, pectins, rhamnogalacturonans, starches, hydroxyalkyl starches, hydroxyethyl starches and other carbohydrate-based polymers, xylans, and copolymers thereof.

Preferably, the at least one crosslinker reagent of step (a-ib) comprises hyaluronic acid or PEG.

In one preferred embodiment, the at least one crosslinker reagent of step (a-ib) comprises hyaluronic acid. Preferably, such hyaluronic acid-comprising crosslinker reagent of step (a-ib) comprises at least 70% hyaluronic acid, more preferably at least 80 % hyaluronic acid and most preferably at least 90% hyaluronic acid and further comprises

(i) at least two carbonyloxy groups (-(C=O)-O- or -O-(C=O)-), and additionally
(ii) at least two functional end groups selected from the group consisting of activated ester groups, activated carbamate groups, activated carbonate groups, activated thiocarbonate groups, amine groups and thiol groups.

In another preferred embodiment, the at least one crosslinker reagent of step (a-ib) comprises PEG. Preferably, such PEG-comprising crosslinker reagent of step (a-ib) comprises at least 70% PEG, more preferably at least 80 % PEG and most preferably at least 90% PEG and further comprises

(i) at least two carbonyloxy groups (-(C=O)-O- or -O-(C=O)-), and additionally
(ii) at least two functional end groups selected from the group consisting of activated ester groups, activated carbamate groups, activated carbonate groups, activated thiocarbonate groups, amine groups and thiol groups.

The at least one crosslinker reagent preferably comprises at least two carbonyloxy groups (-(C=O)-O- or -O-(C=O)-), which are biodegradable linkages. These biodegradable linkages render the hydrogel biodegradable which is advantageous. In addition, the at least one crosslinker reagent comprises at least two functional end groups which during the polymerization of step (a-ii) react with the functional groups $A^{x0}$ of the at least one backbone reagent of step (a-ia).

The crosslinker reagent has a molecular weight ranging from 0.2 to 40 kDa, more preferably ranging from 0.5 to 30 kDa, even more preferably ranging from 0.5 to 20 kDa, even more preferably ranging from 0.5 to 15 kDa and most preferably ranging from 1 to 10 kDa.

Preferably, the reaction of a functional end group of a crosslinker reagent with a functional group $A^{x0}$ of a backbone reagent leads to the formation of an amide linkage between a backbone moiety and a crosslinker moiety, i.e. a backbone moiety and a crosslinker moiety are preferably connected through an amide linkage.

In one preferred embodiment, the crosslinker reagent is a compound of formula (IV-I):

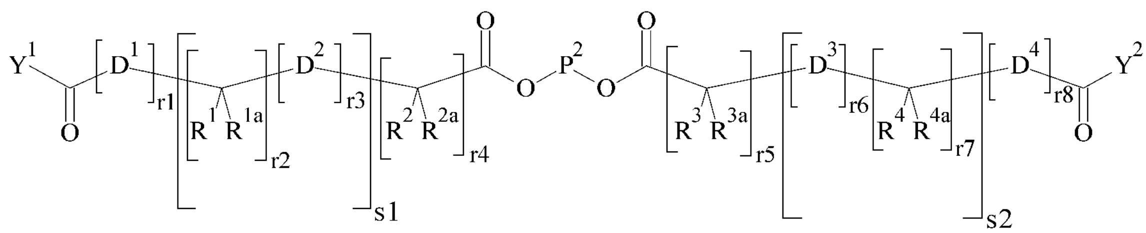

(IV-I),

wherein

| | |
|---|---|
| each $D^1$, $D^2$, $D^3$ and $D^4$ | are identical or different and each is independently of the others selected from the group comprising -O-, $-NR^5-$, -S- and $-CR^6R^{6a}-$; |
| each $R^1$,$R^{1a}$,$R^2$,$R^{2a}$, $R^3$, $R^{3a}$, $R^4$, $R^{4a}$, $R^6$ and $R^{6a}$ | are identical or different and each is independently of the others selected from the group comprising -H, $-OR^7$, $-NR^7R^{7a}$, $-SR^7$ and $C_{1-6}$ alkyl; optionally, each of the pair(s) $R^1/R^2$, $R^3/R^4$, $R^{1a}/R^{2a}$, and $R^{3a}/R^{4a}$ may independently form a chemical bond and/or each of the pairs $R^1/R^{1a}$, $R^2/R^{2a}$, $R^3/R^{3a}$, $R^4/R^{4a}$, $R^6/R^{6a}$, $R^1/R^2$, $R^3/R^4$, $R^{1a}/R^{2a}$, and $R^{3a}/R^{4a}$ are independently of each other joined to- |

gether with the atom to which they are attached to form a $C_{3-8}$ cycloalkyl or to form a ring A or are joined together with the atom to which they are attached to form a 4- to 7-membered heterocyclyl or 8- to 11-membered heterobicyclyl or adamantyl;

each $R^5$ is independently selected from -H and $C_{1-6}$ alkyl; optionally, each of the pair(s) $R^1/R^5$, $R^2/R^5$, $R^3/R^5$, $R^4/R^5$ and $R^5/R^6$ may independently form a chemical bond and/or are joined together with the atom to which they are attached to form a 4- to 7-membered heterocyclyl or 8- to 11-membered heterobicyclyl;

each $R^7$, $R^{7a}$ is independently selected from H and $C_{1-6}$ alkyl;

A is selected from the group consisting of indenyl, indanyl and tetralinyl;

$P^2$ is

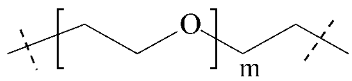

m ranges from 120 to 920, preferably from 120 to 460 and more preferably from 120 to 230;

r1, r2, r7, r8 are independently 0 or 1;

r3, r6 are independently 0, 1, 2, 3, or 4;

r4, r5 are independently 1, 2, 3, 4, 5, 6, 7, 8, 9 or 10;

s1, s2 are independently 1, 2, 3, 4, 5 or 6;

$Y^1$, $Y^2$ are identical or different and each is independently of the other selected from formulas (f-i) to (f-vi):

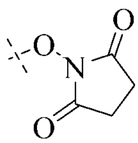 (f-i), 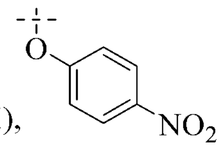 (f-ii), 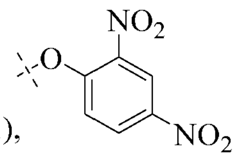 (f-iii),

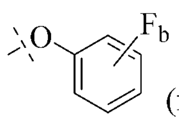 (f-iv), 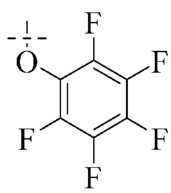 (f-v) and 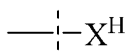 (f-vi),

wherein
the dashed lines indicate attachment to the rest of the molecule,

b is 1, 2, 3 or 4, and
$X^H$ is Cl, Br, I, or F.

Preferably, the crosslinker reagent is a compound of formula (IV-II):

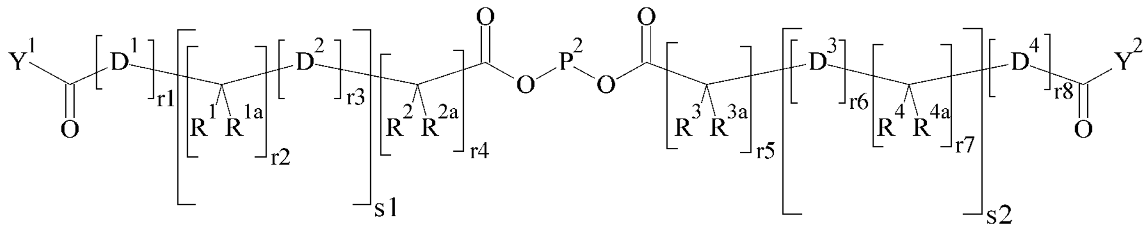

(IV-II),

wherein

| | |
|---|---|
| $D^1$, $D^2$, $D^3$ and $D^4$ | are identical or different and each is independently of the others selected from the group consisting of O, $NR^5$, S and $CR^5R^{5a}$; |
| $R^1$, $R^{1a}$, $R^2$, $R^{2a}$, $R^3$, $R^{3a}$, $R^4$, $R^{4a}$, $R^5$ and $R^{5a}$ | are identical or different and each is independently of the others selected from the group consisting of H and $C_{1-6}$ alkyl; optionally, one or more of the pair(s) $R^1/R^{1a}$, $R^2/R^{2a}$, $R^3/R^{3a}$, $R^4/R^{4a}$, $R^1/R^2$, $R^3/R^4$, $R^{1a}/R^{2a}$, and $R^{3a}/R^{4a}$ form a chemical bond or are joined together with the atom to which they are attached to form a $C_{3-8}$ cycloalkyl or to form a ring A or are joined together with the atom to which they are attached to form a 4- to 7-membered heterocyclyl or 8- to 11-membered heterobicyclyl or adamantyl; |
| A | is phenyl, naphthyl, indenyl, indanyl or tetralinyl; |
| $P^2$ | is 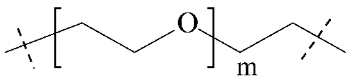 |
| m | ranges from 5 to 920, preferably from 5 to 460 and more preferably from 40 to 230; |
| r1, r2, r7, r8 | are independently 0 or 1; |
| r3, r6 | are independently 0, 1, 2, 3, or 4; |
| r4, r5 | are independently 1, 2, 3, 4, 5, 6, 7, 8, 9 or 10; |
| s1, s2 | are independently 1, 2, 3, 4, 5 or 6; |
| $Y^1$, $Y^2$ | are identical or different and each is independently of the other selected from formulas (f-i) to (f-vi): |

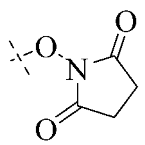 (f-i), 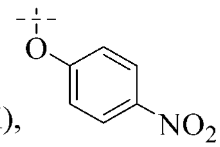 (f-ii), 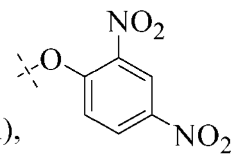 (f-iii),

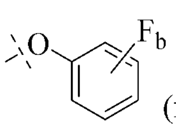 (f-iv), 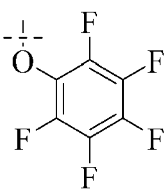 (f-v) and 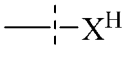 (f-vi),

wherein
the dashed lines indicate attachment to the rest of the molecule,

b is 1, 2, 3 or 4
$X^H$ is Cl, Br, I, or F.

Preferably, $Y^1$ and $Y^2$ of formula (IV-I) and (IV-II) are of formula (f-i), (f-ii) or (f-v). More preferably, $Y^1$ and $Y^2$ of formula (IV-I) and (IV-II) are of formula (f-i) or (f-ii) and most preferably, $Y^1$ and $Y^2$ of formula (IV-I) and (IV-II) are of formula (f-i).

Preferably, both moieties $Y^1$ and $Y^2$ of formula (IV-I) and (IV-II) have the same structure. More preferably, both $Y^1$ and $Y^2$ are of formula (f-i).

It is understood that the moieties

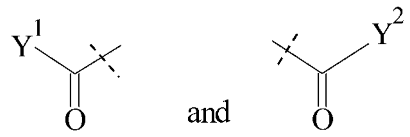

of formula (IV-I) and (IV-II) represent the at least two functional end groups.

Preferably, r1 and r8 of formula (IV-I) and (IV-II) are both 0.

Preferably, r1, r8, s1 and s2 of formula (IV-I) and (IV-II) are all 0.

Preferably, one or more of the pair(s) $R^1/R^{1a}$, $R^2/R^{2a}$, $R^3/R^{3a}$, $R^4/R^{4a}$, $R^1/R^2$, $R^3/R^4$, $R^{1a}/R^{2a}$, and $R^{3a}/R^{4a}$ of formula (IV-I) and (IV-II) form a chemical bond or are joined together with the atom to which they are attached to form a $C_{3-8}$ cycloalkyl or a ring A.

Preferably, one or more of the pair(s) $R^1/R^2$, $R^{1a}/R^{2a}$, $R^3/R^4$, $R^{3a}/R^{4a}$ of formula (IV-I) and (IV-II) are joined together with the atom to which they are attached to form a 4- to 7-membered heterocyclyl or 8- to 11-membered heterobicyclyl.

Preferably, the crosslinker reagent of formula (IV-I) and (IV-II) is symmetric, i.e. the moiety

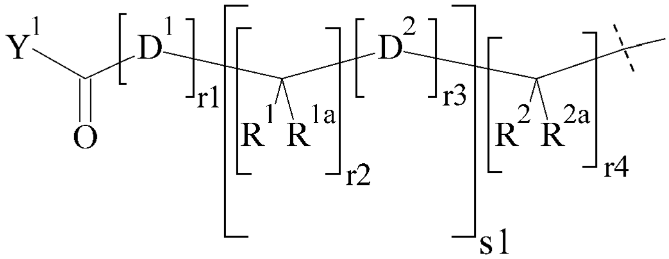

has the same structure as the moiety

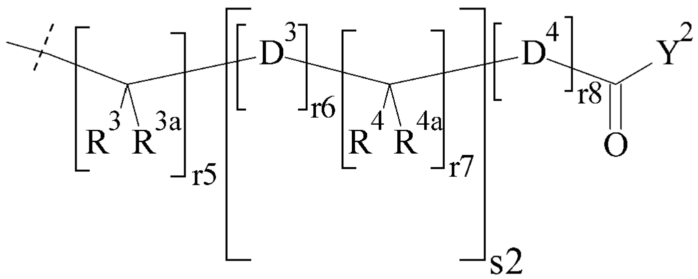

Preferred crosslinker reagents are of formula (g-i) to (g-liv):

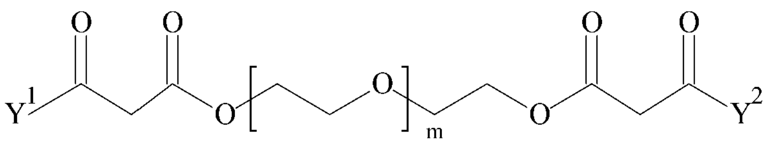

(g-i),

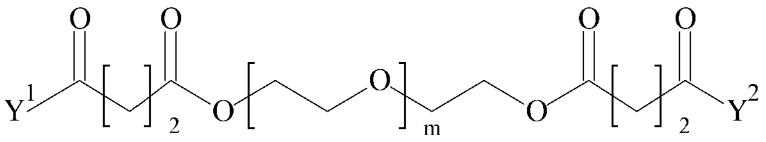

(g-ii),

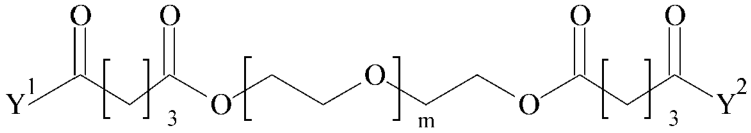

(g-iii)

,

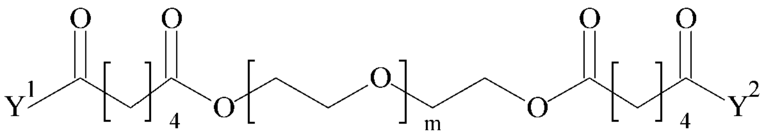

(g-iv),

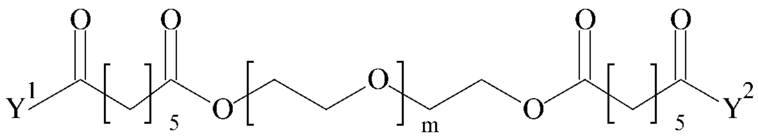

(g-v) ,

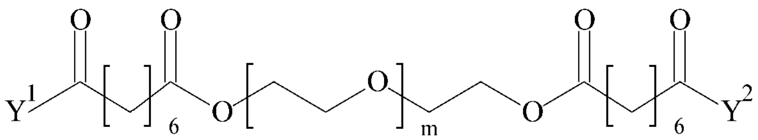

(g-vi),

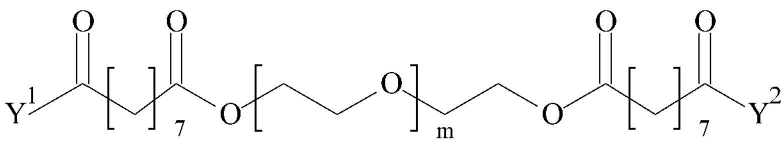

(g-vii)

,

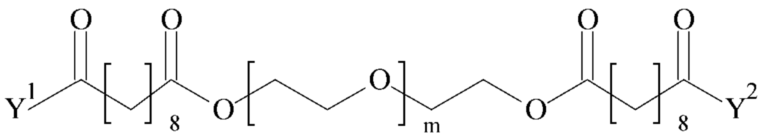

(g-viii)

,

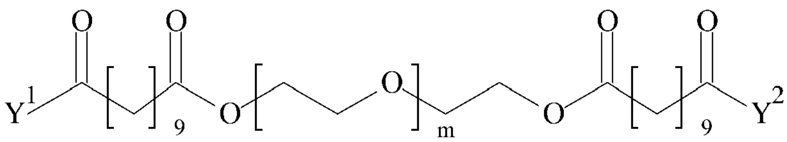

(g-ix)

,

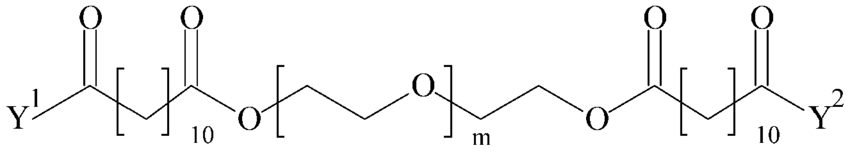

(g-x),

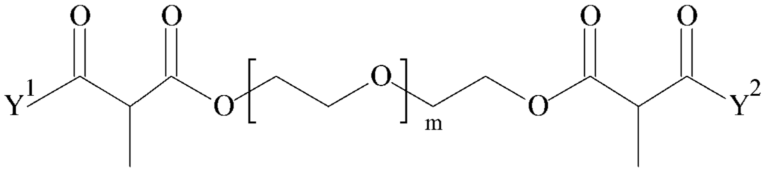

(g-xi),

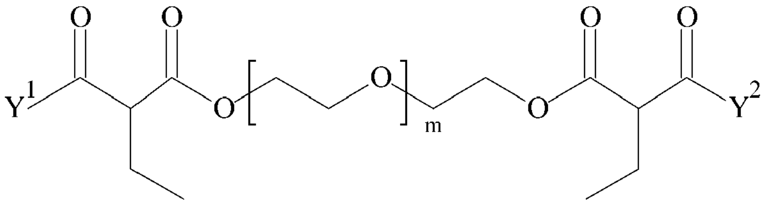

(g-xii),

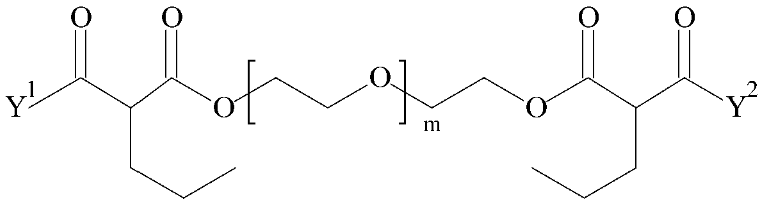

(g-xiii),

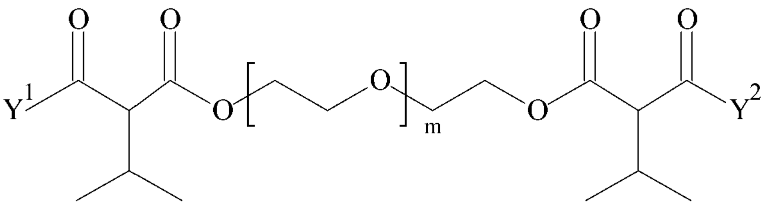

(g-xiv),

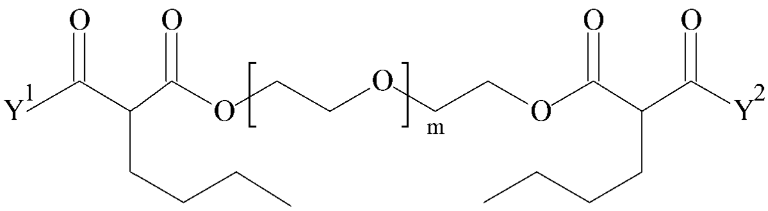

(g-xv),

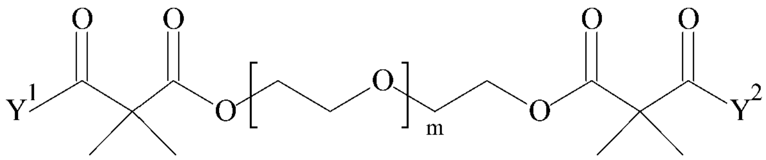

(g-xvi),

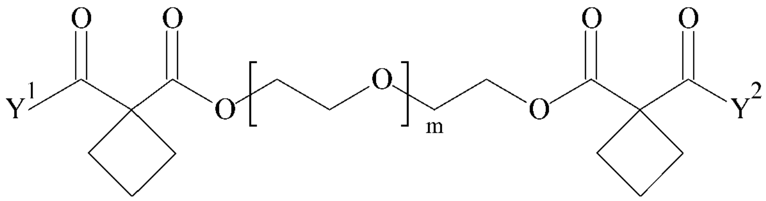

(g-xvii)

,

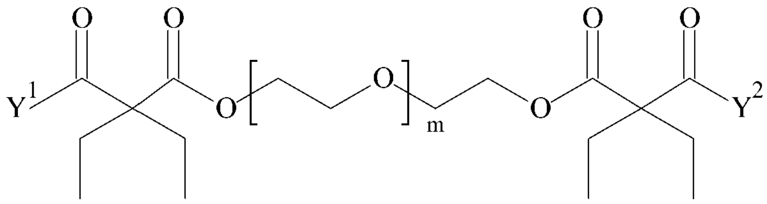

(g-xviii)

,

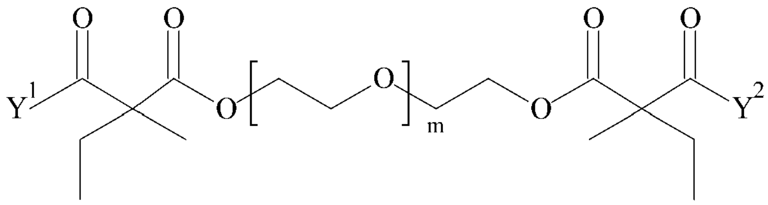

(g-xix)

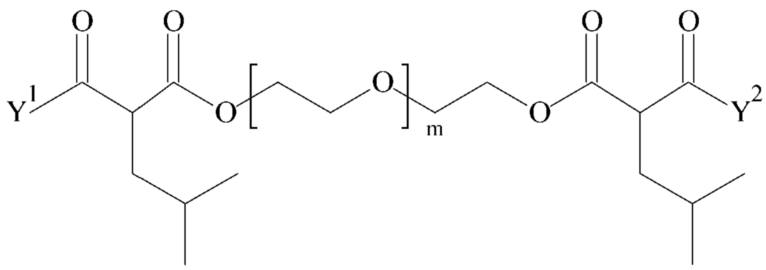

(g-xx)

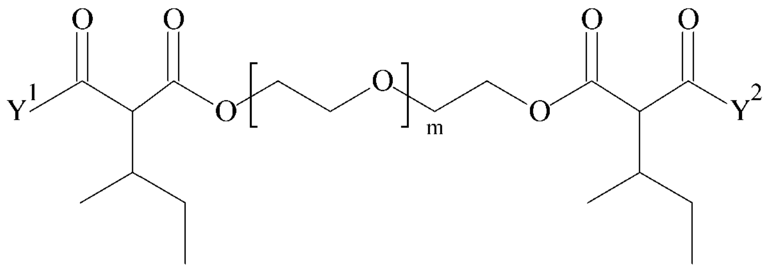

(g-xxi)

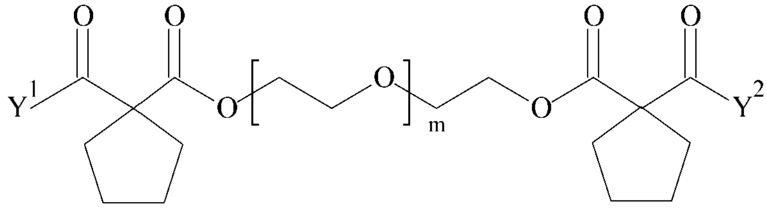

(g-xxii)

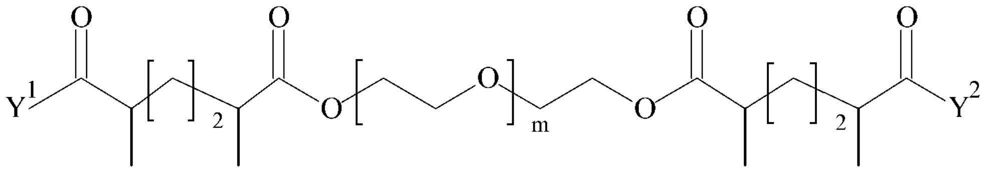

(g-xxiii)

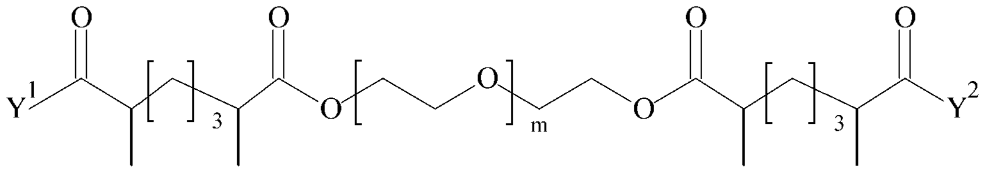

(g-xxiv)

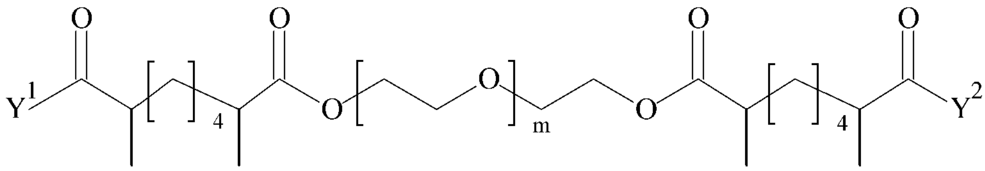

(g-xxv)

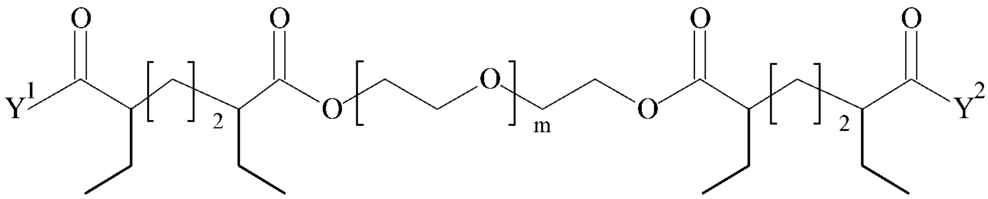

(g-xxvi)

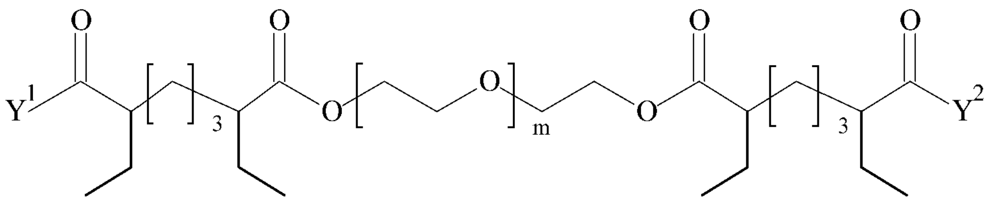

(g-xxvii)

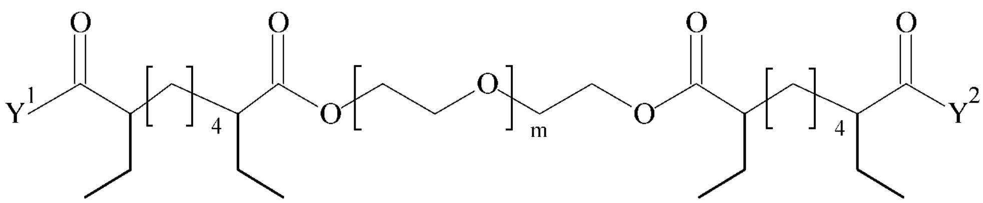

(g-xxviii)

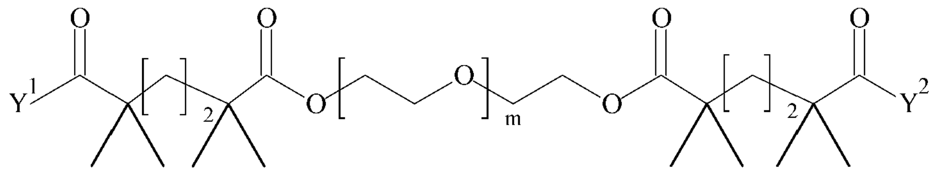

(g-xxix)

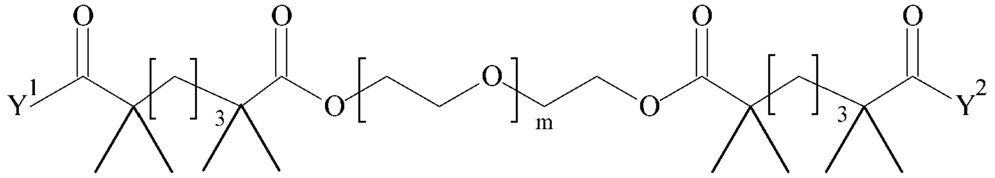

(g-xxx)

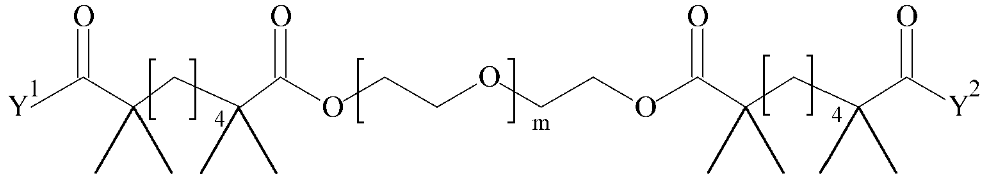

(g-xxxi)

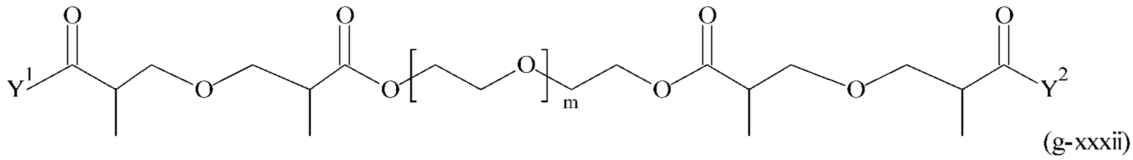

(g-xxxii)

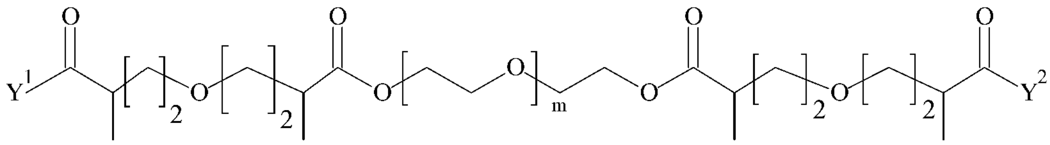

(g-xxxiii)

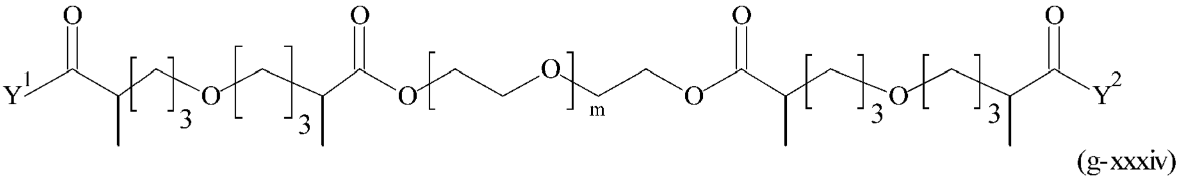

(g-xxxiv)

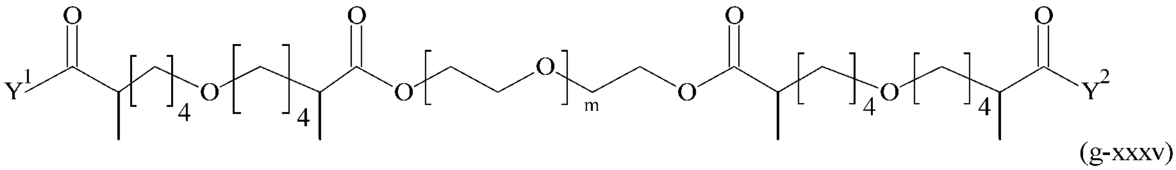

(g-xxxv)

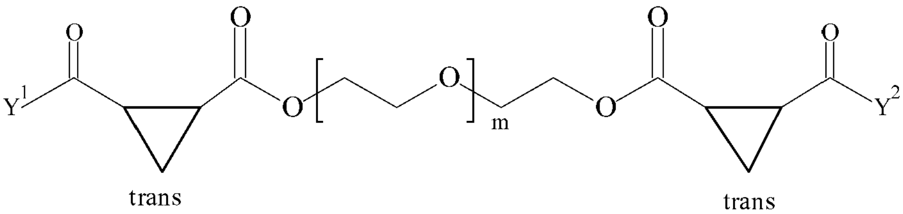

trans trans

(g-xxxvi)

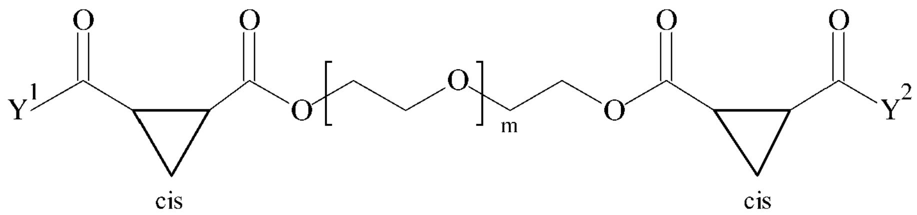

(g-xxxvii)

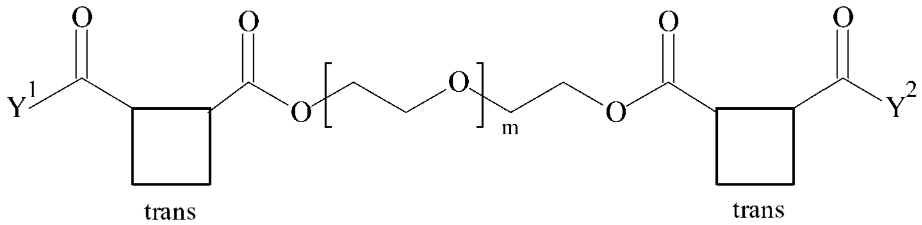

(g-xxxviii)

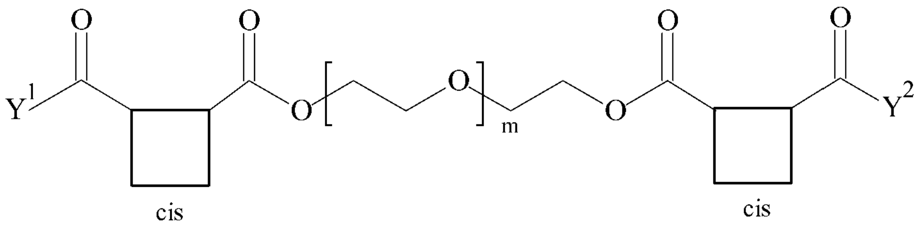

(g-xxxix)

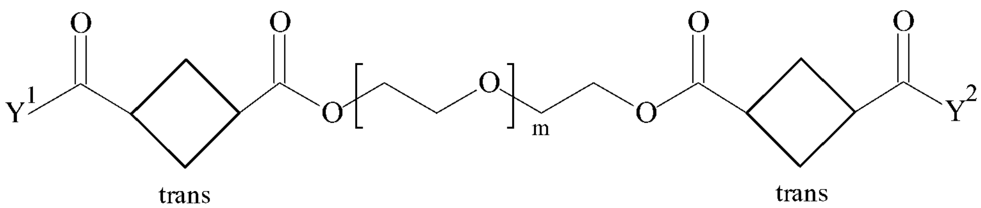

(g-xl)

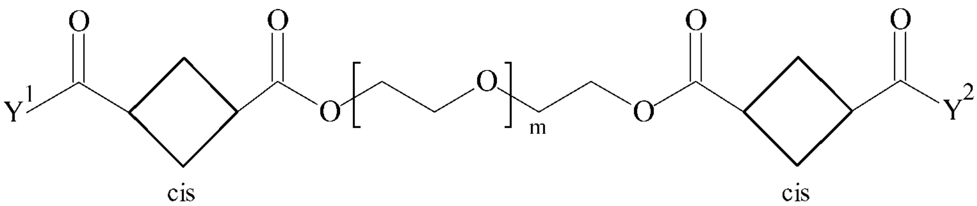

(g-xli)

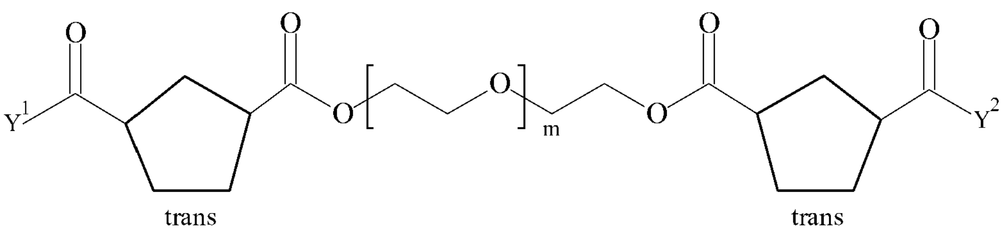

(g-xlii)

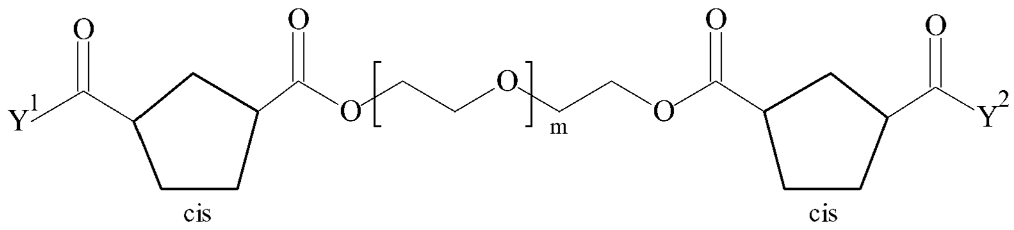

(g-xliii)

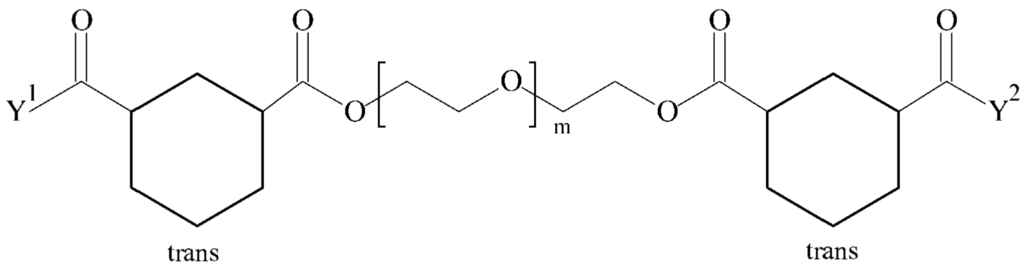

(g-xliv)

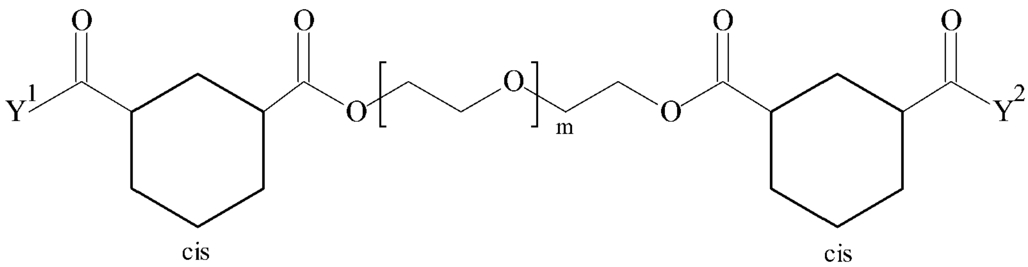

(g-xlv)

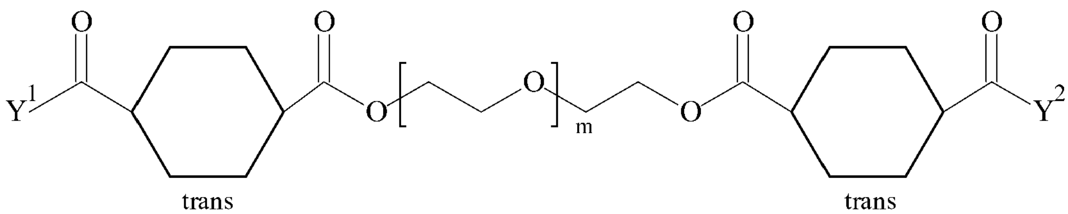

(g-xlvi)

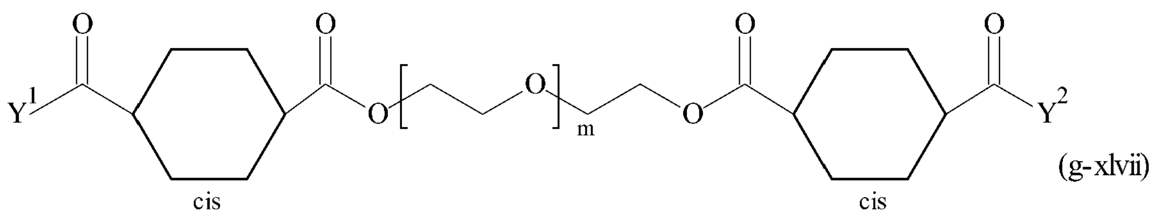

(g-xlvii)

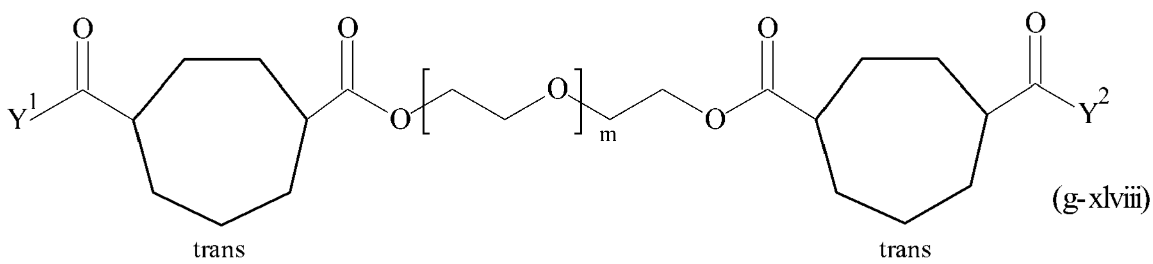

(g-xlviii)

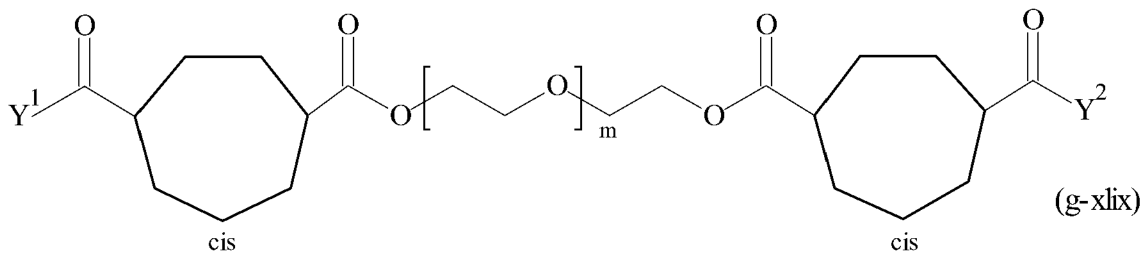

(g-xlix)

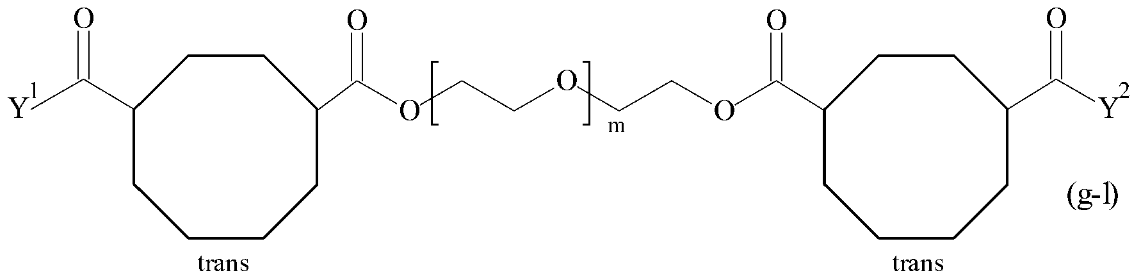

(g-l)

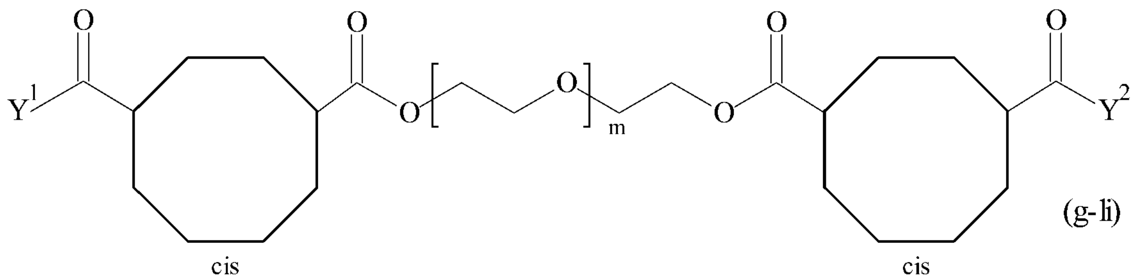

(g-li)

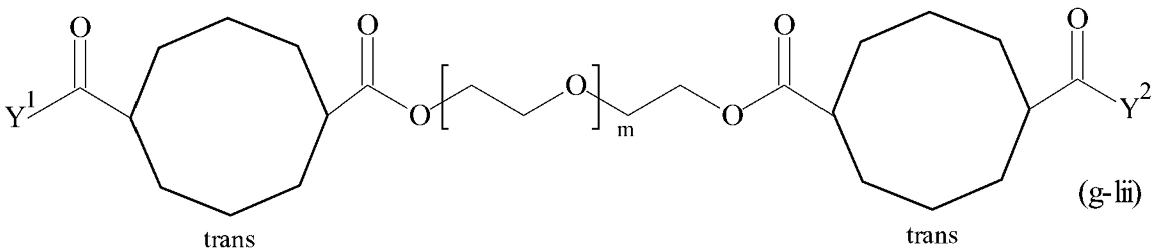

(g-lii)

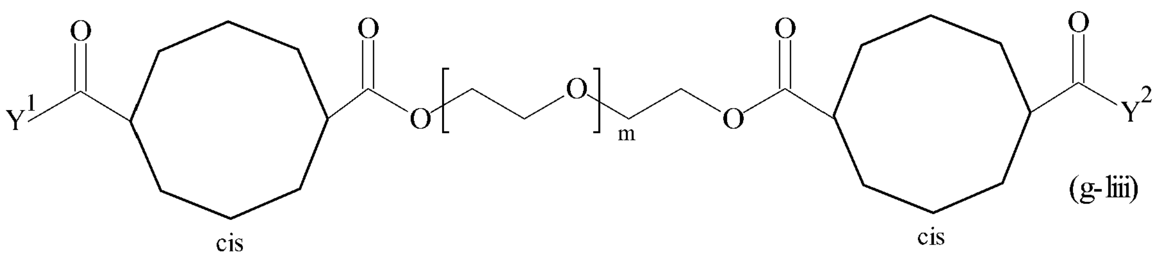

(g-liii)

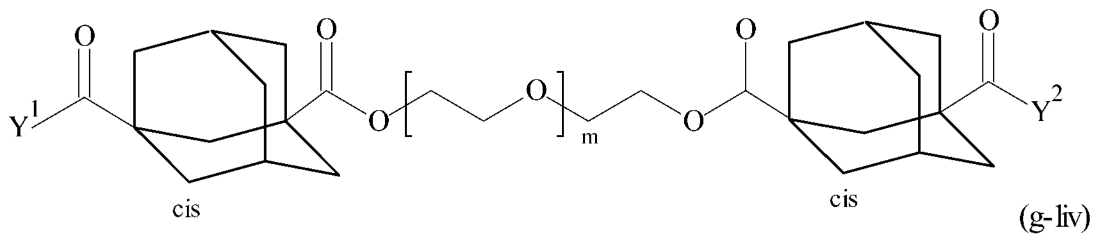

(g-liv)

wherein

each crosslinker reagent may be in the form of its racemic mixture, where applicable; and
m, $Y^1$ and $Y^2$ are defined as above.

Even more preferred crosslinker reagents are of formula (ga-1) to (ga-54):

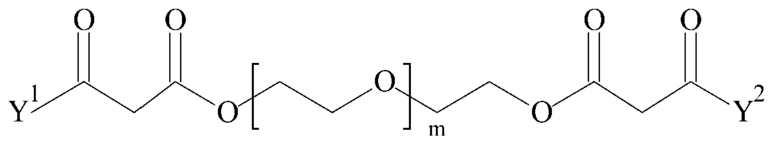

(ga-i)

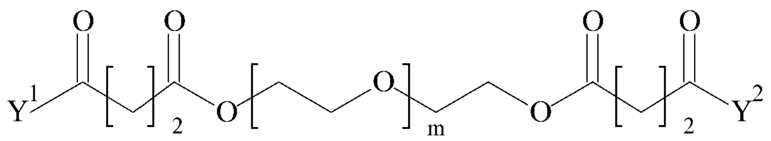

(ga-ii)

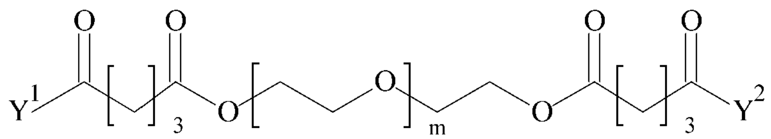

(ga-iii)

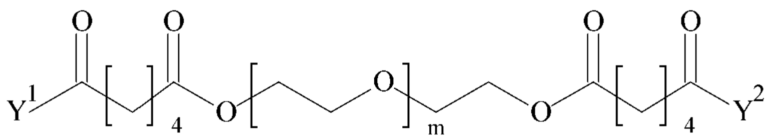

(ga-iv)

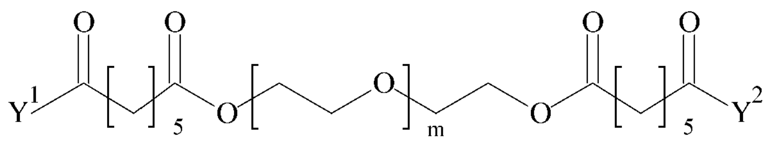

(ga-v)

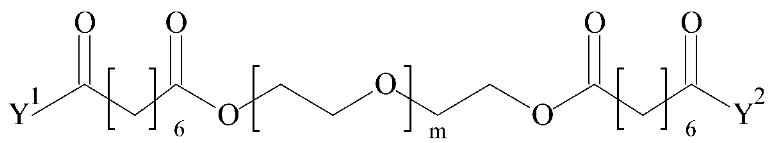

(ga-vi)

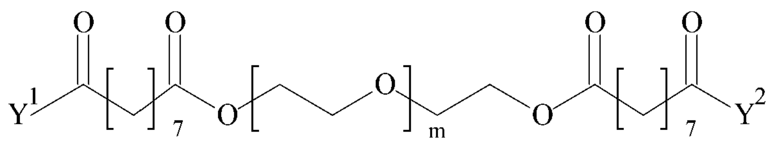

(ga-vii)

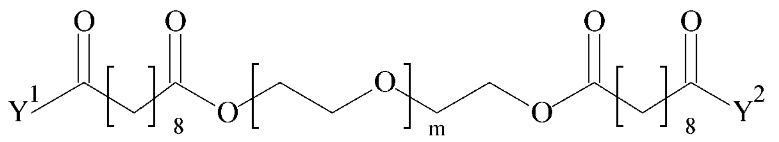

(ga-viii)

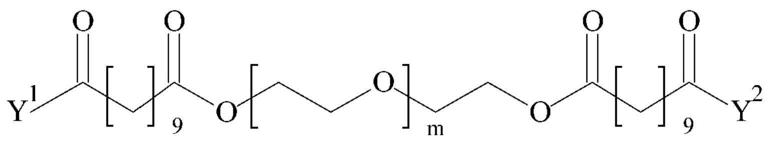

(ix)

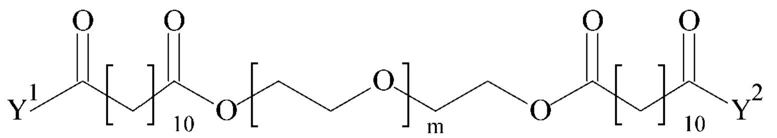

(ga-x)

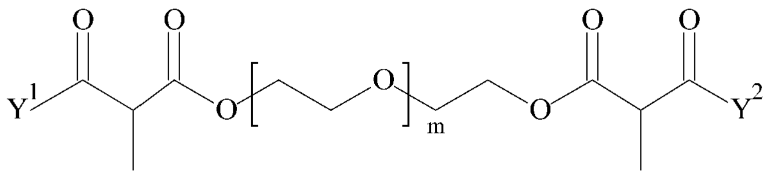

(ga-xi)

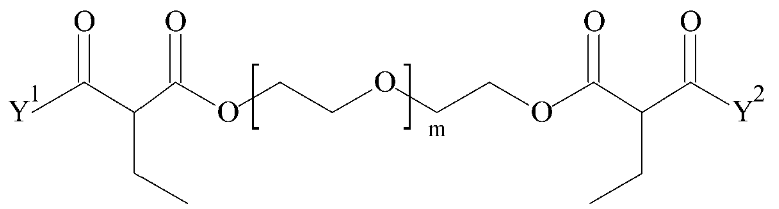

(ga-xii)

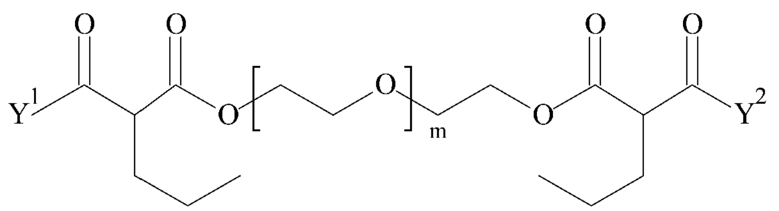

(ga-xiii)

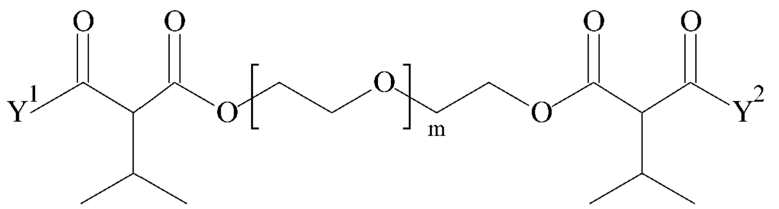

(ga-xiv)

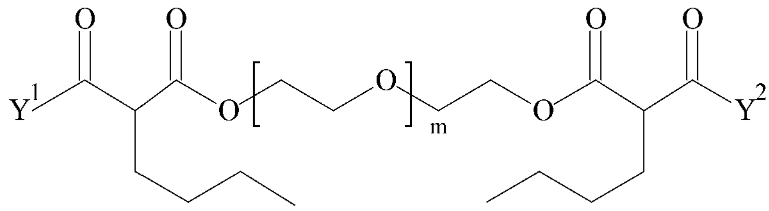

(ga-xv)

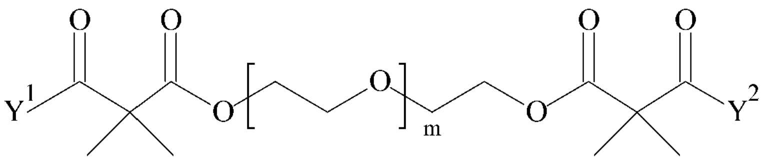

(ga-xvi)

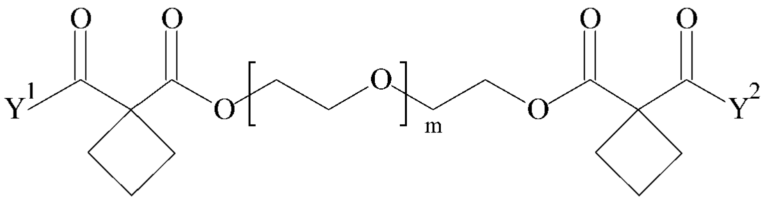

(ga-xvii)

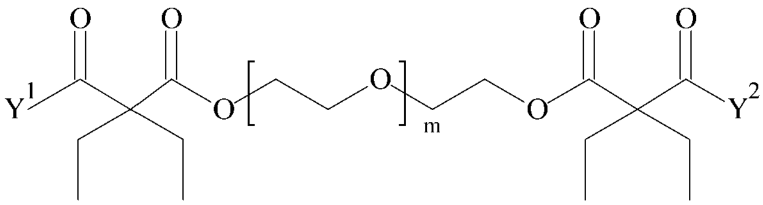

(ga-xviii)

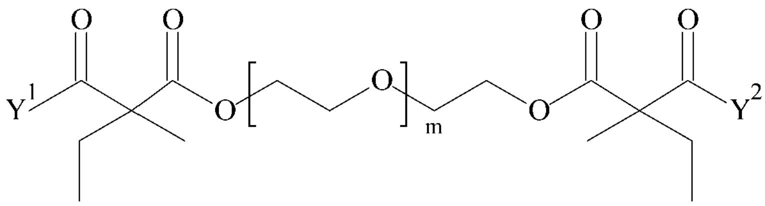

(ga-xix)

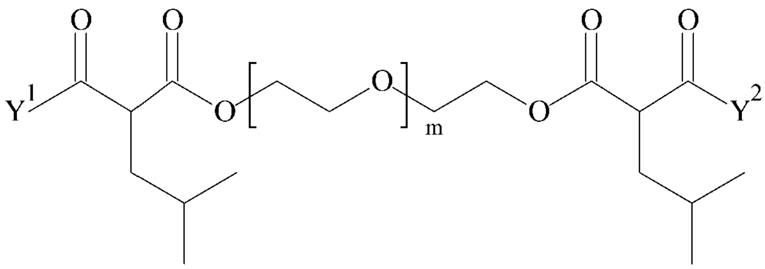

(ga-xx)

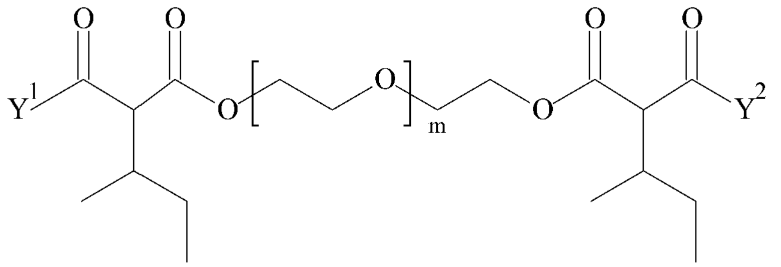

(ga-xxi)

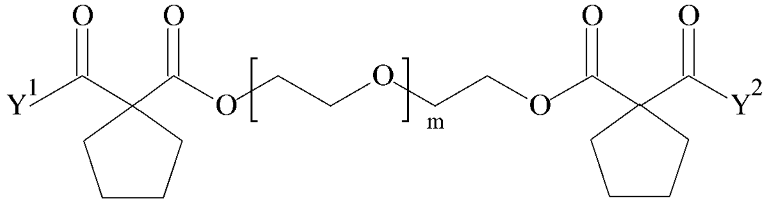

(ga-xxii)

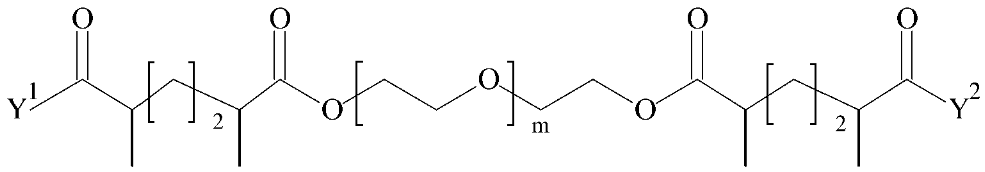

(ga-xxiii)

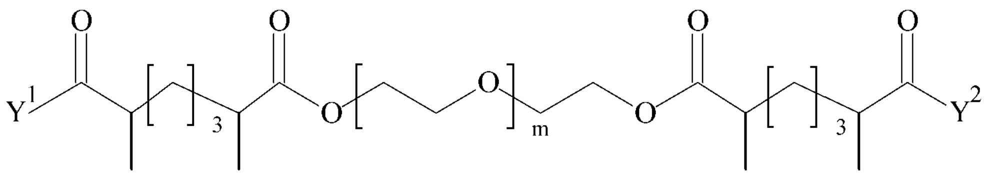

(ga-xxiv)

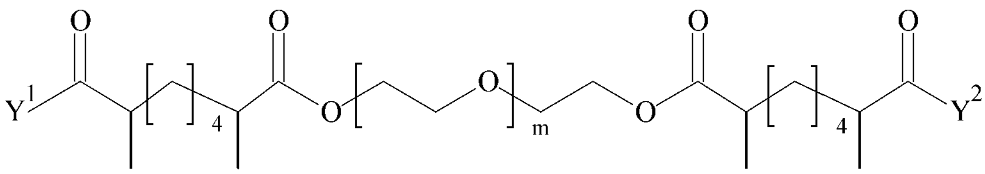

(ga-xxv)

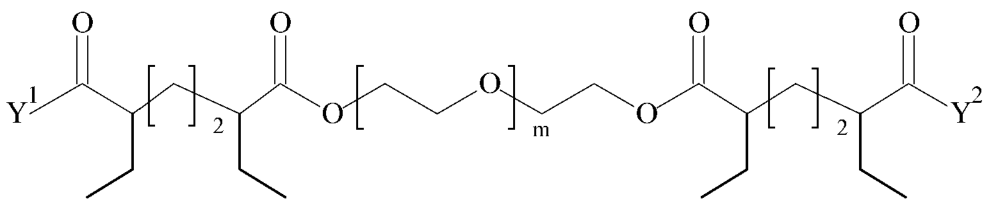

(ga-xxvi)

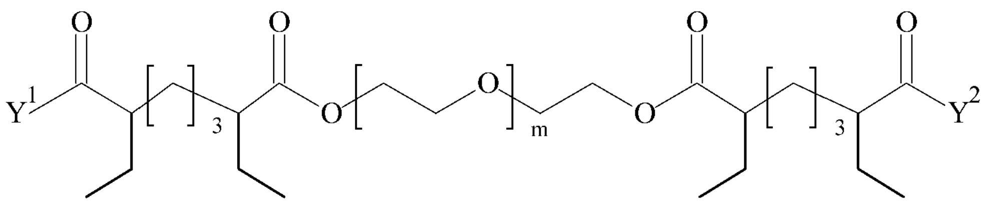

(ga-xxvii)

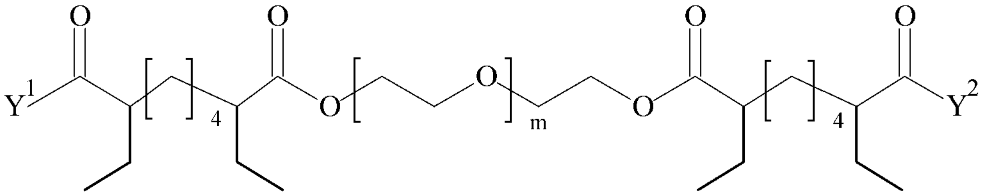

(ga-xxviii)

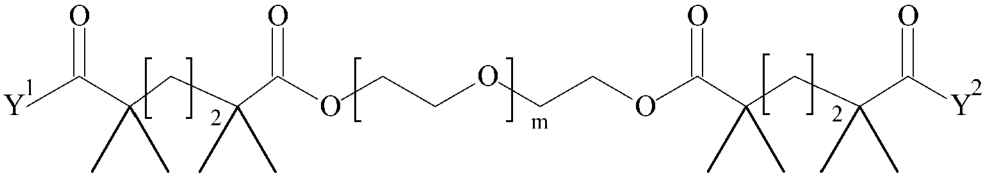

(ga-xxix)

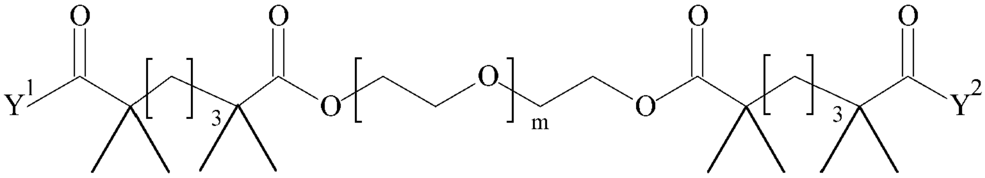

(ga-xxx)

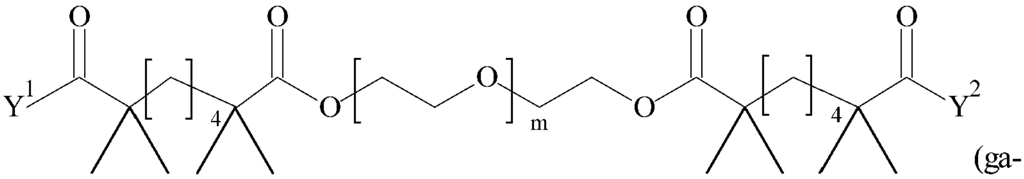

(ga-xxxi)

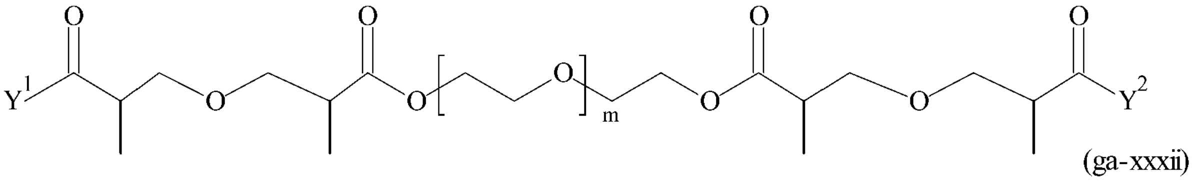

(ga-xxxii)

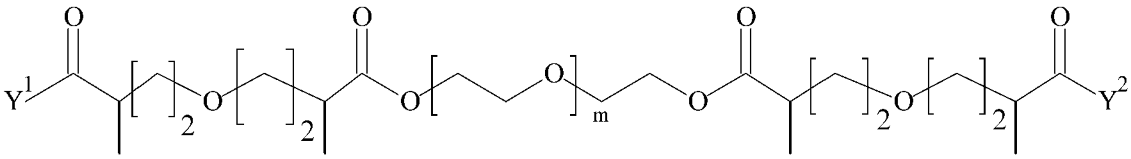

(ga-xxxiii)

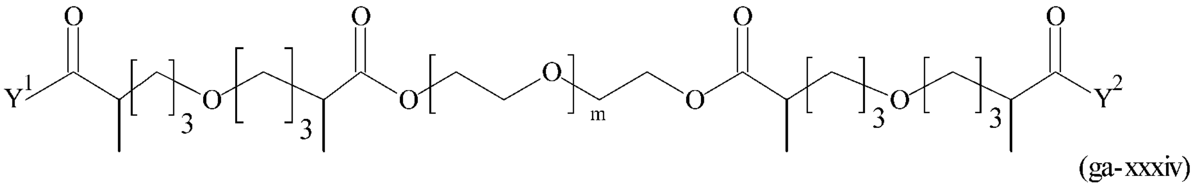

(ga-xxxiv)

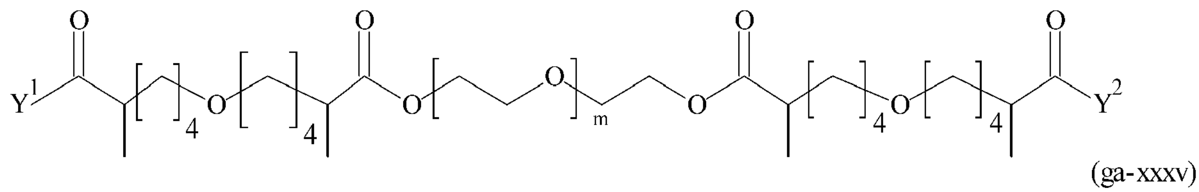

(ga-xxxv)

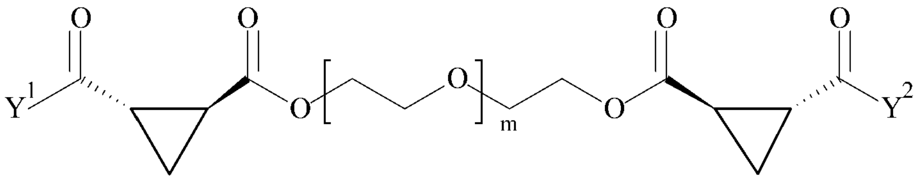

(ga-xxxvi)

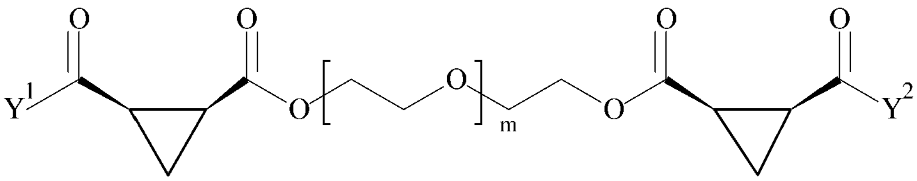

(ga-xxxvii)

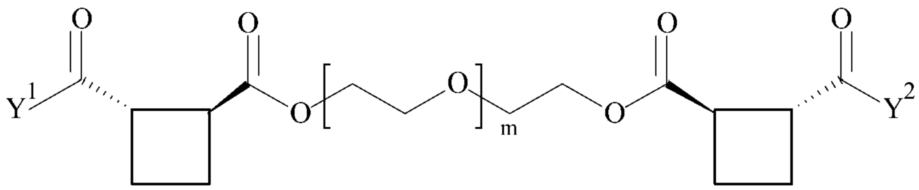

(ga-xxxviii)

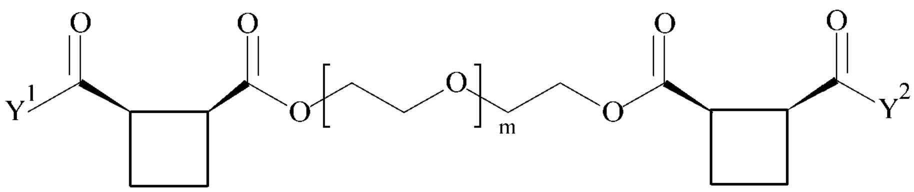

(ga-xxxix)

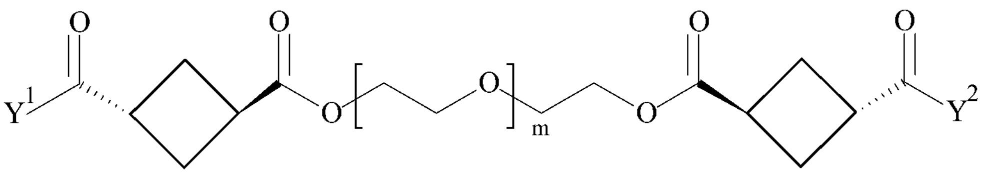

(ga-xl)

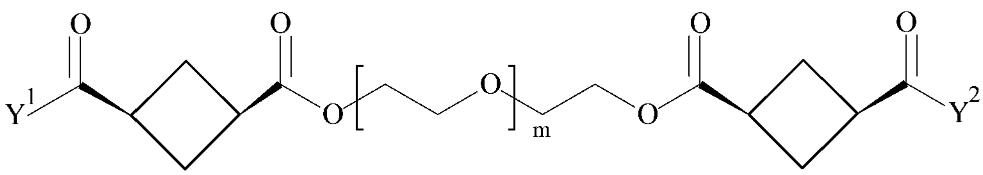

(ga-xli)

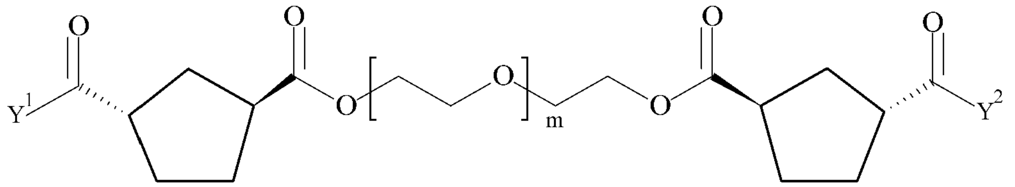

(ga-xlii)

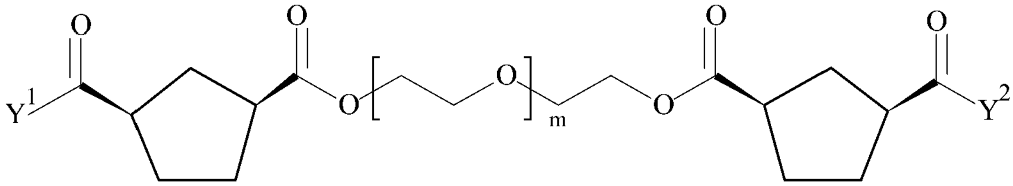

(ga-xliii)

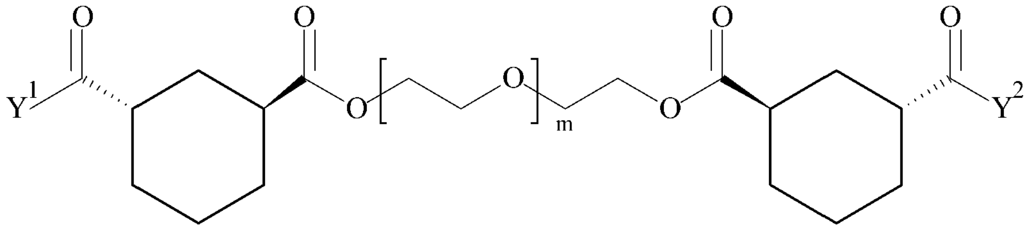

(ga-xliv)

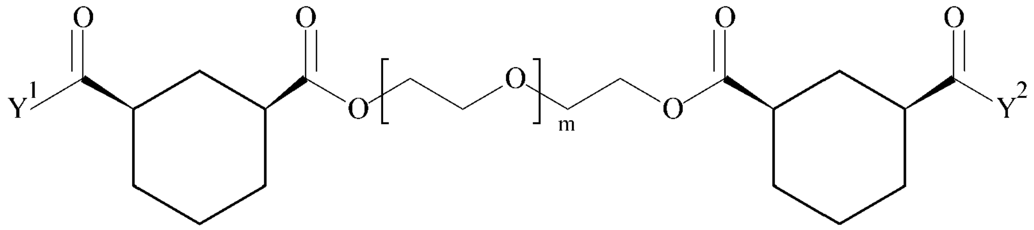

(ga-xlv)

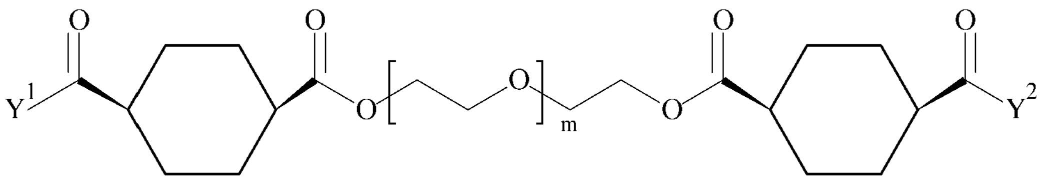

(ga-xlvi)

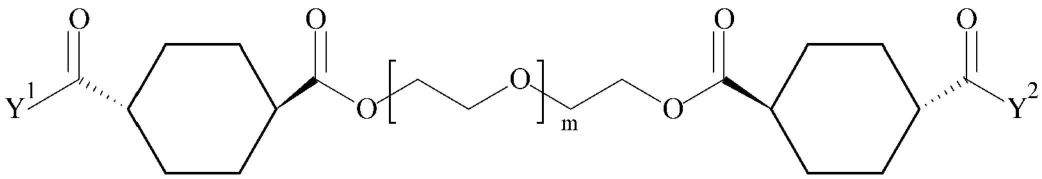

(ga-xlvii)

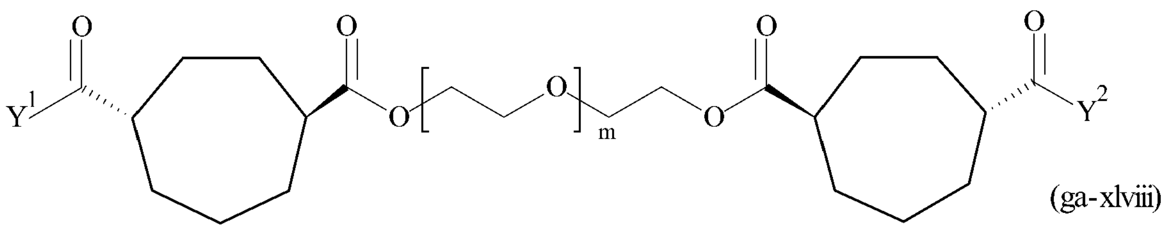

(ga-xlviii)

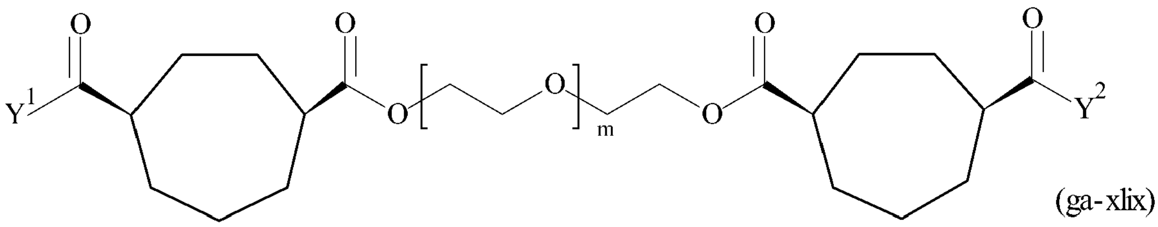

(ga-xlix)

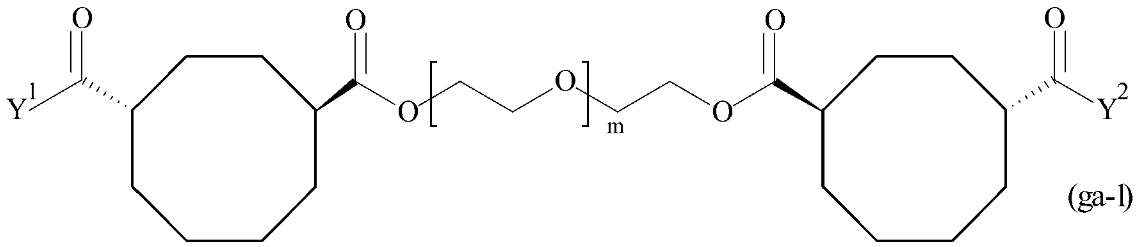

(ga-l)

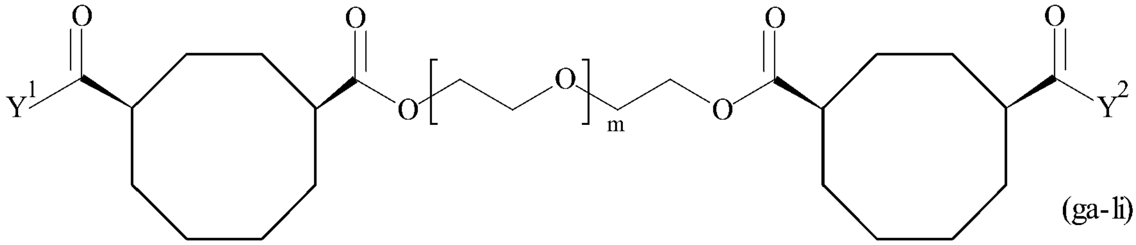

(ga-li)

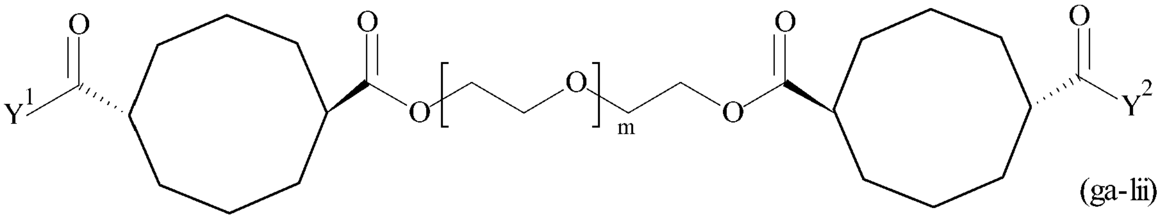

(ga-lii)

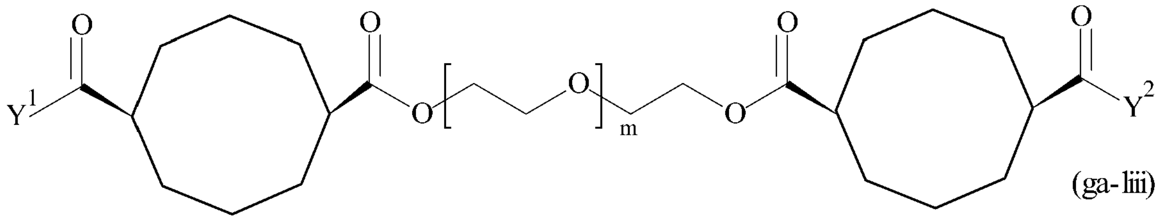

(ga-liii)

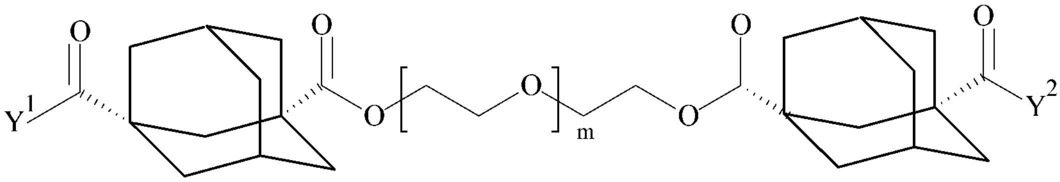

(ga-liv)

wherein

each crosslinker reagent may be in the form of its racemic mixture, where applicable; and

m, $Y^1$ and $Y^2$ are defined as above.

It was surprisingly found that the use of crosslinker reagents with branches, i.e. residues other than H, at the alpha carbon of the carbonyloxy group lead to the formation of hydrogels which are more resistant against enzymatic degradation, such as degradation through esterases.

Similarly, it was surprisingly found that the fewer atoms there are between the (C=O) of a carbonyloxy group and the (C=O) of the adjacent activated ester, activated carbamate, activated carbonate or activated thiocarbamate, the more resistant against degradation the resulting hydrogels are, such as more resistant against degradation through esterases.

In one embodiment crosslinker reagents g-i, g-ii,g-v, g-vi, g-vii, g-viii, g-ix, g-x, g-xi, g-xii, g-xiii, g-xiv, g-xv, g-xvi, g-xvii, g-xviii, g-xix, g-xx, g-xxi, g-xxii, g-xxiii, g-xxiv, g-xxv, g-xxvi, g-xxvii, g-xxviii, g-xxix, g-xxx, g-xxxi, g-xxxii, g-xxxiii, g-xxxiv, g-xxxv, g-xxxvi, g-xxvii, g-xxxviii, g-xxxix, g-xl, g-xli, g-xlii, g-xliii, g-xliv, g-xlv, g-xlvi, g-xlvii, g-xlviii, g-xlix, g-l, g-li, g-lii, g-liii and g-liv are preferred crosslinker reagents. More preferably, the at least one crosslinker reagent is of formula g-v, g-vi, g-vii, g-viii, g-ix, g-x, g-xiv, g-xxxii, g-xxxiii, g-xliii, g-xliv, g-xlv or g-xlvi, and even more preferably, the at least one crosslinker reagent is of formula g-v, g-vi, g-ix or g-xiv. Most preferably, the at least one crosslinker reagent is of formula g-xiv.

In another embodiment crosslinker reagents ga-i, ga-ii,ga-v, ga-vi, ga-vii, ga-viii, ga-ix, ga-x, ga-xi, ga-xii, ga-xiii, ga-xiv, ga-xv, ga-xvi, ga-xvii, ga-xviii, ga-xix, ga-xx, ga-xxi, ga-xxii, ga-xxiii, ga-xxiv, ga-xxv, ga-xxvi, ga-xxvii, ga-xxviii, ga-xxix, ga-xxx, ga-xxxi, ga-xxxii, ga-xxxiii, ga-xxxiv, ga-xxxv, ga-xxxvi, ga-xxvii, ga-xxxviii, ga-xxxix, ga-xl, ga-xli, ga-xlii, ga-xliii, ga-xliv, ga-xlv, ga-xlvi, ga-xlvii, ga-xlviii, ga-xlix, ga-l, ga-li, ga-lii, ga-liii and ga-liv are preferred crosslinker reagents. More preferably, the at least one crosslinker reagent is of formula ga-v, ga-vi, ga-vii, ga-viii, ga-ix, ga-x, ga-xiv, ga-xxxii, ga-xxxiii, ga-xliii, ga-xliv, ga-xlv or ga-xlvi, and even more preferably, the at least one crosslinker reagent is of formula ga-v, ga-vi, ga-ix or ga-xiv. Most preferably, the at least one crosslinker reagent is of formula ga-xiv.

The preferred embodiments of the compound of formula of formula (IV-I) and (IV-II) as mentioned above apply accordingly to the preferred compounds of formulas (g-i) to (g-liv). The hydrogel resulting from step (a-ii) preferably contains from 0.01 to 1.2 mmol/g primary amine groups ($-NH_2$), more preferably from 0.02 to 1.0 mmol/g primary amine groups, even more preferably from 0.02 to 0.5 mmol/g primary amine groups and most preferably from 0.05 to to 0.3 mmol/g primary amine groups, if it is to be used as a carrier in a hydrogel-linked prodrug of a protein drug. If the hydrogel of the present invention is to be used as a carrier in a hydrogel-linked prodrug of a small molecule, it preferably contains from 0.01 to 2 mmol/g primary amine groups, more preferably from 0.02 to 1.8 mmol/g primary amine groups, even most preferably from 0.05 to 1.5 mmol/g primary amine groups.

More preferably, the hydrogel resulting from step (a-ii) preferably contains from 0.01 to 1.2 mmol/g primary amine groups ($-NH_2$), more preferably from 0.02 to 1.0 mmol/g primary amine groups, even more preferably from 0.02 to 0.5 mmol/g primary amine groups and most preferably from 0.05 to to 0.3 mmol/g primary amine groups,

The term "X mmol/g primary amine groups" means that 1 g of dry hydrogel comprises X mmol primary amine groups. Measurement of the amine content of the hydrogel is carried out according to Gude et al. (Letters in Peptide Science, 2002, 9(4): 203-206, which is incorpated by reference in its entirety) and is also described in detail in the Examples section.

Preferably, the term "dry" as used herein means having a residual water content of a maximum of 10 %, preferably less than 5% and more preferably less than 2% (determined according to Karl Fischer). The preferred method of drying is lyophilization.

**Polymerization of step (a-ii)**

In one embodiment the polymerization in step (a-ii) is initiated by adding a base. Preferably, the base is a non-nucleophilic base soluble in alkanes, more preferably the base is selected from the group consisting of N,N,N',N'-tetramethylethylene diamine (TMEDA), 1,4-dimethylpiperazine, 4-methylmorpholine, 4-ethylmorpholine, 1,4-diazabicyclo[2.2.2]octane, 1,1,4,7,10,10-hexamethyltriethylenetetramine, 1,4,7-trimethyl-1,4,7-triazacyclononane, tris[2-(dimethylamino)ethyl]amine, triethylamine, diisopropylethylamine (DIPEA), trimethylamine, N,N-dimethylethylamine, N,N,N',N'-tetramethyl-1,6-hexanediamine, N,N,N',N",N"-pentamethyldiethylenetriamine, 1,8-diazabicyclo[5.4.0]undec-7-ene, 1,5-diazabicyclo[4.3.0]non-5-ene, and hexamethylenetetramine. Even more preferably, the base is selected from TMEDA, 1,4-dimethylpiperazine, 4-methylmorpholine, 4-ethylmorpholine, 1,4-diazabicyclo[2.2.2]octane, 1,1,4,7,10,10-hexamethyltriethylenetetramine, 1,4,7-trimethyl-1,4,7-triazacyclononane, tris[2-(dimethylamino)ethyl]amine, 1,8-diazabicyclo[5.4.0]undec-7-ene, 1,5-diazabicyclo[4.3.0]non-5-ene, and hexamethylenetetramine. Most preferably, the base is TMEDA.

The base to initiate the polymerization in step (a-ii) is added preferably in an amount of 1 to 500 equivalents per activated functional end group in the mixture, more preferably in an amount of 5 to 50 equivalents, even more preferably in an amount of 5 to 25 equivalents and most preferably in an amount of 10 equivalents.

Preferably, the polymerization of step (a-ii) is a condensation reaction, which preferably occurs under continuous stirring of the mixture of step (a). Preferably, the tip speed (tip speed = $\pi \times$ stirrer rotational speed $\times$ stirrer diameter) ranges from 0.2 to 10 meter per second (m/s), more preferably from 0.5 to 4 m/s and most preferably from 1 to 2 m/s.

In a preferred embodiment of step (a-ii), the polymerization reaction is carried out in a cylindrical vessel equipped with baffles. The diameter to height ratio of the vessel preferably ranges from 4:1 to 1:2, more preferably the diameter to height ratio of the vessel ranges from 2:1 to 1:1.

Preferably, the reaction vessel is equipped with an axial flow stirrer selected from the group consisting of pitched blade stirrers, marine type propellers, and Lightnin A-310. More preferably, the stirrer is a pitched blade stirrer.

Step (a-ii) can be performed in a broad temperature range, preferably at a temperature from -10°C to 100 C°, more preferably at a temperature of 0°C to 80°C, even more preferably at a temperature of 10°C to 50 °C and most preferably at ambient temperature.

"Ambient temperature" refers to the temperature present in a typical laboratory environment and preferably means a temperature ranging from 17 to 25°C.

In one preferred embodiment the polymerization in step (a-ii) occurs in a suspension polymerization, in which case the mixture of step (a-ii) further comprises a first solvent and at least a second solvent, which second solvent is immiscible in the first solvent.

Said first solvent is preferably selected from the group consisting of dichloromethane, chloroform, tetrahydrofuran, ethyl acetate, dimethylformamide, acetonitrile, dimethyl sulfoxide, propylene carbonate, N-methylpyrrolidone, methanol, ethanol, isopropanol, water and mixtures thereof.

The at least one backbone reagent and at least one crosslinker reagent are dissolved in the first solvent, i.e. the disperse phase of the suspension polymerization. In one embodiment the at least one backbone reagent and the at least one crosslinker reagent are dissolved separately, i.e. in different containers, using either the same or different solvent and preferably using the same solvent for both reagents. In another embodiment, the at least one backbone reagent and the at least one crosslinker reagent are dissolved together, i.e. in the same container and using the same solvent.

A suitable solvent for the at least one backbone reagent is an organic solvent. Preferably, the solvent is selected from the group consisting of dichloromethane, chloroform, tetrahydrofuran, ethyl acetate, dimethylformamide, acetonitrile, dimethyl sulfoxide, propylene carbonate, N-methylpyrrolidone, methanol, ethanol, isopropanol, water and mixtures thereof. More preferably, the backbone reagent is dissolved in a solvent selected from acetonitrile, dimethyl sulfoxide, methanol and mixtures thereof. Most preferably, the backbone reagent is dissolved in dimethylsulfoxide.

In one embodiment the at least one backbone reagent is dissolved in the solvent in a concentration ranging from 1 to 300 mg/ml, more preferably from 5 to 60 mg/ml and most preferably from 10 to 40 mg/ml.

A suitable solvent for the at least one crosslinker reagent is an organic solvent. Preferably, the solvent is selected from the group consisting of dichloromethane, chloroform, tetrahydrofuran, ethyl acetate, dimethylformamide, acetonitrile, dimethyl sulfoxide, propylene carbonate, N-methylpyrrolidone, methanol, ethanol, isopropanol, water and mixtures thereof. More preferably, the crosslinker reagent is dissolved in a solvent selected from dimethylformamide, acetonitrile, dimethyl sulfoxide, methanol and mixtures thereof. Most preferably, the crosslinker reagent is dissolved in dimethylsulfoxide.

In one embodiment the at least one crosslinker reagent is dissolved in the solvent in a concentration ranging from 5 to 500 mg/ml, more preferably from 25 to 300 mg/ml and most preferably from 50 to 200 mg/ml.

The at least one backbone reagent and the at least one crosslinker reagent are mixed in a weight ratio ranging from 1:99 to 99:1, e.g. in a ratio ranging from 2:98 to 90:10, in a weight ratio ranging from 3:97 to 88:12, in a weight ratio ranging from 3:96 to 85:15, in a weight ratio ranging from 2:98 to 90:10 and in a weight ratio ranging from 5:95 to 80:20; particularly preferred in a weight ratio from 5:95 to 80:20, wherein the first number refers to the at least one backbone reagent and the second number to the at least one crosslinker reagent.

The ratios are selected such that the mixture of step (a-i) comprises a molar excess of functional groups $A^{x0}$ from the at least one backbone reagent compared to the functional end groups of the at least one crosslinker reagent. Consequently, the hydrogel resulting from the process of the present invention has free funcationl groups $A^{x0}$ groups which can be used to couple other moieties to the hydrogel, such as spacer moieties and/or reversible prodrug linker moieties.

The at least one second solvent, i.e. the continuous phase of the suspension polymerization, is preferably an organic solvent, more preferably an organic solvent selected from the group consisting of linear, branched or cyclic $C_{5-30}$ alkanes; linear, branched or cyclic $C_{5-30}$ alkenes; linear, branched or cyclic $C_{5-30}$ alkynes; linear or cyclic poly(dimethylsiloxanes); aromatic $C_{6-20}$ hydrocarbons; and mixtures thereof. Even more preferably, the at least second solvent is selected from the group consisting of linear, branched or cyclic $C_{5-16}$ alkanes; toluene; xylene; mesitylene; hexamethyldisiloxane; and mixtures thereof. Most preferably, the at least second solvent is a linear $C_{7-11}$ alkane, such as heptane, octane, nonane, decane or undecane.

Preferably, the mixture of step (a-i) further comprises a detergent. Preferred detergents are Cithrol DPHS,

Hypermer 70A, Hypermer B246, Hypermer 1599A, Hypermer 2296, and Hypermer 1083.

Preferably, the detergent has a concentration of 0.1 g to 100 g per 1 L total mixture, i.e. disperse phase and continous phase together. More preferably, the detergent has a concentration of 0.5 g to 10 g per 1 L total mixture, and most preferably, the detergent has a concentration of 0.5 g to 5 g per 1 L total mixture.

Preferably, the mixture of step (a-i) is an emulsion.

**Optional step (a-iii)**

Optional step (a-iii) comprises one or more of the following steps:

(a-iiia) removing excess liquid from the polymerization reaction,
(a-iiib) washing the hydrogel to remove solvents used during polymerization,
(a-iiic) transferring the hydrogel into a buffer solution,
(a-iiid) size fractionating/sieving of the hydrogel,
(a-iiie) transferring the hydrogel into a container,
(a-iiif) drying the hydrogel,
(a-iiig) transferring the hydrogel into a specific solvent suitable for sterilization, and/or
(a-iiih) sterilizing the hydrogel, preferably by gamma radiation.

Preferably, the working-up step comprises all of the following steps

(a-iiia) removing excess liquid from the polymerization reaction,
(a-iiib) washing the hydrogel to remove solvents used during polymerization,
(a-iiic) transferring the hydrogel into a buffer solution,
(a-iiid) size fractionating/sieving of the hydrogel,
(a-iiie) transferring the hydrogel into a container,
(a-iiig) transferring the hydrogel into a specific solvent suitable for sterilization, and
(a-iiih) sterilizing the hydrogel, preferably by gamma radiation.

**Optional step (b)**

In a preferred embodiment $A^{x0}$ is an amine and $A^{x1}$ is $ClSO_2$-, R’(C=O)-, I-, Br-, Cl-, SCN-, CN-, O=C=N-, $Y^1$-(C=O)-, $Y^1$-(C=O)-NH-, or $Y^1$-(C=O)-O-,
wherein

$R^1$ is H, $C_{1-6}$ alkyl, $C_{2-6}$ alkenyl, $C_{2-6}$ alkynyl, $C_{3-8}$ cycloalkyl, 4- to 7-membered heterocyclyl, 8- to 11-membered heterobicyclyl, phenyl, naphthyl, indenyl, indanyl, or tetralinyl; and
$Y^1$ is selected from formulas (f-i) to (f-vi):

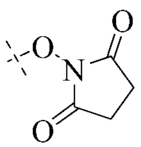 (f-i), 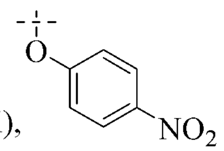 (f-ii), 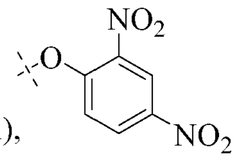 (f-iii),

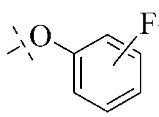 (f-iv), 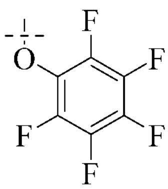 (f-v) and 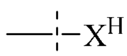 (f-vi),

wherein
the dashed lines indicate attachment to the rest of the molecule,

b is 1, 2, 3 or 4, and
$X^H$ is Cl, Br, I, or F.

In another preferred embodiment $A^{x0}$ is a hydroxyl group (-OH) and $A^{x1}$ is O=C=N-, I-, Br-, SCN-, or $Y^1$-(C=O)-NH-,
wherein $Y^1$ is selected from formulas (f-i) to (f-vi):

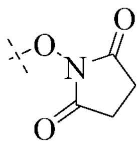 (f-i), 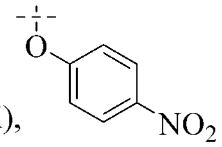 (f-ii), 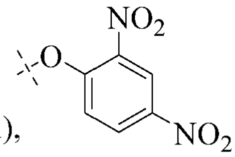 (f-iii),

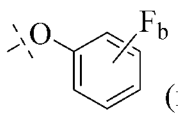 (f-iv), 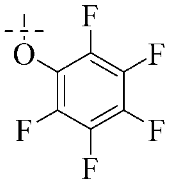 (f-v) and 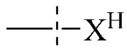 (f-vi),

wherein
the dashed lines indicate attachment to the rest of the molecule,

b is 1, 2, 3 or 4, and
$X^H$ is Cl, Br, I, or F.

In another preferred embodiment $A^{x0}$ is a carboxylic acid (-(C=O)OH) and $A^{x1}$ is a primary amine or secondary amine.
In another preferred embodiment $A^{x0}$ is a maleimide and $A^{x1}$ is a thiol.
More preferably, $A^{x0}$ is an amine and $A^{x1}$ is $Y^1$-(C=O)-, $Y^1$-(C=O)-NH-, or $Y^1$-(C=O)-O- and most preferably $A^{x0}$ is an amine and $A^{x1}$ is $Y^1$-(C=O)-.
$A^{x1}$ may optionally be present in protected form.
Suitable activating reagents to obtain the activated carboxylic acid are for example N,N'-dicyclohexyl-carbodiimide (DCC), 1-ethyl-3-carbodiimide (EDC), benzotriazol-1-yl-oxytripyrrolidinophosphonium hexafluorophosphate (PyBOP), bromotripyrrolidinophosphonium hexafluorophosphate (PyBrOP), 1-cyano-2-ethoxy-2-oxoethylidenaminooxy)dimethylamino-morpholino-carbenium hexafluorophosphate (COMU), 1-hydroxybenzotriazole (HOBT), 1-hydroxy-7-azabenzotriazole (HOAT), *O*-(6-chlorobenzotriazol-1-yl)-*N,N,N',N'*-tetramethyluronium hexafluorophosphate (HCTU), 1-H-benzotriazolium (HBTU), (*O*-(7-azabenzotriazol-1-yl)-*N,N,N',N'*-tetramethyluronium hexafluorophosphate (HATU), and *O*-(benzotriazol-l-yl)-*N,N,N',N'*-tetramethyluronium tetrafluoroborate (TBTU). These reagents are commercially available and well-known to the skilled person.
Preferably, $A^{x2}$ is selected from the group consisting of -maleimide, -SH, $-NH_2$, -SeH, $-N_3$, -C=CH, $-CR^1=CR^{1a}R^{1b}$, -OH, -(CH=X)-$R^1$, -(C=O)-S-R', -(C=O)-H, $-NH-NH_2$, $-O-NH_2$, -Ar-$X^0$, -Ar-Sn($R^1$)($R^{1a}$)($R^{1b}$), -Ar-B(OH)(OH), Br, I, $Y^1$-(C=O)-, $Y^1$-(C=O)-NH-, $Y^1$-(C=O)-O-,

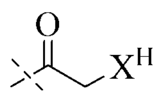, 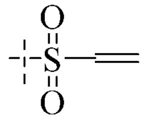, 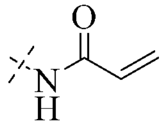, 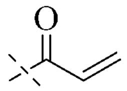,

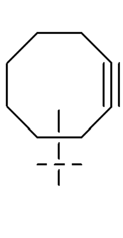, 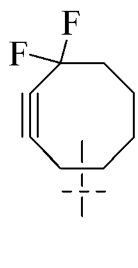, 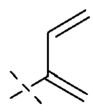, 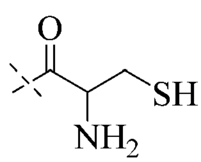,

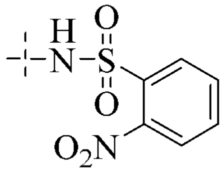, and 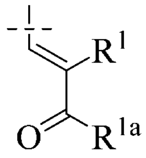 ;

with optional protecting groups;
dwherein
dashed lines indicate attachment to $SP^2$;

| | |
|---|---|
| X | is O, S, or NH, |
| $X^0$ | is -OH, $-NR^1R^{1a}$, -SH, or -SeH, |
| $X^H$ | is Cl, Br, I or F; |
| Ar | is phenyl, naphthyl, indenyl, indanyl, or tetralinyl; |
| $R^1$, $R^{1a}$, $R^{1b}$ | are independently of each other H, $C_{1-6}$ alkyl, $C_{2-6}$ alkenyl, $C_{2-6}$ alkynyl, $C_{3-8}$ cycloalkyl, 4- to 7-membered heterocyclyl, 8- to 11-membered heterobicyclyl, phenyl, naphthyl, indenyl, indanyl, or tetralinyl; and |
| $Y^1$ | is selected from formulas (f-i) to (f-vi): |

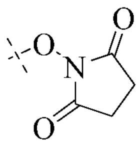 (f-i), 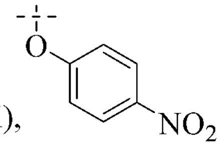 (f-ii), 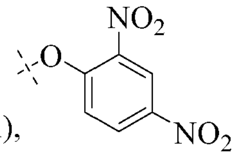 (f-iii),

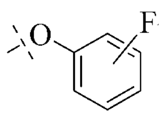 (f-iv), 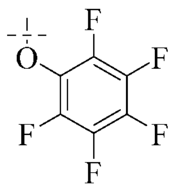 (f-v) and 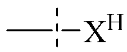 (f-vi),

wherein
the dashed lines indicate attachment to the rest of the molecule,

| | |
|---|---|
| b | is 1, 2, 3 or 4, and |
| $X^H$ | is Cl, Br, I, or F. |

More preferably, $A^{x2}$ is $-NH_2$, maleimide or thiol and most preferably $A^{x2}$ is maleimide.
It is equally preferred that $A^{x2}$ is thiol.
$A^{x2}$ may optionally be present in protected form.
If the hydrogel of step (a) is covalently conjugated to a spacer moiety, the resulting hydrogel-spacer moiety conjugate is of formula (V):

$$\vdots\, A^{y1} - SP^2 - A^{x2} \quad (V),$$

wherein

the dashed line indicates attachment to the hydrogel of step (a);
$A^{y1}$ is the linkage formed between $A^{x0}$ and $A^{x1}$; and
$SP^2$ and $A^{x2}$ are used as in formula (I).

Preferably, $A^{y1}$ is a stable linkage.
Preferably, $A^{y1}$ is selected from the group consisting of

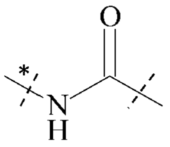, 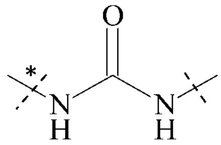 and 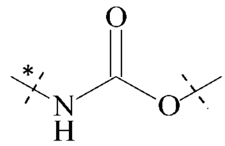 ,

wherein
dashed lines marked with an asterisk indicate attachment to the hydrogel; and unmarked dashed lines indicate attachment to $SP^2$.

Suitable reaction conditions are described in the Examples sections and are known to the person skilled in the art.

Process step (b) may be carried out in the presence of a base. Suitable bases include customary inorganic or organic bases. These preferably include alkaline earth metal or alkali metal hydrides, hydroxides, amides, alkoxides, acetates, carbonates or bicarbonates such as, for example, sodium hydride, sodium amide, sodium methoxide, sodium ethoxide, potassium tert-butoxide, sodium hydroxide, potassium hydroxide, ammonium hydroxide, sodium acetate, potassium acetate, calcium acetate, ammonium acetate, sodium carbonate, potassium carbonate, potassium bicarbonate, sodium bicarbonate or ammonium carbonate, and tertiary amines such as trimethylamine, triethylamine, tributylamine, *N,N*-dimethylaniline, *N,N*-dimethylbenzylamine, pyridine, *N*-methylpiperidine, *N*-methylmorpholine, *N,N*-dimethylaminopyridine, diazabicyclooctane (DABCO), diazabicyclononene (DBN), *N,N*-diisopropylethylamine (DIPEA), diazabicycloundecene (DBU) or collidine.

Process step (b) may be carried out in the presence of a solvent. Suitable solvents for carrying out the process step (b) of the invention include organic solvents. These preferably include water and aliphatic, alicyclic or aromatic hydrocarbons such as, for example, petroleum ether, hexane, heptane, cyclohexane, methylcyclohexane, benzene, toluene, xylene or decalin; halogenated hydrocarbons such as, for example, chlorobenzene, dichlorobenzene, dichloromethane, chloroform, carbon tetrachloride, dichloroethane or trichloroethane; alcohols such as methanol, ethanol, n- or i-propanol, n-, i-, sec- or tert-butanol, ethanediol, propane-1,2-diol, ethoxyethanol, methoxyethanol, diethylene glycol monomethyl ether, dimethylether, diethylene glycol; acetonitrile, N-methyl-2-pyrrolidone (NMP), dimethylformamide (DMF), dimethyl sulfoxide (DMSO), N,N-dimethylacetamide, nitromethane, nitrobenzene, hexamethylphosphoramide (HMPT), 1,3-dimethyl-2-imidazolidinone (DMI), 1,3-dimethyl-3,4,5,6-tetrahydro-2(1H)-pyrimidinone (DMPU), ethyl acetate, acetone, butanone; ethers such as diethyl ether, diisopropyl ether, methyl t-butyl ether, methyl t-amyl ether, dioxane, tetrahydrofuran, 1,2-dimethoxyethane, 1,2-diethoxyethane or anisole; or mixtures thereof. Preferably, the solvent is selected from the group consisting of water, acetonitrile and N-methyl-2-pyrrolidone.

Step (c)

Preferably, $A^{x3}$ is selected from the group consisting of -SH, $-NH_2$, -SeH, -maleimide, -C=CH, $-N_3$, $-CR^1=CR^{1a}R^{1b}$, $-(C=X)-R^1$, -OH, $-(C=O)-S-R^1$, $-NH-NH_2$, $-O-NH_2$, $-Ar-Sn(R^1)(R^{1a})(R^{1b})$, -Ar-B(OH)(OH), $-Ar-X^0$,
, , , , ,

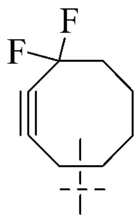 , 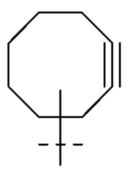 , 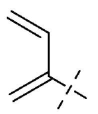 , 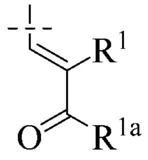 , 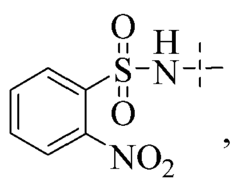 ,

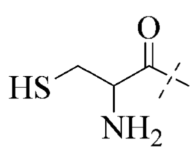 , 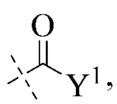 , 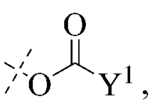 , 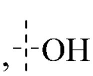 , 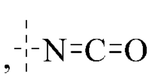 and 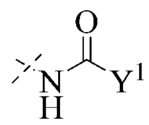 ;

wherein
dashed lines indicate attachment to Z;

| | |
|---|---|
| X | is O, S, or NH, |
| $X^0$ | is -OH, $-NR^1R^{1a}$, -SH, or -SeH; |

$R^1$, $R^{1a}$, $R^{1b}$ are independently of each other H, $C_{1-6}$ alkyl, $C_{2-6}$ alkenyl, $C_{2-6}$ alkynyl, $C_{3-8}$ cycloalkyl, 4- to 7-membered heterocyclyl, 8- to 11-membered heterobicyclyl, phenyl, naphthyl, indenyl, indanyl, or tetralinyl; and

Ar is phenyl, naphthyl, indenyl, indanyl, or tetralinyl.

$Y^1$ is an activated carboxylic acid, activated carbonate or activated carbamate, preferably $Y^1$ is selected from formulas (f-i) to (f-vi):

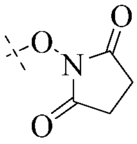 (f-i), 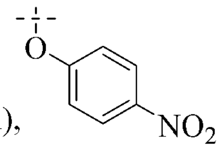 (f-ii), 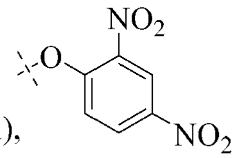 (f-iii),

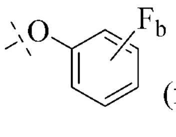 (f-iv), 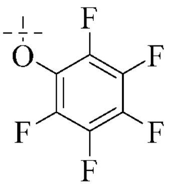 (f-v) and 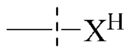 (f-vi),

wherein
the dashed lines indicate attachment to the rest of the molecule,

b is 1, 2, 3 or 4, and
$X^H$ is Cl, Br, I, or F

In a preferred embodiment, $Y^1$ is selected from formulas (f-i) to (f-vi):

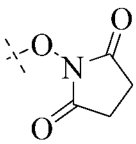 (f-i), 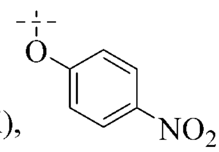 (f-ii), 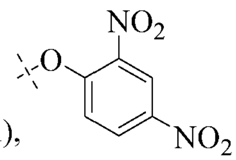 (f-iii),

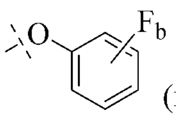 (f-iv), 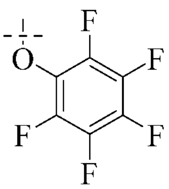 (f-v) and 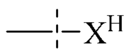 (f-vi),

wherein
the dashed lines, b and $X^H$ are used as above.

More preferably, $A^{x3}$ is-SH or -maleimide and most preferably $A^{x3}$ is -SH. In another preferred embodiment $A^{x3}$ is of formula (aI)

$$PG^0 - S \vdots \quad (aI),$$

wherein
the dashed line indicates attachment to Z of formula (II);

$PG^0$ is a sulfur-activating moiety; and
S is sulfur;

Preferably, $PG^0$ of formula (aI) is selected from the group consisting of

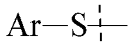 (i), 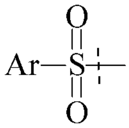 (ii), 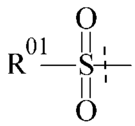 (iii), 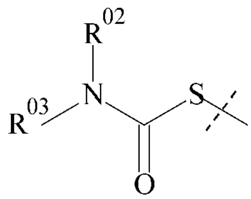 (iv),

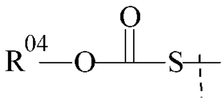 (v), 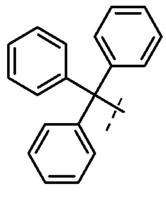 (vi), and 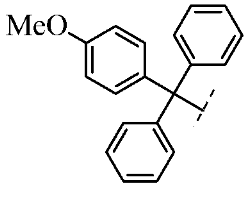 (vii);

wherein
the dashed lines indicate attachment to the sulfur of formula (al);

Ar — is an aromatic moiety which is optionally further substituted;

$R^{01}$, $R^{02}$, $R^{03}$, $R^{04}$ — are independently of each other -H; $C_{1-50}$ alkyl; $C_{2-50}$ alkenyl; or $C_{2-50}$ alkynyl, wherein $C_{1-50}$ alkyl; $C_{2-50}$ alkenyl; and $C_{2-50}$ alkynyl are optionally substituted with one or more $R^3$, which are the same or different and wherein $C_{1-50}$ alkyl; $C_{2-50}$ alkenyl; and $C_{2-50}$ alkynyl are optionally interrupted by one or more groups selected from the group consisting of -Q-, -C(O)O-; -O-; -C(O)-; $-C(O)N(R^4)-$; $-S(O)_2N(R^4)-$;$-S(O)N(R^4)-$; $-S(O)_2-$; -S(O)-; $-N(R^4)S(O)_2N(R^{4a})-$; -S-; $-N(R^4)-$;$-OC(O)R^4$; $-N(R^4)C(O)-$; $-N(R^4)S(O)_2-$; $-N(R^4)S(O)-$; $-N(R^4)C(O)O-$;$-N(R^4)C(O)N(R^{4a})-$; and $-OC(O)N(R^4R^{4a})$;

Q — is selected from the group consisting of phenyl; naphthyl; indenyl; indanyl; tetralinyl; $C_{3-10}$ cycloalkyl; 4- to 7-membered heterocyclyl; and 8- to 11- membered heterobicyclyl, wherein T is optionally substituted with one or more $R^3$, which are the same or different;

$R^3$ — is halogen; -CN; oxo (=O); $-COOR^5$; $-OR^5$; $-C(O)R^5$; $-C(O)N(R^5R^{5a})$; $-S(O)_2N(R^5R^{5a})$; $-S(O)N(R^5R^{5a})$; $-S(O)_2R^5$; $-S(O)R^5$; $-N(R^5)S(O)_2N(R^{5a}R^{5b})$; $-SR^5$; $-N(R^5R^{5a})$; $-NO_2$; $-OC(O)R^5$; $-N(R^5)C(O)R^{5a}$; $-N(R^5)S(O)_2R^{5a}$; $-N(R^5)S(O)R^{5a}$; $-N(R^5)C(O)OR^{5a}$; $-N(R^5)C(O)N(R^{5a}R^{5b})$; $-OC(O)N(R^5R^{5a})$; or $C_{1-6}$ alkyl, wherein $C_{1-6}$ alkyl is optionally substituted with one or more halogen, which are the same or different; and

$R^4$, $R^{4a}$, $R^5$, $R^{5a}$, $R^{5b}$ — are independently selected from the group consisting of -H; or $C_{1-6}$ alkyl, wherein $C_{1-6}$ alkyl is optionally substituted with one or more halogen, which are the same or different.

Preferably, $R^{01}$, $R^{03}$ and $R^{04}$ are independently of each other $C_{1-6}$ alkyl.

Preferably, $R^{02}$ is selected from H and $C_{1-6}$ alkyl.

Preferably, Ar is selected from the group consisting of

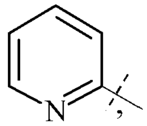, 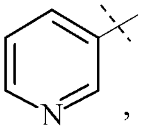, 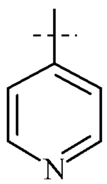, 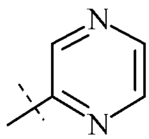, 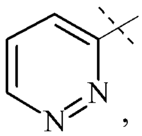,

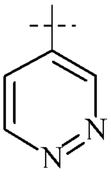, 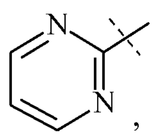, 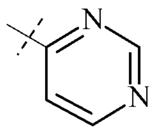, 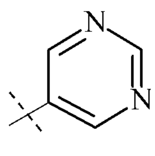, 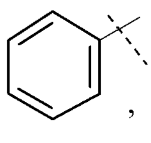,

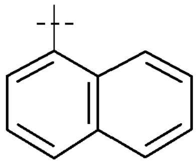, 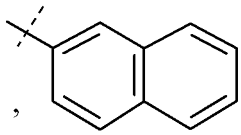,

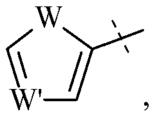, 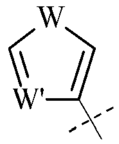, 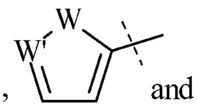 and 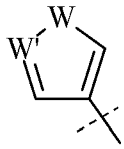.

wherein

dashed lines indicate attachment to the rest of $PG^0$ of formula (aI);

W is independently of each other O, S, or N;
W' is N; and

wherein Ar is optionally substituted with one or more substituent(s) independently selected from the group consisting of $NO_2$, Cl and F.

More preferably, $PG^0$ of formula (aI) is selected from the group consisting of

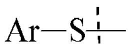 (i), 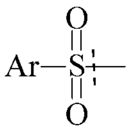 (ii), 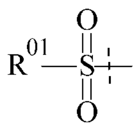 (iii), 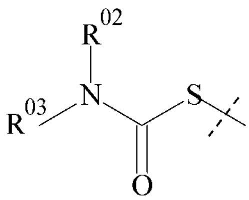 (iv)

and

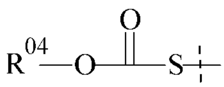 (iv),

wherein

the dashed lines indicate attachment to the sulfur of formula (aI); and
Ar, $R^{01}$, $R^{02}$, $R^{03}$ and $R^{04}$ are used as above.

More preferably, $PG^0$ of formula (aI) is

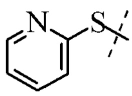,

wherein
the dashed line indicates attachment to the sulfur of formula (aI).
$A^{x3}$ may optionally be present in protected form.
Preferred combinations of $A^{x2}$ and $A^{x3}$ are the following:

| $A^{x2}$ | $A^{x3}$ |
|---|---|
| -maleimide | HS-, $H_2N$- or HSe- |
| -maleimide | -NH- |
| -SH, $-NH_2$ or -SeH | maleimide- |
| -NH- | maleimide- |
| $-NH_2$ | $Y^1$-(C=O)-, $Y^1$-(C=O)-NH-, or $Y^1$-(C=O)-O- |
| $-N_3$ | HC=C-,<br>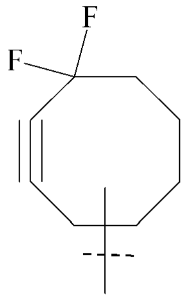<br>, or<br>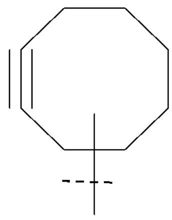 |
| -C=CH,<br>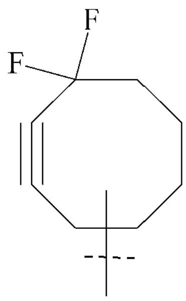<br>, or<br>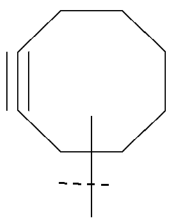 | $N_3$- |
| $-CR^{1a}=CR^{1a}R^{1b}$ | $R^{1b}R^{1a}C=CR^1$- or 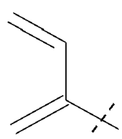 |

(continued)

| $A^{x2}$ | $A^{x3}$ |
|---|---|
| 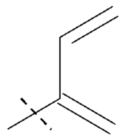 | $R^{1b}R^{1a}C=CR^1$- |
| -(C=X)-$R^1$ | 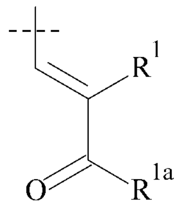 |
| 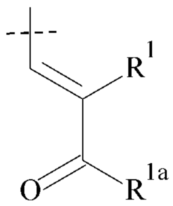 | $R^1$-(C=X)- |
| -OH | $H_2N$- or 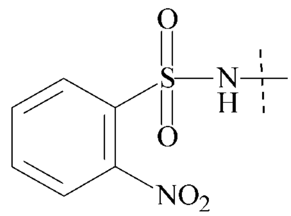 |
| -$NH_2$ or 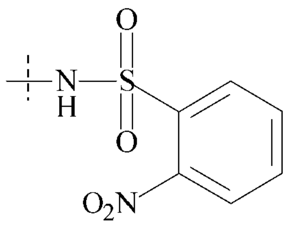 | HO- |
| -(C=O)-S-$R^1$ | 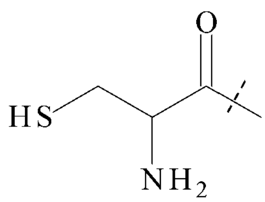 |
| 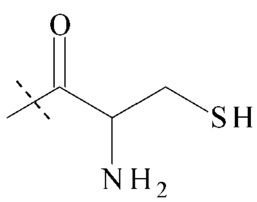 | $R^1$-S-(C=O)- |
| -(C=O)-H | $H_2N$-NH- or $H_2N$-O- |
| -NH-$NH_2$ or -O-$NH_2$ | H-(C=O)- |

(continued)

| $A^{x2}$ | $A^{x3}$ |
|---|---|
| $-Ar-X^0$ | $-Ar-Sn(R^1)(R^{1a})(R^{1b})$ or $-Ar-B(OH)(OH)$ |
| $(R^{1b})(R^{1a})(R^1)Sn-Ar-$ or $-Ar-B(OH)(OH)$ | $X^0-Ar-$ |

wherein

X is O, S, or NH;

$X^0$ is -OH, $-NR^1R^{1a}$, -SH, or -SeH;

$R^1$, $R^{1a}$, $R^{1b}$ are independently of each other selected from the group consisting of H, $C_{1-6}$ alkyl, $C_{2-6}$ alkenyl, $C_{2-6}$ alkynyl, $C_{3-8}$ cycloalkyl, 4- to 7-membered heterocyclyl, 8- to 11-membered heterobicyclyl, phenyl, naphthyl, indenyl, indanyl, and tetralinyl; and

Ar is phenyl, naphthyl, indenyl, indanyl, or tetralinyl.

In another preferred embodiment $A^{x2}$ is -SH and $A^{x3}$ is of formula (aI), wherein $PG^0$ is of formula (i), (ii), (iii), (iv), (v), (vi) or (viii). More preferably, $PG^0$ of formula (aI) is of formula (i), (ii), (iii), (iv) or (v) and even more preferably, $PG^0$ of formula (aI) is of formula (i). Most preferably, $PG^0$ of formula (aI) is of formula

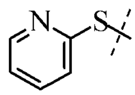

,

wherein

the dashed line indicates attachment to the sulfur of formula (aI).

In one preferred embodiment, $A^{x2}$ is an amine and $A^{x3}$ is $Y^1$-(C=O)-, $Y^1$-(C=O)-NH-, or $Y^1$-(C=O)-O- and most preferably $A^{x2}$ is an amine and $A^{x3}$ is $Y^1$-(C=O)-.

In another preferred embodiment $A^{x2}$ is maleimide and $A^{x3}$ is -SH.

In one embodiment the optional step (b) is omitted, $A^{x0}$ is an amine and $A^{x3}$ is $ClSO_2$-, R (C=O)-, I-, Br-, Cl-, SCN-, CN-, O=C=N-, $Y^1$-(C=O)-, $Y^1$-(C=O)-NH-, or $Y^1$-(C=O)-O-,

wherein

$R^1$ is H, $C_{1-6}$ alkyl, $C_{2-6}$ alkenyl, $C_{2-6}$ alkynyl, $C_{3-8}$ cycloalkyl, 4- to 7-membered heterocyclyl, 8- to 11-membered heterobicyclyl, phenyl, naphthyl, indenyl, indanyl, or tetralinyl; and

$Y^1$ is selected from formulas (f-i) to (f-vi):

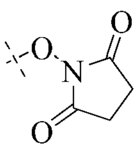 (f-i), 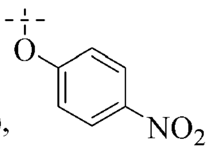 (f-ii), 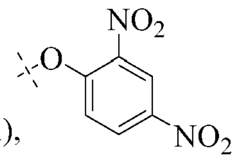 (f-iii),

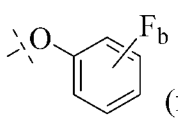 (f-iv), 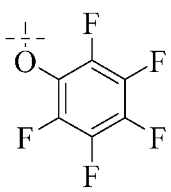 (f-v) and 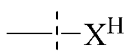 (f-vi),

wherein

the dashed lines indicate attachment to the rest of the molecule,

b is 1, 2, 3 or 4, and

$X^H$ is Cl, Br, I, or F.

In another embodiment the optional step (b) is omitted, $A^{x0}$ is a hydroxyl group (-OH) and $A^{x3}$ is O=C=N-, I-, Br-, SCN-, or $Y^1$-(C=O)-NH-,
wherein $Y^1$ is selected from formulas (f-i) to (f-vi):

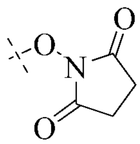 (f-i), 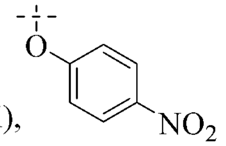 (f-ii), 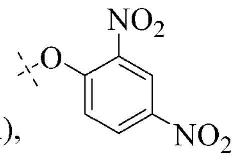 (f-iii),

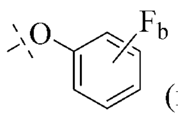 (f-iv), 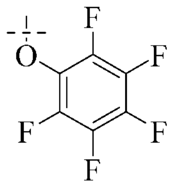 (f-v) and 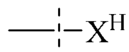 (f-vi),

wherein
the dashed lines indicate attachment to the rest of the molecule,

b is 1, 2, 3 or 4, and
$X^H$ is Cl, Br, I, or F.

In another embodiment the optional step (b) is omitted, $A^{x0}$ is a carboxylic acid (-(C=O)OH) and $A^{x3}$ is a primary amine or secondary amine.
In another embodiment the optional step (b) is omitted, $A^{x0}$ is an amine and $A^{x3}$ is $Y^1$-(C=O)-, $Y^1$-(C=O)-NH-, or $Y^1$-(C=O)-O-.
In another embodiment the optional step (b) is omitted, $A^{x0}$ is a maleimide and $A^{x3}$ is thiol.
In a preferred embodiment the optional step (b) is omitted, $A^{x0}$ is an amine and $A^{x3}$ is $Y^1$-(C=O)-.
In another preferred embodiment the optional step (b) is omitted, $A^{x0}$ is -SH and $A^{x3}$ is of formula (al), wherein $PG^0$ is of formula (i), (ii), (iii), (iv), (v), (vi) or (viii). More preferably, $PG^0$ of formula (al) is of formula (i), (ii), (iii), (iv) or (v) and even more preferably, $PG^0$ of formula (al) is of formula (i). Most preferably, $PG^0$ of formula (al) is of formula

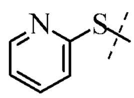,

wherein
the dashed line indicates attachment to the sulfur of formula (al).
The hydrogel obtained from step (c) has the structure of formula (VIa) or (VIb):

$$\text{-}\!\!\!\!\mid A^{y0} - Z \qquad \text{(VIa)}$$

$$\text{-}\!\!\!\!\mid A^{y1} - SP^2 - A^{y2} - Z \qquad \text{(VIb);}$$

wherein
the dashed line indicates attachment to the hydrogel of step (a);

$A^{y0}$ is the linkage formed between $A^{x0}$ and $A^{x3}$;
$A^{y1}$ is used as in formula (V);
$A^{y2}$ is the linkage formed between $A^{x2}$ and $A^{x3}$;
$SP^2$ is used as in formula (I); and
Z is used as in formula (II).

Preferably, $A^{y0}$ and $A^{y2}$ are selected from the group consisting of amide, carbamate,

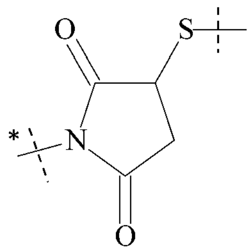 and 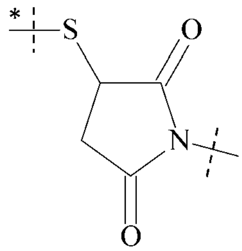 ;

wherein

the dashed lines marked with an asterisk indicate attachment to the hydrogel or $SP^2$, respectively; and
the unmarked dashed lines indicate attachment to Z.

Z is an inert linear or branched PEG-based polymer having a molecular weight ranging from 10 kDa to 250 kDa, preferably ranging from 10 to 50 kDa and most preferably Z has a molecular weight of 40 kDa.
Z is an inert linear or branched PEG-based polymer comprising at least 70% PEG, even more preferably comprising at least 80% PEG and most preferably comprising at least 90% PEG. Step (c) comprises reacting the hydrogel of step (a) or step (b) with a reagent of formula (II) in such manner that no more than 80 mol-% of $A^{x0}$ or $A^{x2}$ react with $A^{x3}$, even more preferably, such that no more than 60 mol-% of $A^{x0}$ or $A^{x2}$ react with $A^{x3}$, even more preferably, such that no more than 40 mol-% of $A^{x0}$ or $A^{x2}$ react with $A^{x3}$, even more preferably, such that no more than 20 mol-% of $A^{x0}$ or $A^{x2}$ react with $A^{x3}$ and most preferably, such that no more than 15 mol-% of $A^{x0}$ or $A^{x2}$ react with $A^{x3}$.
This can be achieved, for example, by reacting at most 0.8, 0.6, 0.4, 0.2 or 0.15 chemical equivalents of the reagent of formula (II) relative to $A^{x0}$ or $A^{x2}$ with the hydrogel of step (a) or (b), respectively.
Methods to prevent the reaction of more chemical equivalents are described above.
Based on the measurements of the amount of substance of $A^{x0}$ of step (a) and after step (c) the amount of substance of reacted $A^{x0}$ can be calculated with equation (1):

$$\text{(1) Amount of substance of reacted } A^{x0} \text{ in mmol/g} = ({A^{x0}}_1 - {A^{x0}}_2) / ({A^{x0}}_2 \times MW_Z + 1),$$

wherein

${A^{x0}}_1$ is the amount of substance of functional groups $A^{x0}$ of the hydrogel of step (a) in mmol/g;
${A^{x0}}_2$ is the amount of substance of functional groups $A^{x0}$ of the hydrogel after step (c) in mmol/g; and
$MW_Z$ is the molecular weight of Z in g/mmol.

If the optional spacer reagent was covalently conjugated to the hydrogel of step (a), the calculation of the number of reacted $A^{x2}$ is done accordingly.
The percentage of reacted functional groups $A^{x0}$ relative to the functional groups $A^{x0}$ of the hydrogel of step (a) is calculated according to equation (2):

$$\text{(2) mol-\% of reacted } A^{x0} = 100 \times [({A^{x0}}_1 - {A^{x0}}_2) / ({A^{x0}}_2 \times MW_Z + 1)] / {A^{x0}}_1,$$

wherein the variables are used as above.
In one embodiment Z is conjugated to the surface of the hydrogel. This can be achieved by selecting the size and structure of the reagent $A^{x3}$-Z such that it is too large to enter the pores or network of the hydrogel. Accordingly, the minimal size of $A^{x3}$-Z depends on the properties of the hydrogel. The person skilled in the art however knows methods how to test whether a reagent $A^{x3}$-Z is capable of entering into the hydrogel using standard experimentation, for example by using size exclusion chromatography with the hydrogel as stationary phase.

**Other Aspects**

Another aspect of the present invention is a hydrogel obtainable from the process of the present invention.
Another aspect of the present invention is the use of the hydrogel of the present invention as a carrier in a hydrogel-linked prodrug.
Another aspect of the present invention is a hydrogel-linked prodrug comprising a covalently conjugated hydrogel

of the present invention.

It is preferred that the conjugates of formula (VIa) and (VIb) are such that they shield the biologically active moieties conjugated to the surface of carrier-linked prodrugs in which the hydrogels of the present invention are used as carriers.

This can be tested by using immune assays in which labelled antibodies against the biologically active moiety are used to test the binding of said labelled antibodies to biologically active moieties conjugated to hydrogels of step c) and of step a) and to calculate the relative binding of the labelled antibodies to biologically active moieties conjugated to hydrogels of step c) relative to those conjugated to hydrogels of step a).

Preferably, the binding of such labelled antibody to a biologically active moiety conjugated to a hydrogel of step c) is no more than 50% of the binding of said labelled antibody to said biologically active moiety conjugated to a hydrogel of step a), e.g. no more than 40%, no more than 30%, no more than 20%, no more than 10%, no more than 5%, no more than 2% or no more than 1%.

It was surprisingly found that hydrogels of the present invention that carry polymeric moieties Z effectively shield remaining functional groups on the surface of the hydrogel and/or effectively shield biologically active moieties conjugated to the surface of the hydrogel.

When such hydrogel is used as a carrier in a carrier-linked prodrug it has the technical effect of rendering the carrier-linked prodrug better tolerable by the patient and causes reduced immune responses.

Another technical effect obtained with the hydrogels of the present invention is that carrier-linked prodrugs comprising such hydrogels can be injected using reduced force, in particular in the case of carrier-linked prodrugs of hydrophobic drugs.

In another embodiment the process of the present invention can be performed such that instead of the hydrogel a hydrogel-linked prodrug is used in step (a) and steps (b) and (c) are performed as detailed above.

## Examples

### Materials and Methods

#### Materials:

Amino 4-arm PEG5000 was obtained from JenKem Technology, Beijing, P. R. China. CithrolTM DPHS was obtained from Croda International Pic, Cowick Hall, United Kingdom.

Isopropylmalonic acid was obtained from ABCR GmbH & Co. KG, 76187 Karlsruhe, Germany.

N-maleimido propionic acid NHS-ester was obtained from TCI Deutschland, 65760 Eschbom, Germany.

All other chemicals were from Sigma-ALDRICH Chemie GmbH, Taufkirchen, Germany.

5 kDa PEG-SH, 10kDa PEG-SH, 20kDa PEG-SH, 10 kDa PEG-NHS and 20 kDa PEG-NHS were obtained from RAPP POLYMERE GmbH, 72072 Tübingen, Germany.

N-(3-maleimidopropionyl)-21-amino-4,7,10,13,16,19-hexaoxa-heneicosanoic acid Pfp ester (Mal-$PEG_6$-Pfp) was obtained from Biomatrik, China.

30 kDa PEG-NHS, 40 kDa PEG-NHS, branched 40kDa PEG-NHS, branched 60kDa PEG-NHS and branched 80 kDa PEG-NHS were obtained from NOF Corporation, Tokyo 150-6019, Japan.

#### Methods:

RP-HPLC was done on a 100x20 mm or 100x40 mm C18 ReproSil-Pur 300 ODS-3 5μ column (Dr. Maisch, Ammerbuch, Germany) connected to a Waters 600 or 2535 HPLC System and Waters 2487 or 2489 Absorbance detector, respectively. Linear gradients of solution A (0.1% TFA in H2O) and solution B (0.1% TFA in acetonitrile) were used. HPLC fractions containing product were combined and lyophilized.

Flash chromatography purifications were performed on an Isolera One system from Biotage AB, Sweden, using Biotage KP-Sil silica cartridges and n-heptane, ethyl acetate, and methanol as eluents. Products were detected at 254 nm. For products showing no absorbance above 240 nm fractions were screened by LC/MS.

Analytical ultra-performance LC (UPLC) was performed on a Waters Acquity system equipped with a Waters BEH300 C18 column (2.1 × 50 mm, 1.7 μm particle size) coupled to a LTQ Orbitrap Discovery mass spectrometer from Thermo Scientific.

HPLC-Electrospray ionization mass spectrometry (HPLC-ESI-MS) was performed on a Waters Acquity UPLC with an Acquity PDA detector coupled to a Thermo LTQ Orbitrap Discovery high resolution/high accuracy mass spectrometer equipped with a Waters ACQUITY UPLC BEH300 C18 RP column (2.1 × 50 mm, 300 Å, 1.7 μm, flow: 0.25 mL/min; solvent A: UP-$H_20$ + 0.04% TFA, solvent B: UP-Acetonitrile + 0.05% TFA.

MS spectra of PEG products showed a series of $(CH_2CH_2O)_n$ moieties due to polydispersity of PEG staring

materials. For easier interpretation only one single representative m/z signal is given in the examples.

**Example 1**

**Synthesis of backbone reagent Ia and 1b:**

$$[PEG1250 — {}_{L}Lys\text{-}{}_{L}Lys_2\text{-}{}_{L}Lys_4(NH_2)_8]_4$$

**1a**

=

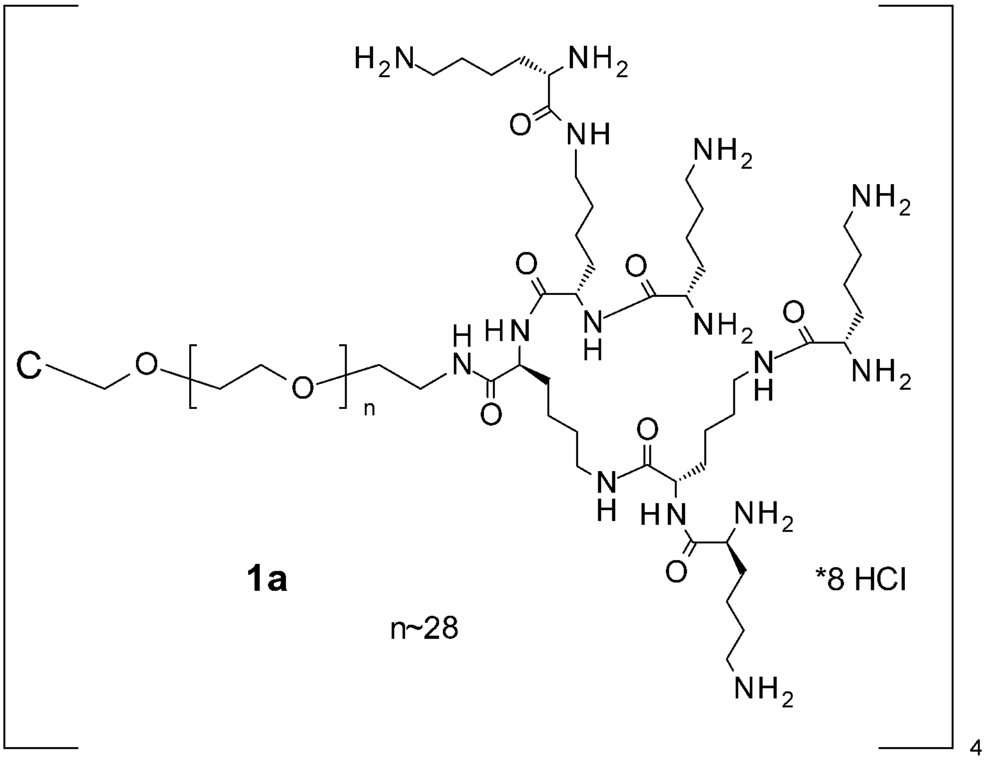

1a

n~28

*8 HCl

Backbone reagent **1a** was synthesized as described in example 1 of WO 2011/012715 A1. Backbone reagent **1b** was synthesized as described in example 1 of WO 2011/012715 A1 except for the use of Boc-DLys(Boc)-OH instead of Boc-LLys(Boc)-OH.

MS: m/z 888.50 = $[M+10H^+]^{10+}$ (calculated = 888.54)

For mass spectra of polydisperse PEG containing compounds, one single mass peak was selected.

**Example 2**

**Synthesis of Crosslinker reagents 2d, *rac-2h,* 2k, 2n, *rac*-2q, and *rac-2t***

Crosslinker reagent **2d** was prepared from azelaic acid monobenzyl ester and PEG2000 according to the following scheme:

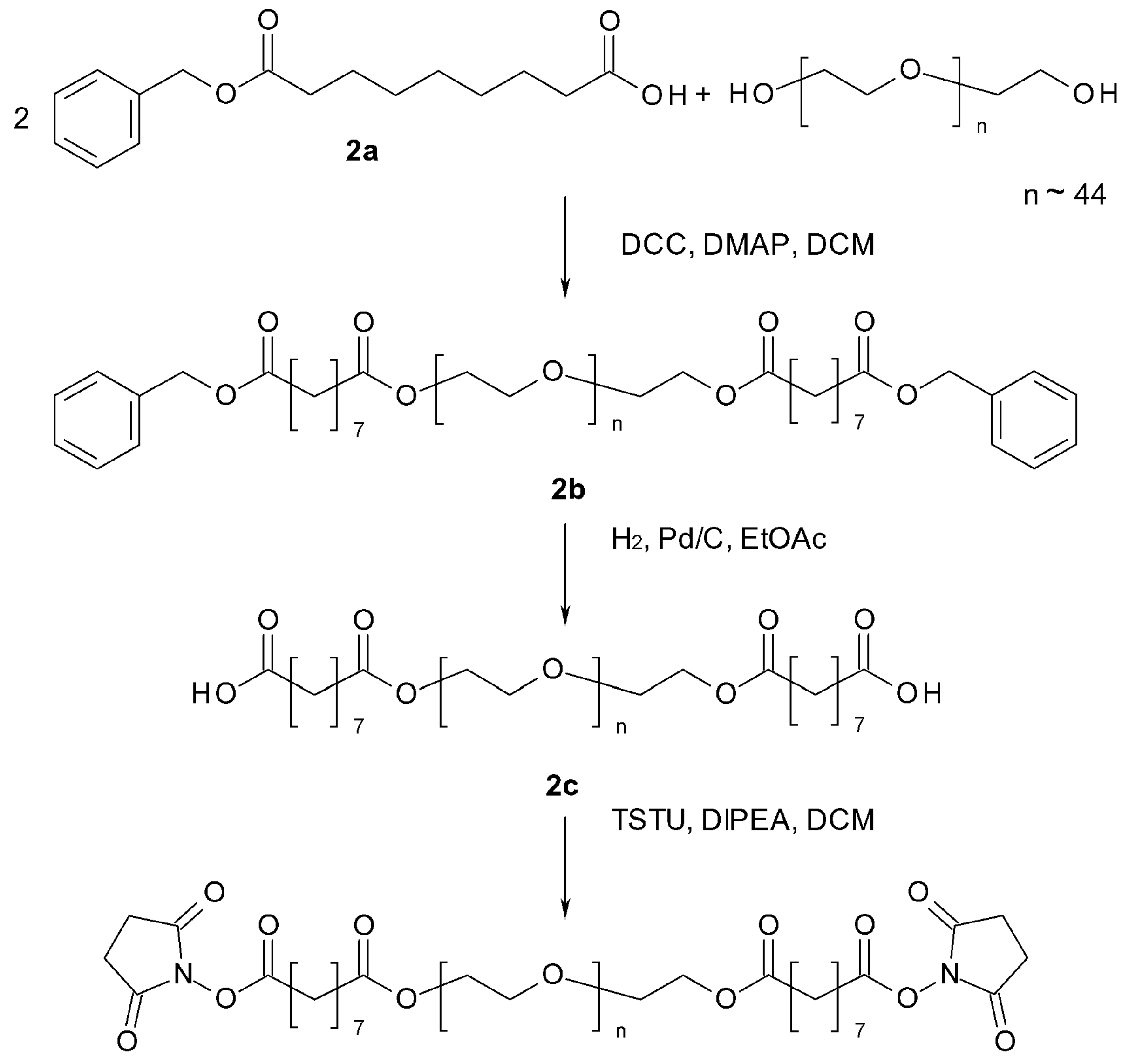

2a
2b
2c
2d

For the synthesis of azelaic acid monobenzyl ester **2a,** a mixture of azelaic acid (37.6 g, 200 mmol), benzyl alcohol (21.6 g, 200 mmol), *p*-toluenesulfonic acid (0.80 g, 4.2 mmol), and 240 mL toluene was heated to reflux for 7 h in a Dean-Stark apparatus. After cooling down, the solvent was evaporated and 300 mL sat. aqueous NaHCOa solution were added. This mixture was extracted with 3 × 200 mL MTBE. The combined organic phases were dried over $Na_2SO_4$ and the solvent was evaporated. The product was purified on 2 × 340 g silica using ethyl acetate / heptane (10:90 → 25:75) as eluent. The eluent was evaporated and the residue was dried *in vacuo* over night.

Yield 25.8 g (46%) colorless **oil 2a.**

MS: m/z 279.16 = $[M+H]^+$ (calculated = 279.16).

For synthesis of compound **2b,** azelaic acid monobenzyl ester **2a** (14.6 g, 52.5 mmol) and PEG 2000 (30.0 g, 15 mmol) were dissolved in 50 mL dichloromethane and cooled with an ice bath. A solution of DCC (10.8 g, 52.5 mmol) and DMAP (91.6 mg, 0.8 mmol) in 30 mL dichloromethane was added. The ice bath was removed and mixture was stirred at room temperature overnight. The resulting suspension was cooled to 0 °C and the solid was filtered off. The solvent was evaporated *in vacuo.*

The residue was dissolved in 26 mL dichloromethane and diluted with 780 mL MTBE at room temperature. The mixture was stored over night at -20 °C. The precipitate was collected by filtration through a glass filter Por. 3 and washed with 350 mL of cooled MTBE (-20 °C). The product was dried *in vacuo* over night.

Yield 32.5 g (86%) white powder **2b.**

MS: m/z = 826.49 $[M+3H]^{3+}$ (calculated = 856.05).

For synthesis of compound **2c,** compound **2b** (32.2 g, 12.8 mmol) was dissolved in ethyl acetate (196 mL) and

306 mg of palladium on charcoal was added. Under a hydrogen atmosphere of ambient pressure, the mixture was stirred overnight at room temperature. The reaction mixture was filtered through a pad of celite and the filtrate was evaporated and dried *in vacuo* over night.

Yield 28.8 g (96%) glassy solid **2c.**

MS: m/z 751.78 = $[M+3H]^{3+}$ (calculated = 751.91).

For mass spectra of polydisperse PEG containing compounds, one single mass peak was selected.

For synthesis of compound **2d,** compound **2c** (28.7 g, 12.3 mmol) and TSTU (14.8 g, 49.1 mmol) were dissolved in 100 mL dichloromethane at room temperature. Then DIPEA (6.3 g, 49.1 mmol) was added and the mixture was stirred for 1 h. The resulting suspension was filtered and 170 mL dichloromethane was added. The filtrate was washed with 200 mL aqueous solution (3 g NaOH, 197 g NaCl and 750 g $H_2O$). The organic phase was dried over $MgSO_4$ and the solvent was evaporated *in vacuo.* The residue was dissolved in 200 mL toluene, diluted with 200 mL MTBE at room temperature and stored over night at -20 °C. The precipitate was collected by filtration through a glass filter Por. 3 and washed with 250 mL of cooled MTBE (-20 °C). The product was dried *in vacuo* over night.

Yield 24.0 g (77.4 %) white powder **2d.**

MS: m/z 845.82 =$[M+3H]^{3+}$ (calculated = 860.68).

For mass spectra of polydisperse PEG containing compounds, one single mass peak was selected.

**Synthesis** of **crosslinker reagent *rac-2h***

Crosslinker reagent ***rac-2h*** was prepared from isopropylmalonic acid monobenzyl ester and PEG3300 according to the following scheme:

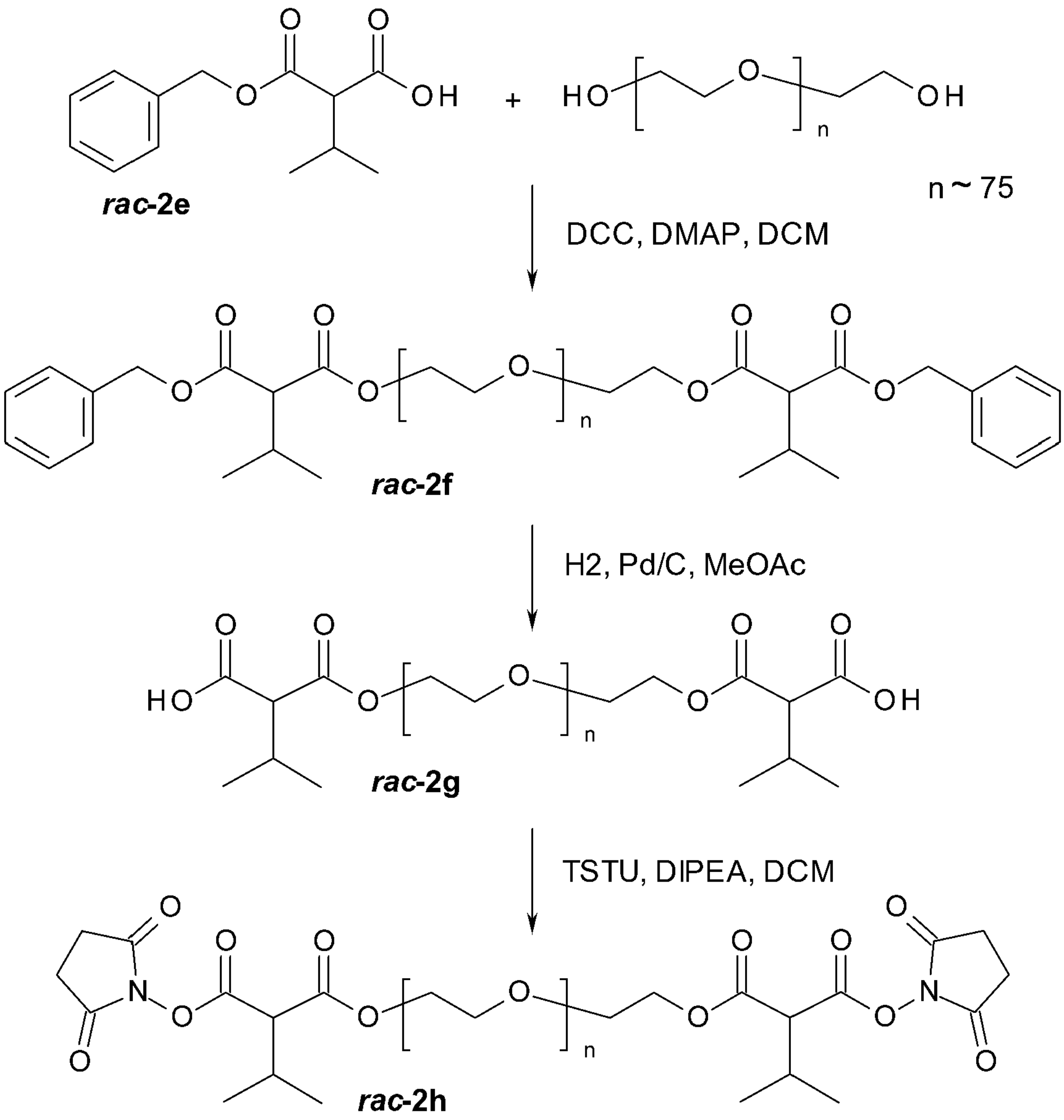


For the synthesis of isopropylmalonic acid monobenzyl ester *rac-2e,* isopropylmalonic acid (35.0 g, 239 mmol), benzyl alcohol (23.3 g, 216 mmol) and DMAP (1.46 g, 12.0 mmol) were dissolved in 100 mL acetonitrile. Mixture was cooled to 0 °C with an ice bath. A solution of DCC (49.4 g, 239 mmol) in 150 mL acetonitrile was added within 15 min at 0 °C. The ice bath was removed and the reaction mixture was stirred over night at room temperature, then the solid was filtered off. The filtrate was evaporated at 40 °C *in vacuo* and the residue was dissolved in 300 mL MTBE. This solution was extracted with 2 × 300 mL sat. aqueous $NaHCO_3$ solution, then the combined aqueous phases were acidified to pH = 1-3 using 6 N hydrochloric acid. The resulting emulsion was extracted with 2 × 300 mL MTBE and the solvent was evaporated. The combined organic phases were washed with 200 mL sat. aqueous NaCl and dried over $MgSO_4$. The product was purified on 340 g silica using ethyl acetate / heptane (10:90 → 20:80) as eluent. The eluent was evaporated and the residue was dried *in vacuo* over night.

Yield 9.62 g (17%) colorless oil ***rac-2e.***

MS: m/z 237.11 = $[M+H]^+$ (calculated = 237.11).

For synthesis of compound ***rac-2f,*** isopropylmalonic acid monobenzyl ester ***rac-2e*** (5.73 g, 24.2 mmol) and PEG 3300 (20.0 g, 6.06 mmol) were dissolved in 105 mL dichloromethane and cooled with an ice bath. A solution of DCC (5.00 g, 24.2 mmol) and DMAP (37 mg, 0.30 mmol) in 15 mL dichloromethane was added. The ice bath was removed and mixture was stirred at room temperature overnight. The resulting suspension was cooled to 0 °C and the solid was filtered off. The solvent was evaporated *in vacuo.*

The residue was dissolved in 70 mL dichloromethane and diluted with 725 mL MTBE at room temperature. The mixture was stored over night at -20 °C. The precipitate was collected by filtration through a glass filter Por. 3 and washed with 650 mL of cooled MTBE (-20 °C). The product was dried *in vacuo* over night.

Yield 21.3 g (94%) white powder ***rac-2f.***

MS: m/z 671.39 =$[M+6H]^{6+}$ (calculated = 671.47).

For mass spectra of polydisperse PEG containing compounds, one single mass peak was selected.

For synthesis of compound ***rac-2g,*** compound ***rac-2f*** (21.2 g, 5.65 mmol) was dissolved in ethyl acetate (130 mL) and 234 mg of palladium on charcoal was added. Under a hydrogen atmosphere of ambient pressure, the mixture was stirred overnight at room temperature. The reaction mixture was filtered through a pad of celite and the filtrate was evaporated and dried *in vacuo* over night.

Yield 20.0 g (99%) glassy solid ***rac-2g.***

MS: m/z 597.35 =$[M+6H]^{6+}$ (calculated = 597.38).

For mass spectra of polydisperse PEG containing compounds, one single mass peak was selected.

For synthesis of compound ***rac-2h,*** compound ***rac-2g*** (7.50 g, 2.10 mmol) and TSTU (2.52 g, 8.38 mmol) were dissolved in 105 mL dichloromethane at room temperature. Then DIPEA (1.08 g, 8.38 mmol) was added and the mixture was stirred for 45 min. The reaction mixture was filtered into 8 individual 50 mL PP Falcon tubes, then 10 mL phosphate buffer 0.5 M pH = 6.5 was added into each Falcon tubes. The mixture was shaken and centrifuged (2000 $min^{-1}$, 1 min). The lower (organic) phases were removed, combined and centrifuged (2000 $min^{-1}$, 1 min) again. Then the lower phases were dried over $MgSO_4$ and the solvent was evaporated *in vacuo.* The residue was dissolved in 100 mL toluene, filtered, diluted with 145 mL MTBE at room temperature and stored over night at -20 °C. The precipitate was collected by filtration through a glass filter Por. 3 and washed with 500 mL of cooled MTBE (-20 °C). The product was dried *in vacuo* over night.

Yield 7.19 g (91%) white powder ***rac*-2h.**

MS: m/z 673.71 =$[M+6H]^{6+}$ (calculated = 673.77).

For mass spectra of polydisperse PEG containing compounds, one single mass peak was selected.

Crosslinker reagent **2k** was prepared from azelaic acid monobenzyl ester and PEG4000 according to the following scheme:

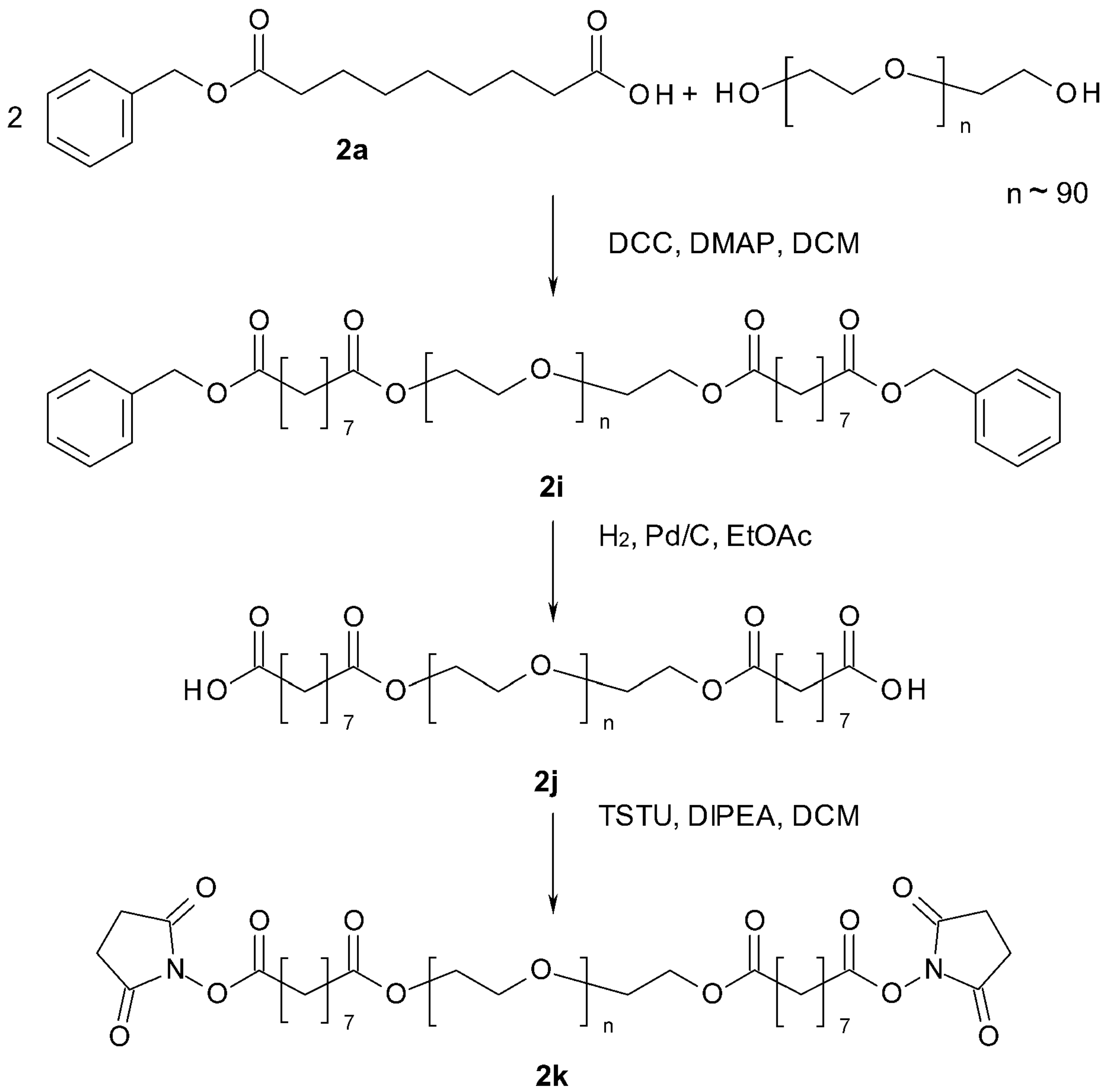


For synthesis of compound **2i,** azelaic acid monobenzyl ester **2a** (9.74 g, 35.0 mmol) and PEG 4000 (40.0 g, 10.0 mmol) were dissolved in 130 mL dichloromethane and cooled with an ice bath. A solution of DCC (7.22 g, 35.0 mmol) and DMAP (61 mg, 0.5 mmol) in 40 mL dichloromethane was added. The ice bath was removed and mixture was stirred at room temperature overnight. The resulting suspension was cooled to 0 °C and the solid was filtered off. The solvent was evaporated *in vacuo.*

The residue was dissolved in 59 mL dichloromethane and diluted with 314 mL MTBE at room temperature. The mixture was stored over night at -20 °C. The precipitate was collected by filtration through a glass filter Por. 3 and washed with 250 mL of cooled MTBE (-20 °C). The product was dried *in vacuo* over night.

Yield 42.9 g (95%) white powder **2i.**

MS: m/z 795.48 = $[M+6H]^{6+}$ (calculated = 751.58).

For mass spectra of polydisperse PEG containing compounds, one single mass peak was selected.

For synthesis of compound **2j,** compound **2i** (42.8 g, 9.47 mmol) was dissolved in ethyl acetate (230 mL) and 35 mL ethanol and 400 mg of palladium on charcoal was added. Under a hydrogen atmosphere of ambient pressure, the mixture was stirred overnight at room temperature. The reaction mixture was filtered through a pad of celite and the filtrate was evaporated and dried *in vacuo* over night.

Yield 37.5 g (91%) glassy solid **2j.**

MS: m/z 758.13 = $[M+6H]^{6+}$ (calculated = 721.54).

For mass spectra of polydisperse PEG containing compounds, one single mass peak was selected.

For synthesis of compound **2k,** compound **2j** (37.3 g, 8.60 mmol) and TSTU (10.4 g, 34.4 mmol) were dissolved

in 120 mL dichloromethane at room temperature. Then DIPEA (4.45 g, 34.4 mmol) was added and the mixture was stirred for 45 min. The resulting suspension was filtered and 100 mL dichloromethane was added. The filtrate was washed with 200 mL aqueous solution (3 g NaOH, 197 g NaCl and 750 g $H_2O$). The organic phase was dried over $MgSO_4$ and the solvent was evaporated *in vacuo.* The residue was dissolved in 200 mL toluene, diluted with 380 mL MTBE at room temperature and stored over night at - 20 °C. The precipitate was collected by filtration through a glass filter Por. 3 and washed with 450 mL of cooled MTBE (-20 °C). The product was dried *in vacuo* over night.

Yield 32.7 g (84%) white powder **2k.**

MS: m/z 790.46 =$[M+6H]^{6+}$ (calculated = 753.90).

For mass spectra of polydisperse PEG containing compounds, one single mass peak was selected.

Crosslinker reagent **2n** was prepared from suberic acid monobenzyl ester 2a and PEG6000 accordingly to the following scheme:

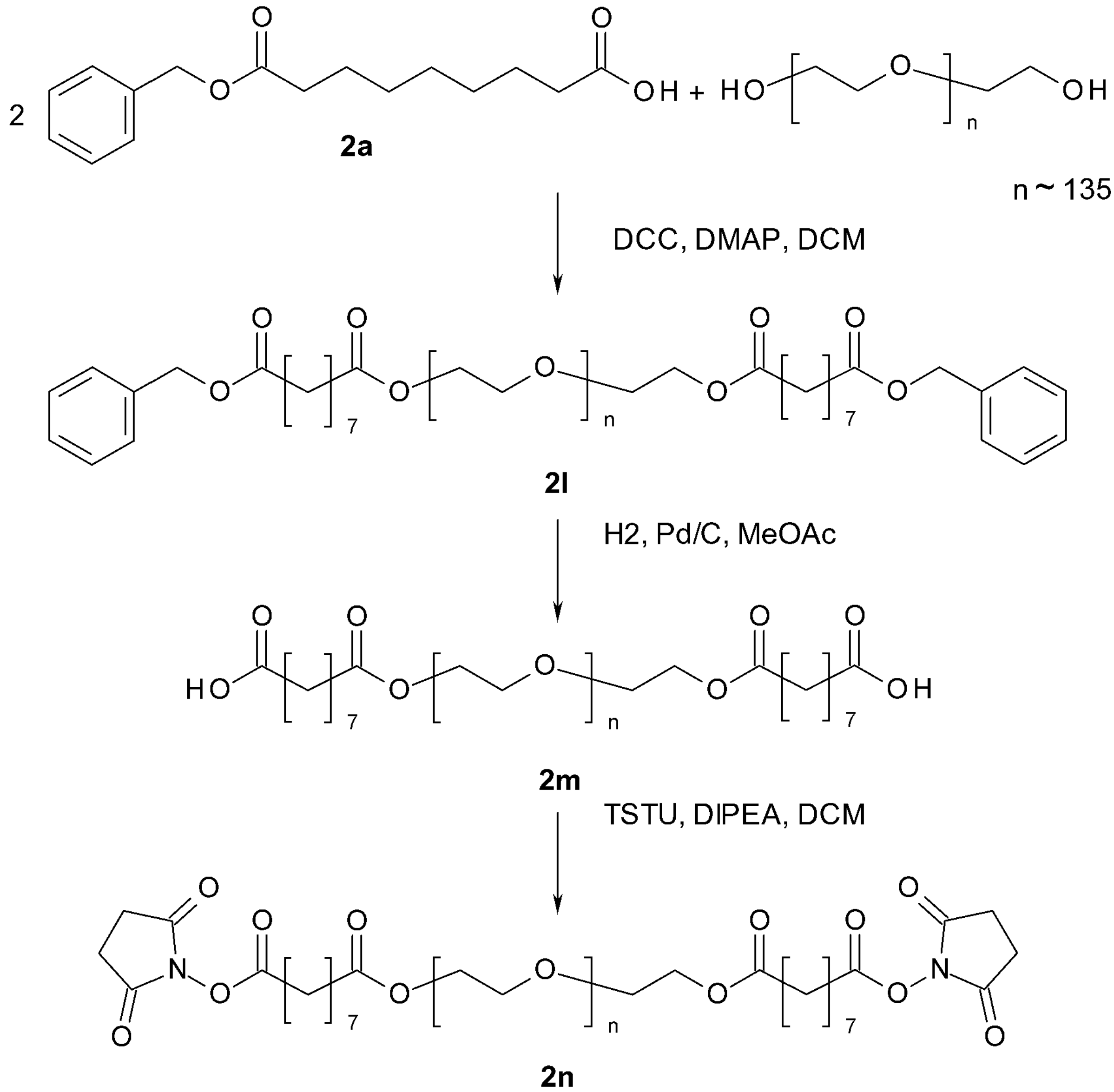


For synthesis of compound **21,** azelaic acid monobenzyl ester **2a** (6.50 g, 23.3 mmol) and PEG 6000 (40.0 g, 6.67 mmol) were dissolved in 140 mL dichloromethane and cooled with an ice bath. A solution of DCC (4.81 g, 23.3 mmol) and DMAP (0.040 g, 0.33 mmol) in 40 mL dichloromethane was added. The ice bath was removed and mixture was stirred at room temperature overnight. The resulting suspension was cooled to 0 °C and the solid was filtered off. The solvent was evaporated *in vacuo.*

The residue was dissolved in 70 mL dichloromethane and diluted with 300 mL MTBE at room temperature. The mixture was stored over night at -20 °C. The precipitate was collected by filtration through a glass filter Por. 3, and

washed with 500 mL of cooled MTBE (-20 °C). The product was dried *in vacuo* over night.

Yield 41.2 g (95%) white powder **21.**

MS: m/z 833.75 = $[M+8H]^{8+}$ (calculated = 833.74).

For mass spectra of polydisperse PEG containing compounds, one single mass peak was selected.

For synthesis of compound **2m,** compound **21** (41.2 g, 6.32 mmol) was dissolved in methyl acetate (238 mL) and ethanol (40 mL), then 400 mg of palladium on charcoal was added. Under a hydrogen atmosphere of ambient pressure, the mixture was stirred overnight at room temperature. The reaction mixture was filtered through a pad of celite and the filtrate was evaporated and dried *in vacuo* over night.

Yield 38.4 g (96%) glassy solid **2m.**

MS: m/z 750.46 = $[M+9H]^{9+}$ (calculated = 750.56).

For mass spectra of polydisperse PEG containing compounds, one single mass peak was selected.

For synthesis of compound **2n,** compound **2m** (38.2 g, 6.02 mmol) and TSTU (7.25 g, mmol) were dissolved in 130 mL dichloromethane at room temperature. Then DIPEA (3.11 g, 24.1 mmol) was added and the mixture was stirred for 1 h. The resulting suspension was filtered, the filtrate was diluted with 100 mL dichloromethane and washed with 200 mL of a solution of 750 g water / 197 g NaCl / 3 g NaOH. The organic phase was dried over $MgSO_4$ and the solvent was evaporated *in vacuo.*

The residue was dissolved in 210 mL toluene, diluted with 430 mL MTBE at room temperature and stored over night at -20 °C. The precipitate was collected by filtration through a glass filter Por. 3, and washed with 450 mL of cooled MTBE (-20 °C). The product was dried *in vacuo* over night.

Yield 35.8 g (91%) white powder **2n.**

MS: m/z 857.51 = $[M+8H]^{8+}$ (calculated = 857.51).

For mass spectra of polydisperse PEG containing compounds, one single mass peak was selected.

Crosslinker reagent **2q** was prepared from isopropylmalonic acid monobenzyl ester and PEG8000 according to the following scheme:

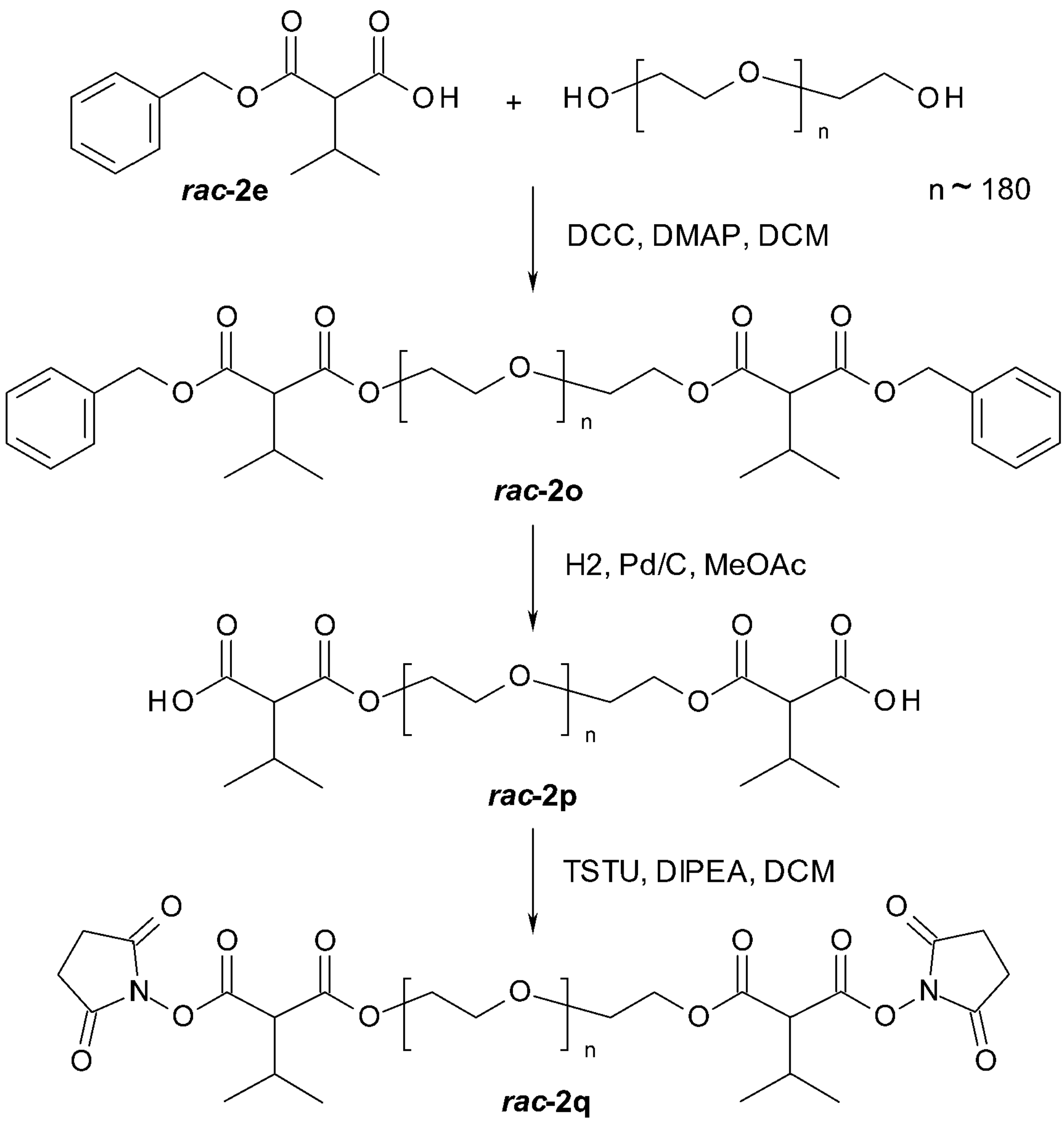


For synthesis of compound ***rac-2o,*** isopropylmalonic acid monobenzyl ester ***rac-2e*** (2.25 g, 9.50 mmol) and PEG 8000 (19.0 g, 2.38 mmol) were dissolved in 100 mL dichloromethane and cooled with an ice bath. A solution of DCC (1.96 g, 9.50 mmol) and DMAP (14 mg, 0.12 mmol) in 10 mL dichloromethane was added. The ice bath was removed and mixture was stirred at room temperature overnight. The resulting suspension was cooled to 0 °C and the solid was filtered off. The solvent was evaporated *in vacuo.*

The residue was dissolved in 40 mL dichloromethane and diluted with 270 mL MTBE at room temperature. The mixture was stored over night at -20 °C. The precipitate was collected by filtration through a glass filter Por. 3, and washed with 500 mL of cooled MTBE (-20 °C). The product was dried *in vacuo* over night.

Yield 18.5 g (92%) white powder ***rac-2o.***

MS: m/z 737.43 =$[M+13H]^{13+}$ (calculated = 737.42).

For mass spectra of polydisperse PEG containing compounds, one single mass peak was selected.

For synthesis of compound ***rac-2p,*** compound ***rac-2o*** (18.4 g, 2.18 mmol) was dissolved in methyl acetate (160 mL) and 254 mg of palladium on charcoal was added. Under a hydrogen atmosphere of ambient pressure, the mixture was stirred overnight at room temperature. The reaction mixture was filtered through a pad of celite and the filtrate was evaporated and dried *in vacuo* over night.

Yield 17.7 g (98%) glassy solid ***rac-2p.***

MS: m/z 723.51 =$[M+13H]^{13+}$ (calculated = 723.55).

For mass spectra of polydisperse PEG containing compounds, one single mass peak was selected.

For synthesis of compound ***rac*-2q**, compound ***rac*-2p** (13.6 g, 1.65 mmol) and TSTU (1.96 g, 6.60 mmol) were dissolved in 60 mL dichloromethane at room temperature. Then DIPEA (852 mg, 6.60 mmol) was added and the mixture

was stirred for 45 min. The resulting suspension was filtered, the filtrate was diluted with 70 mL ethyl acetate and washed with 70 mL of a 0.5 M phosphate buffer pH = 6.5. The organic phase was dried over $MgSO_4$ and the solvent was evaporated *in vacuo.* The residue was dissolved in 80 mL toluene, the remaining solid was filtered off and washed with 20 mL of toluene. The combined toluene fractions were diluted with 35 mL MTBE at room temperature and stored over night at - 20 °C. The precipitate was collected by filtration through a glass filter Por. 3, and washed with 600 mL of cooled MTBE (-20 °C). The product was dried *in vacuo* over night.

Yield 12.1 g (87%) white powder ***rac-2q.***

MS: m/z 738.51 =$[M+13H]^{13+}$ (calculated = 738.49).

For mass spectra of polydisperse PEG containing compounds, one single mass peak was selected.

Crosslinker reagent 2t was prepared from isopropylmalonic acid monobenzyl ester and PEG10000 according to the following scheme:

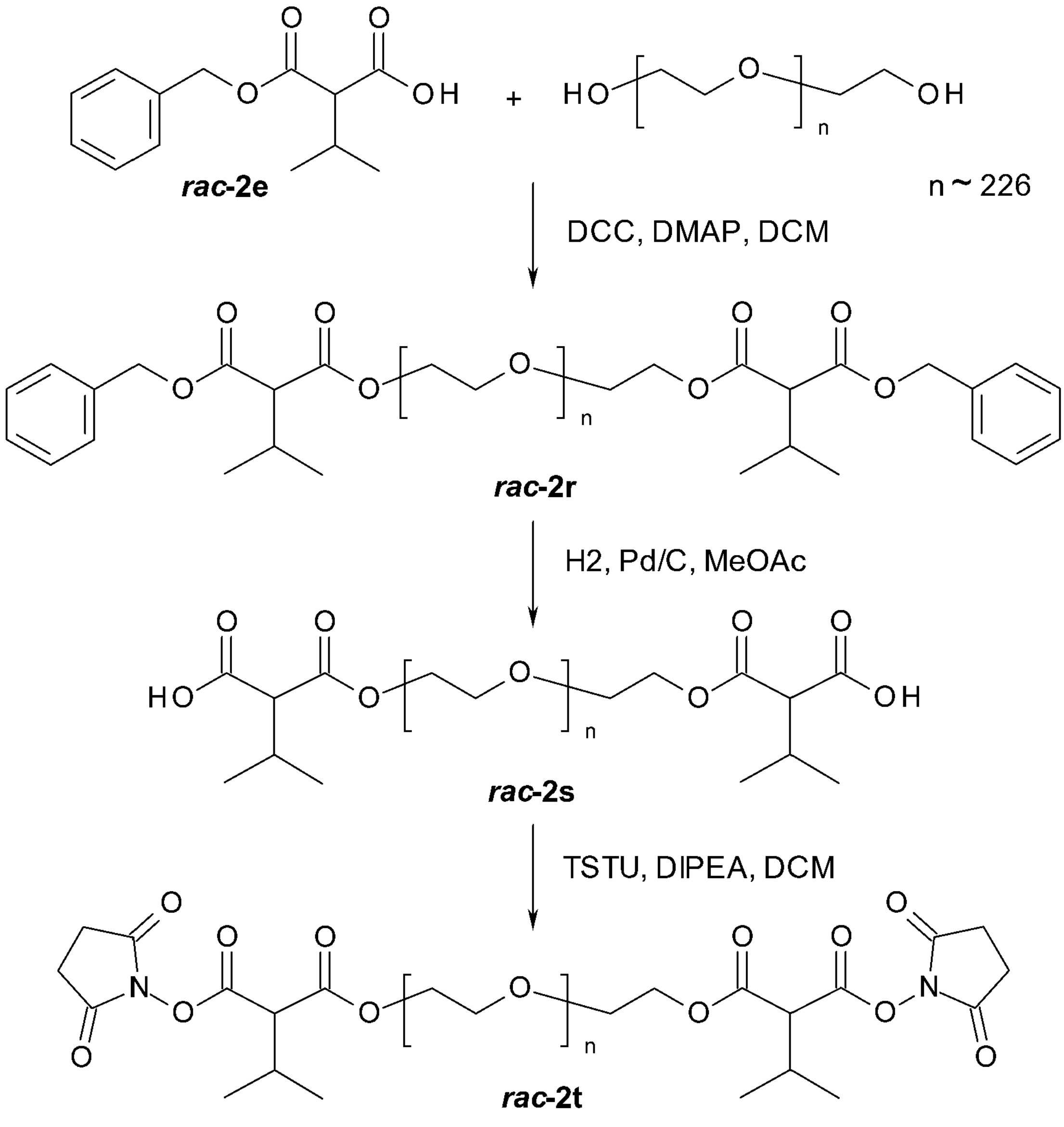


***rac*-2e**

***rac*-2r**

***rac*-2s**

***rac*-2t**

For synthesis of compound ***rac-2r,*** isopropylmalonic acid monobenzyl ester ***rac-2e*** (945 mg, 4.00 mmol) and PEG 10000 (10.0 g, 4.00 mmol) were dissolved in 20 mL dichloromethane and cooled with an ice bath. A solution of DCC (825 mg, 4.00 mmol) and DMAP (6 mg, 0.05 mmol) in 10 mL dichloromethane was added. The ice bath was removed and mixture was stirred at room temperature overnight. The resulting suspension was cooled to 0 °C and the solid was filtered off. The solvent was evaporated *in vacuo.*

The residue was dissolved in 20 mL dichloromethane and diluted with 150 mL MTBE at room temperature. The mixture was stored over night at -20 °C. The precipitate was collected by filtration through a glass filter Por. 3, and washed with 500 mL of cooled MTBE (-20 °C). The product was dried *in vacuo* over night.

Yield 9.63 g (92%) white powder ***rac-2r.***

MS: m/z 742.50 =$[M+16H]^{16+}$ (calculated = 742.51).

For mass spectra of polydisperse PEG containing compounds, one single mass peak was selected.

For synthesis of compound ***rac-2s,*** compound ***rac-2r*** (3.38 g, 0.323 mmol) was dissolved in methyl acetate (100 mL) and 105 mg of palladium on charcoal was added. Under a hydrogen atmosphere of ambient pressure, the mixture was stirred overnight at room temperature. The reaction mixture was filtered through a pad of celite and the filtrate was evaporated and dried *in vacuo* over night.

Yield 3.25 g (98%) glassy solid ***rac-2s.***

MS: m/z 731.25 =$[M+16H]^{16+}$ (calculated = 731.25).

For mass spectra of polydisperse PEG containing compounds, one single mass peak was selected.

For synthesis of compound ***rac-2t,*** compound ***rac-2s*** (3.10 g, 0.302 mmol) and TSTU (0.364 g, 1.21 mmol) were dissolved in 15 mL dichloromethane at room temperature. Then DIPEA (0.156 g, 1.21 mmol) was added and the mixture was stirred for 45 min. The resulting suspension was filtered and the filtrate was washed with 2 × 10 mL of a 0.5 M phosphate buffer pH = 6.5. The organic phase was dried over $MgSO_4$ and the solvent was evaporated *in vacuo.* The residue was dissolved in 20 mL toluene, diluted with 10 mL MTBE at room temperature and stored over night at -20 °C. The precipitate was collected by filtration through a glass filter Por. 3, and washed with 250 mL of cooled MTBE (-20 °C). The product was dried *in vacuo* over night.

Yield 2.66 g (84%) white powder ***rac-2t.***

MS: m/z 743.37 =$[M+16H]^{16+}$ (calculated = 743.38).

For mass spectra of polydisperse PEG containing compounds, one single mass peak was selected.

**Example 3**

**Preparation of hydrogel beads 3a containing free amino groups.**

In a cylindrical 250 mL reactor with bottom outlet, diameter 60 mm, equipped with baffles, an emulsion of 258 mg Cithrol™ DPHS in 100 mL undecane was stirred with an isojet stirrer, diameter 50 mm at 750 rpm, at ambient temperature. A solution of 2400 mg **Ia** and 3120 mg **2d** in 22.1 g DMSO was added and stirred for 10 min to form a suspension. 10.7 mL TMEDA were added to effect polymerization. The mixture was stirred for 16 h at ambient temperature. 16.5 mL of acetic acid were added and then after 10 min 100 mL of a 15wt% solution of sodium chloride in water was added. After 10 min, the stirrer was stopped and phases were allowed to separate. After 2 h the aqueous phase containing the hydrogel was drained.

For bead size fractionation, the water-hydrogel suspension was diluted with 50 mL ethanol and wet-sieved on 100, 75, 63, 50, 40, and 32 μm steel sieves using a Retsch AS200 control sieving machine for 15 min. Sieving amplitude was 1.5 mm, eluent was 3 L of 15 wt% aqueous NaCl solution, then 1 L of pure water, both with a flow of 300 mL/min. Bead fractions that were retained on the 40, 50, 63, and 75 μm sieves were washed 3 times with 0.1% AcOH, 10 times with ethanol and dried for 16 h at 0.1 mbar to give 0.65 g, 0.82 g, 0.42 g, and 0.07 g respectively, of **3a** as a white powder.

Amino group content of the hydrogel was of the 75 μm fraction was determined to be 1.003 mmol/g by conjugation of a Fmoc-amino acid to the free amino groups on the hydrogel and subsequent Fmoc-determination as described by Gude, M., J. Ryf, et al. (2002) Letters in Peptide Science 9(4): 203-206.

**Preparation of hydrogel beads 3b containing free amino groups.**

**3b** was prepared as described for **3a** except applying a stirrer speed of 740 rpm, the use of 2570 mg **Ib,** 3341 mg **2d,** 23.6 g DMSO, 257 mg Cithrol™ DPHS, 11.5 mL TMEDA, 17.6 mL acetic acid, yielding 0.20 g on the 40 μm sieve, 0.37 g on the 50 μm sieve, 0.81 g on the 63 μm sieve, and 0.68 g on the 75 μm sieve of **3b** as a white powder, free amino groups 1.020 mmol/g.

**Preparation of hydrogel beads 3c containing free amino groups.**

**3c** was prepared as described for **3a** except applying a stirrer speed of 480 rpm, the use of 1000 mg **Ib,** 4466 mg ***rac*-2h,** 49.2 g DMSO, 486 mg Cithrol™ DPHS, 4.5 mL TMEDA, 6.9 mL acetic acid, sieving on 125, 100, 75, 63, 50, 40, and 32 μm steel sieves using 4 L of pure water as an eluent, yielding 0.14 g on the 40 μm sieve, 0.20 g on the 50 μm sieve, 0.26 g on the 63 μm sieve, 1.17 g on the 75 μm sieve, and 0.50 g on the 100 μm sieve of 3c as a white powder, free amino groups 0.201 mmol/g.

**Preparation of hydrogel beads 3d containing free amino groups.**

**3d** was prepared as described for **3c** except using a 1000 mL reactor with 100 mm diameter, applying a stirrer speed of 520 rpm, the use of 565 mg Cithrol™ DPHS in 440 mL undecane, 1000 mg **1b**, 5355 mg **2k,** 57.2 g DMSO, 4.5 mL TMEDA, 6.9 mL acetic acid, addition of 200 mL 15wt% solution of sodium chloride in water and after phase separation addition of 80 mL ethanol, yielding 0.27 g on the 50 μm sieve, 0.69 g on the 63 μm sieve, 1.23 g on the 75 μm sieve, and 0.27 g on the 100 μm sieve of **3d** as a white powder, free amino groups 0.168 mmol/g.

**Preparation of hydrogel beads 3e containing free amino groups.**

3e was prepared as described for **3d** except applying a stirrer speed of 540 rpm, the use of 573 mg Cithrol™, 1000 mg **lb,** 5445 mg **2k,** 58.0 g DMSO, 573 mg Cithrol™ DPHS, yielding 0.34 g on the 40 μm sieve, 0.51 g on the 50 μm sieve, 0.83 g on the 63 μm sieve, and 1.16 g on the 75 μm sieve of **3e** as a white powder, free amino groups 0.142 mmol/g.

**Preparation of hydrogel beads 3f containing free amino groups.**

**3f** was prepared as described for **3c** except applying a stirrer speed of 560 rpm, the use of 398 mg **1b**, 2690 mg **2n,** 27.8 g DMSO, 274 mg Cithrol™ DPHS, 1.8 mL TMEDA, 2.7 mL acetic acid, yielding 0.22 g on the 50 μm sieve, 0.33 g on the 63 μm sieve, and 0.52 g on the 75 μm sieve of **3f** as a white powder, free amino groups 0.152 mmol/g.

**Preparation of hydrogel beads 3g containing free amino groups.**

**3g** was prepared as described for **3c** except applying a stirrer speed of 580 rpm, the use of 250 mg **1b**, 2168 mg ***rac-2q,*** 21.8 g DMSO, 215 mg Cithrol™ DPHS, 1.1 mL TMEDA, 1.7 mL acetic acid, yielding 0.09 g on the 50 μm sieve, 0.17 g on the 63 μm sieve, and 0.54 g on the 75 μm sieve of **3g** as a white powder, free amino groups 0.154 mmol/g.

**Preparation of hydrogel beads 3h containing free amino groups.**

**3h** was prepared as described for **3c** except applying a stirrer speed of 600 rpm, the use of 250 mg **lb,** 2402 mg ***rac-2t,*** 23.9 g DMSO, 235 mg Cithrol™ DPHS, 1.1 mL TMEDA, 1.7 mL acetic acid, yielding 0.27 g on the 63 μm sieve, 0.54 g on the 75 μm sieve, and 0.02 g on the 100 μm sieve of **3h** as a white powder, free amino groups 0.144 mmol/g.

**Example 4**

**Preparation of maleimide functionalized hydrogel beads 4**

228.4 mg of dry weight hydrogel beads **3b** (0.142 mmol/g amino groups/ 0.032 mmol amino groups) were swollen in 10 mL NMP and washed five times with NMP and five times with 2% DIEA in NMP. 108.7 mg (0.162 mmol, 5.1 eq) of Mal-$PEG_6$-Pfp were dissolved in NMP and added to the washed hydrogel beads **3b.** The hydrogel suspension was incubated for 2 h at room temperature. Resulting maleimide functionalized hydrogel beads **4** were washed five times each with NMP and afterwards with 0.1% acetic acid, 0.01% Tween20.

Maleimide content of the hydrogel beads was determined to be 0.1204 mmol/g by conjugation of a Fmoc-cysteine to the maleimide groups on the hydrogel and subsequent Fmoc-determination as described by Gude, M., J. Ryf, et al. (2002) Letters in Peptide Science 9(4): 203-206.

**Example 5**

**Synthesis of PEGylated hydrogel beads 5a-c**

50 mg of maleimide functionalized hydrogel beads **4** ($6.02\times10^{-3}$ mmol maleimide groups) as a suspension in 0.1% acetic acid, 0.01% Tween20 were transferred into a syringe with a frit and the solvent was expelled. The hydrogel was washed ten times with PBS-T/5 mM EDTA/pH 6.5.

PEG-SH was dissolved in 0.5 mL PBS-T/5 mM EDTA/pH 6.5. The PEGsolution was drawn into the syringe and the resulting hydrogel suspension was allowed to incubate for 2.5 hours at ambient temperature under gentle shaking. The solvent was expelled and the hydrogel was washed five times with PBS-T/5 mM EDTA/pH 6.5 and transferred into a 5 mL Sarstedtvial.vial to give a hydrogel suspension of **5a**.

Maleimide content on the hydrogel beads was determined via conjugation of a Fmoc-cysteine to the maleimide

groups on the hydrogel and subsequent Fmoc-determination as described by Gude, M., J. Ryf, et al. (2002) Letters in Peptide Science 9(4): 203-206.

Maleimide content after PEGylation was determined to be 0.0895 mmol/g. Based on equation (2) this refers to a degree of PEGylation of 17.7 % of the initial maleimides can be determined.

**5b** was synthesized according to the procedure described for **5a,** starting with 50 mg of maleimide functionalized hydrogel beads **4** ($6.02\times10^{-3}$ mmol maleimide groups) and proceeding with addition of 15.4 mg 10kDaPEG-SH ($1.54\times10^{-3}$ mmol, 0.26 eq). Maleimide content after PEGylation was determined to be 0.0761 mmol/g. Based on equation (2) this refers to a PEGylation of 20.9 % of the initial maleimides.

**5c** was synthesized according to the procedure described for **5a,** starting with 50 mg of maleimide functionalized hydrogel beads **4** ($6.02\times10^{-3}$ mmol maleimide groups) and proceeding with addition of 26.5 mg 20 kDaPEG-SH ($1.325\times10^{-3}$ mmol, 0.22 eq). Maleimide content after PEGylation was determined to be 0.0951 mmol/g. Based on equation (2) this refers to a PEGylation of 7.2 % of the initial maleimides

**Example 6**

**Synthesis of PEGylated hydrogel beads 6a-c**

75 mg of dry weight hydrogel beads **3a** were transferred into a 10 mL syringe equipped with a frit and 2 NMP were added. The hydrogel was allowed to swell for 10 min at ambient temperature under gentle shaking. The solvent was expelled and the hydrogel washed 10 times with each time 3 mL 1% TMEDA/DMSO, the solvent was each time discarded.

180 mg ($3\times10^{-3}$ mmol, 0.2 eq based on amine content) branched 60kDa PEG-NHS were dissolved in 1 mL DMSO at 37°C and 4.5 μL of TMEDA (0.03 mmol, 3.5mg, 2 eq. based on amine content) were added. The solution was drawn into the syringe and the resulting hydrogel suspension was allowed to incubate for 64 hours at ambient temperature under gentle shaking. The solvent was expelled and the hydrogel was washed 5 times with each time 3 mL DMSO, followed by 5 washing cycles with each time 3 mL 0.1% acetic acid/0.01% Tween 20. Fresh 0.1% acetic acid/0.01% Tween 20 was pulled into the syringe the give a suspension of 25 mg/mL hydrogel based on initial weight to give **6a.** Amino content of the hydrogel beads was determined to be 0.19 mmol/g for dry weight hydrogel beads. Based on equation (2) this refers to a PEGylation of 0.4 % of the initial amines.

Preparation of **6b** was performed according to the procedure described for 6a except for the use of 121 mg ($3\times10^{-3}$ mmol, 0.2 eq based on amine content) branched 40kDa PEG-NHS instead of 180 mg ($3\times10^{-3}$ mmol, 0.2 eq based on amine content) branched 60kDa PEG-NHS resulting in an amino content of 0.191 mmol/g for dry weight hydrogel beads. Based on equation (2) this refers to a PEGylation of 0.6 % of the initial amines.

Preparation of 6c was performed according to the procedure described for 6a except for the use of 60.3 mg ($3\times10^{-3}$ mmol, 0.2 eq based on amine content) 20kDa PEG-NHS instead of 180 mg ($3\times10^{-3}$ mmol, 0.2 eq based on amine content) branched 60kDa PEG-NHS resulting in an amino content of 0.126 mmol/g for dry weight hydrogel beads. Based on equation (2) this refers to a PEGylation of 10.6 % of the initial amines.

**Example 7**

**Preparation of maleimide functionalized hydrogel beads** 7

117.7 mg dry hydrogel beads 3e were swollen in 5 mL NMP and washed five times with NMP and five times with 2% DIEA in NMP. 5 eq (56 mg) of Mal-$PEG_6$-Pfp (based on amine content of the hydrogel beads) were dissolved in 0.5 mL NMP and added to the washed hydrogel beads 3e. The hydrogel suspension was incubated for 2.5 h at room temperature. Resulting maleimide functionalized hydrogel beads were washed five times each with NMP and afterwards with 0.1% acetic acid, 0.01% Tween20.

**Example 8**

**General procedure for preparation of PEGylated hydrogel beads via Michael-addition reaction**

Maleimide functionalized hydrogel beads as a suspension in 0.1% acetic acid, 0.01% Tween20 were transferred into a syringe with a frit and the solvent was expelled. The hydrogel was washed ten times with PBS-T/5 mM EDTA/pH 6.5.

0.2 eq PEG-SH (based on maleimide content of the hydrogel beads)was dissolved in PBS-T/5 mM EDTA/pH 6.5 (1 mL/15 mg reagent). The PEGsolution was drawn into the syringe and the resulting hydrogel suspension was allowed to incubate for 3.5 hours at ambient temperature under gentle shaking. The solvent was expelled and the hydrogel was washed five times with PBS-T/5 mM EDTA/pH 6.5 and transferred into a Sarstedt vial.

Maleimide content on the hydrogel beads was determined via conjugation of a Fmoc-cysteine to the maleimide groups on the hydrogel and subsequent Fmoc-determination as described by Gude, M., J. Ryf, et al. (2002) Letters in Peptide Science 9(4): 203-206.

Based on equation (2) degree of PEGylation can be determined.

Example **8a** was prepared according to the procedure described in Example **8** starting with 50 mg (based on dry hydrogel weight) **7** using 13.3 mg 10kDa PEG-SH. The final compound has a maleimide content of 0.0761 mmol/g.

Example **8b** was prepared according to the procedure described in Example **8** starting with 50 mg (based on dry hydrogel weight) **7** using 26.6 mg 20kDa PEG-SH. The final compound has a maleimide content of 0.0951 mmol/g.

## Example 9

### General procedure for the preparation of PEGylated hydrogel beads in DMSO

Dry hydrogel beads as e.g. described in **3a** were transferred into a syringe equipped with a frit and NMP (5 mL/100 mg hydrogel beads) was added. The hydrogel was allowed to swell for 10 min at ambient temperature under gentle shaking. The solvent was expelled and the hydrogel washed ten times with each time DMSO, followed by ten washes with each time 1% TMEDA/DMSO (5 mL/100 mg hydrogel beads), the solvent was each time discarded.

0.2 eq (based on amine content of the hydrogel beads) PEG-NHS were dissolved in a solution containing 2 eq (based on amine content of the hydrogel beads) TMEDA in DMSO at 37°C for 15 min. The solution was drawn into the syringe and the resulting hydrogel suspension was allowed to incubate under gentle shaking. The solvent was expelled and the hydrogel was washed five times with DMSO (5 mL/100 mg hydrogel beads), followed by five washing cycles with 0.1% acetic acid/0.01% Tween 20 (5 mL/100 mg hydrogel beads). Fresh 0.1% acetic acid/0.01% Tween 20 was pulled into the syringe to give a suspension of 10 mg/mL hydrogel based on initial weight. Amine content of the hydrogel beads was determined as described above. Based on equation (2) this refers to the degree of PEGylation.

Example **9a** was prepared according to the general procedure in Example **9** with 80 mg hydrogel **3b** and 326.4 mg 20 kDa PEG-NHS with a reaction time of 16h resulting in a PEGylated hydrogel with an amine content of 0.862 mmol/g. Based on equation (2) 0.8% of the amines have been PEGylated.

Example **9b** was prepared according to the general procedure in Example **9** with 80 mg hydrogel **3b** and 163.2 mg 10 kDa PEG-NHS with a reaction time of 16h resulting in a PEGylated hydrogel with an amine content of 0.756 mmol/g. Based on equation (2) 3.0% of the amines have been PEGylated.

Example **9c** was prepared according to the general procedure in Example **9** with 50 mg hydrogel **3a** and 200 mg branched 20 kDa PEG-NHS with a reaction time of 16h resulting in a PEGylated hydrogel with an amine content of 0.932 mmol/g. Based on equation (2) 0.4% of the amines have been PEGylated.

Example **9d** was prepared according to the general procedure in Example **9** with 75 mg hydrogel **3c** and 180 mg branched 60 kDa PEG-NHS with a reaction time of 64h resulting in a PEGylated hydrogel with an amine content of 0.190 mmol/g. Based on equation (2) 0.4% of the amines have been PEGylated.

Example **9e** was prepared according to the general procedure in Example 9 with 75 mg hydrogel **3c** and 120.6 mg branched 40 kDa PEG-NHS with a reaction time of 64h resulting in a PEGylated hydrogel with an amine content of 0.191 mmol/g. Based on equation (2) 0.6% of the amines have been PEGylated.

Example **9f** was prepared according to the general procedure in Example **9** with 75 mg hydrogel **3c** and 60.3 mg 20 kDa PEG-NHS with a reaction time of 64h resulting in a PEGylated hydrogel with an amine content of 0.126 mmol/g. Based on equation (2) 10.6% of the amines have been PEGylated.

Example **9g** was prepared according to the general procedure in Example **9** with 25 mg hydrogel **3d** and 50.3 mg branched 60 kDa PEG-NHS with a reaction time of 3h. The amine content of the PEGylated hydrogel was not determined.

Example **9h** was prepared according to the general procedure in Example **9** with 25 mg hydrogel **3d** and 50.3 mg branched 60 kDa PEG-NHS with a reaction time of 1h followed by addition of 33.6 mg branched 40 kDa PEG-NHS with a reaction time of 2h. The amine content of the PEGylated hydrogel was not determined.

Example **9i** was prepared according to the general procedure in Example **9** with 25 mg hydrogel **3d** and 50.3 mg branched 60 kDa PEG-NHS with a reaction time of 1h followed by addition of 33.6 mg branched 40 kDa PEG-NHS with a reaction time of 1h followed by addition of 16.8 mg branched 20 kDa PEG-NHS with a reaction time of 1h. The amine content of the PEGylated hydrogel was not determined.

Example **9j** was prepared according to the general procedure in Example **9** with 50 mg hydrogel **3f** and 30.4 mg 20 kDa PEG-NHS with a reaction time of 72h resulting in a PEGylated hydrogel with an amine content of 0.125 mmol/g. Based on equation (2) 0.8% of the amines have been PEGylated. Based on equation (2) 5.1% of the amines have been PEGylated.

Example **9k** was prepared according to the general procedure in Example **9** with 50 mg hydrogel **3f** and 45.6 mg 30 kDa PEG-NHS with a reaction time of 72h resulting in a PEGylated hydrogel with an amine content of 0.133

mmol/g. Based on equation (2) 2.5% of the amines have been PEGylated.

Example **9l** was prepared according to the general procedure in Example **9** with 50 mg hydrogel **3f** and 60.8 mg 40 kDa PEG-NHS with a reaction time of 72h resulting in a PEGylated hydrogel with an amine content of 0.141 mmol/g. Based on equation (2) 1.1% of the amines have been PEGylated.

Example **9m** was prepared according to the general procedure in Example **9** with 50 mg hydrogel **3f** and 121.6 mg branched 80 kDa PEG-NHS with a reaction time of 72h resulting in a PEGylated hydrogel with an amine content of 0.131 mmol/g. Based on equation (2) 1.2% of the amines have been PEGylated.

Example **9n** was prepared according to the general procedure in Example **9** with 50 mg hydrogel **3f** and 91.2 mg branched 60 kDa PEG-NHS with a reaction time of 72h resulting in a PEGylated hydrogel with an amine content of 0.137 mmol/g. Based on equation (2) 1.1% of the amines have been PEGylated.

Example **9o** was prepared according to the general procedure in Example **9** with 50 mg hydrogel **3g** and 29.6 mg 20 kDa PEG-NHS with a reaction time of 72h resulting in a PEGylated hydrogel with an amine content of 0.096 mmol/g. Based on equation (2) 12.9% of the amines have been PEGylated.

Example **9p** was prepared according to the general procedure in Example **9** with 50 mg hydrogel **3g** and 47.4 mg 30 kDa PEG-NHS with a reaction time of 72h resulting in a PEGylated hydrogel with an amine content of 0.098 mmol/g. Based on equation (2) 9.2% of the amines have been PEGylated.

Example **9q** was prepared according to the general procedure in Example **9** with 50 mg hydrogel **3g** and 59.5 mg 40 kDa PEG-NHS with a reaction time of 72h resulting in a PEGylated hydrogel with an amine content of 0.118 mmol/g. Based on equation (2) 4.1% of the amines have been PEGylated.

Example **9r** was prepared according to the general procedure in Example **9** with 50 mg hydrogel **3g** and 88.2 mg branched 60 kDa PEG-NHS with a reaction time of 72h resulting in a PEGylated hydrogel with an amine content of 0.121 mmol/g. Based on equation (2) 2.6% of the amines have been PEGylated.

Example **9s** was prepared according to the general procedure in Example **9** with 50 mg hydrogel **3g** and 128.3 mg branched branched 80 kDa PEG-NHS with a reaction time of 72h resulting in a PEGylated hydrogel with an amine content of 0.116 mmol/g. Based on equation (2) 2.4% of the amines have been PEGylated.

Example **9t** was prepared according to the general procedure in Example **9** with 40 mg hydrogel **3g** and 50 mg branched 40 kDa PEG-NHS with a reaction time of 72h resulting in a PEGylated hydrogel with an amine content of 0.114 mmol/g. Based on equation (2) 4.7% of the amines have been PEGylated.

Example **9u** was prepared according to the general procedure in Example **9** with 40 mg hydrogel **3g** and 50 mg branched 40 kDa PEG-NHS with a reaction time of 72h at 37°C resulting in a PEGylated hydrogel with an amine content of 0.132 mmol/g. Based on equation (2) 2.3% of the amines have been PEGylated.

Example **9v** was prepared according to the general procedure in Example **9** with 40 mg hydrogel **3g** and 25 mg branched 40 kDa PEG-NHS with a reaction time of 24h after which an additional 25 mg branched 40 kDa PEG-NHS were added and the reaction was allowed to proceed for another 24h after which an additional 25 mg branched 40 kDa PEG-NHS were added and the reaction was allowed to proceed for another 24h resulting in a PEGylated hydrogel with an amine content of 0.119 mmol/g. Based on equation (2) 3.9% of the amines have been PEGylated.

Example **9w** was prepared according to the general procedure in Example **9** with 40 mg hydrogel **3g** and 25 mg branched 40 kDa PEG-NHS with a reaction time of 24h at 37°C after which an additional 25 mg branched 40 kDa PEG-NHS were added and the reaction was allowed to proceed for another 24h at 37°C after which an additional 25 mg branched 40 kDa PEG-NHS were added and the reaction was allowed to proceed for another 24h resulting at 37°C in a PEGylated hydrogel with an amine content of 0.118 mmol/g. Based on equation (2) 4.1% of the amines have been PEGylated.

Example **9x** was prepared according to the general procedure in Example **9** with 70 mg hydrogel **3h** and 89 mg branched 40 kDa PEG-NHS with a reaction time of 48h resulting in a PEGylated hydrogel with an amine content of 0.108 mmol/g. Based on equation (2) 6.0% of the amines have been PEGylated.

Example **9y** was prepared according to the general procedure in Example **9** with 70 mg hydrogel **3h** and 133.6 mg branched 60 kDa PEG-NHS with a reaction time of 48h resulting in a PEGylated hydrogel with an amine content of 0.114 mmol/g. Based on equation (2) 3.6% of the amines have been PEGylated.

Example **9z** was prepared according to the general procedure in Example **9** with 70 mg hydrogel **3h** and 89 mg 40 kDa PEG-NHS with a reaction time of 48h resulting in a PEGylated hydrogel with an amine content of 0.085 mmol/g. Based on equation (2) 10.6% of the amines have been PEGylated.

Example **9aa** was prepared according to the general procedure in Example **9** with 70 mg hydrogel **3h** and 44.5 mg 20 kDa PEG-NHS with a reaction time of 48h resulting in a PEGylated hydrogel with an amine content of 0.125 mmol/g. Based on equation (2) 6.1% of the amines have been PEGylated.

**Example 10**

**General procedure for the preparation of PEGylated hydrogel beads under aqueous conditions**

Dry hydrogel beads as e.g. described in **3a** were transferred into a syringe equipped with a frit and NMP (5 mL/100 mg hydrogel beads) was added. The hydrogel was allowed to swell for 10 min at ambient temperature under gentle shaking. The solvent was expelled and the hydrogel washed ten times with 50 mM phosphate/acetonitrile pH 7.4, the solvent was discarded each time.

0.2 eq (based on amine content of the hydrogel beads) PEG-NHS were dissolved in 50 mM phosphate/acetonitrile pH 7.4. The solution was drawn into the syringe and the resulting hydrogel suspension was allowed to incubate under gentle shaking. The solvent was expelled and the hydrogel was washed five times with 50 mM phosphate/acetonitrile pH 7.4 (5 mL/100 mg hydrogel beads), followed by five washing cycles with 0.1% acetic acid/0.01% Tween 20 (5 mL/100 mg hydrogel beads). Fresh 0.1% acetic acid/0.01% Tween 20 was pulled into the syringe to give a suspension of 10 mg/mL hydrogel based on initial weight. Amine content of the hydrogel beads was determined as described above. Based on equation (2) this refers to the degree of PEGylation.

Example **10a** was prepared according to the general procedure in Example **10** with 21.9 mg hydrogel **3d** and 44 mg branched 60 kDa PEG-NHS with a reaction time of 20h. The amine content of the PEGylated hydrogel was not determined.

Example **10b** was prepared according to the general procedure in Example **10** with 40 mg hydrogel **3g** and 25 mg branched 40 kDa PEG-NHS with a reaction time of 24h after which an additional 25 mg branched 40 kDa PEG-NHS were added and the reaction was allowed to proceed for another 24h after which an additional 25 mg branched 40 kDa PEG-NHS were added and the reaction was allowed to proceed for another 24h resulting in a PEGylated hydrogel with an amine content of 0.136 mmol/g. Based on equation (2) 1.8% of the amines have been PEGylated.

**Example 11**

**General procedure for the preparation of maleimide functionalized PEGylated hydrogel beads**

PEGylated hydrogel beads as a suspension of 10 mg/mL based on initial weight of hydrogel beads prior to PEGylation were transfered into a syringe equipped with a frit. The solvent was expelled and the hydrogel washed ten times with water (5 mL/100 mg hydrogel beads), the solvent was discarded each time. The hydrogel beads were then washed ten times with NMP and five times with 2% DIEA in NMP. 5 eq of Mal-$PEG_6$-Pfp (based on amine content of the hydrogel beads) were dissolved in NMP (1 mL/50 mg reagent) and added to the washed hydrogel beads. The hydrogel suspension was incubated for 2 h at room temperature. Resulting maleimide functionalized hydrogel beads were washed five times each with NMP and afterwards with 0.1% acetic acid/ 0.01% Tween20.

Maleimide content of hydrogel beads was determined by conjugation of a Fmoc-cysteine to the maleimide groups on the hydrogel and subsequent Fmoc-determination as described by Gude, M., J. Ryf, et al. (2002) Letters in Peptide Science 9(4): 203-206.

Example **11a** was prepared according to the general procedure described in Example **11** starting with 47 mg of **9d** (based on dry weight **3c**) using 31.7 mg Mal-$PEG_6$-Pfp.

Example **11b** was prepared according to the general procedure described in Example **11** starting with 47 mg of **9e** (based on dry weight **3c**) using 31.7 mg Mal-$PEG_6$-Pfp.

Example **11c** was prepared according to the general procedure described in Example **11** starting with 47 mg of **9f** (based on dry weight **3c**) using 31.7 mg Mal-$PEG_6$-Pfp.

Example **11d** was prepared according to the general procedure described in Example **11** starting with 25 mg of **9g** (based on dry weight **3d**) using 13.5 mg Mal-$PEG_6$-Pfp.

Example **11e** was prepared according to the general procedure described in Example **11** starting with 25 mg of **9h** (based on dry weight **3d**) using 13.5 mg Mal-$PEG_6$-Pfp.

Example **11f** was prepared according to the general procedure described in Example **11** starting with 25 mg of **9g** (based on dry weight **3d**) using 13.5 mg Mal-$PEG_6$-Pfp.

Example **11g** was prepared according to the general procedure described in Example **11** starting with 21.9 mg of **10a** (based on dry weight **3d)** using 13.5 mg Mal-$PEG_6$-Pfp. Example **11h** was prepared according to the general procedure described in Example **11** starting with 35 mg of **9j** (based on dry weight **3f)** using 13 mg Mal-$PEG_6$-Pfp.

Example **11i** was prepared according to the general procedure described in Example **11** starting with 35 mg of **9k** (based on dry weight **3f)** using 13 mg Mal-$PEG_6$-Pfp.

Example **11j** was prepared according to the general procedure described in Example **11** starting with 35 mg of **9l** (based on dry weight **3f)** using 13 mg Mal-$PEG_6$-Pfp.

Example **11k** was prepared according to the general procedure described in Example **11** starting with 35 mg

of **9m** (based on dry weight **3f)** using 13 mg Mal-PEG$_6$-Pfp.

Example **11l** was prepared according to the general procedure described in Example **11** starting with 35 mg of **9n** (based on dry weight 3f) using 13 mg Mal-PEG$_6$-Pfp.

Example **11m** was prepared according to the general procedure described in Example **11** starting with 35 mg of **9o** (based on dry weight 3g) using 17 mg Mal-PEG$_6$-Pfp.

Example **11n** was prepared according to the general procedure described in Example **11** starting with 35 mg of **9p** (based on dry weight **3g)** using 17 mg Mal-PEG$_6$-Pfp.

Example **11o** was prepared according to the general procedure described in Example **11** starting with 35 mg of **9q** (based on dry weight **3g)** using 17 mg Mal-PEG$_6$-Pfp.

Example **11p** was prepared according to the general procedure described in Example **11** starting with 35 mg of **9r** (based on dry weight **3g)** using 17 mg Mal-PEG$_6$-Pfp.

Example **11q** was prepared according to the general procedure described in Example **11** starting with 35 mg of **9s** (based on dry weight **3g)** using 17 mg Mal-PEG$_6$-Pfp.

Example **11r** was prepared according to the general procedure described in Example **11** starting with 25 mg of **9t** (based on dry weight **3g)** using 13 mg Mal-PEG$_6$-Pfp.

Example **11s** was prepared according to the general procedure described in Example **11** starting with 25 mg of **9u** (based on dry weight 3g) using 13 mg Mal-PEG$_6$-Pfp.

Example **11t** was prepared according to the general procedure described in Example **11** starting with 25 mg of **9v** (based on dry weight 3g) using 13 mg Mal-PEG$_6$-Pfp.

Example **11u** was prepared according to the general procedure described in Example **11** starting with 25 mg of **9w** (based on dry weight 3g) using 13 mg Mal-PEG$_6$-Pfp.

Example **11v** was prepared according to the general procedure described in Example **11** starting with 25 mg of **10b** (based on dry weight **3g)** using 13 mg Mal-PEG$_6$-Pfp.

Example **11w** was prepared according to the general procedure described in Example **11** starting with 40 mg of **9x** (based on dry weight **3h)** using 21 mg Mal-PEG$_6$-Pfp.

Example **11w** was prepared according to the general procedure described in Example **11** starting with 40 mg of **9x** (based on dry weight **3h)** using 21 mg Mal-PEG$_6$-Pfp.

Example **11x** was prepared according to the general procedure described in Example **11** starting with 40 mg of **9y** (based on dry weight **3h)** using 21 mg Mal-PEG$_6$-Pfp.

Example **11y** was prepared according to the general procedure described in Example **11** starting with 40 mg of **9z** (based on dry weight **3h)** using 21 mg Mal-PEG$_6$-Pfp.

Example **11z** was prepared according to the general procedure described in Example **11** starting with 40 mg of **9aa** (based on dry weight **3h)** using 21 mg Mal-PEG$_6$-Pfp.

## Example 12

### General procedure for the preparation of Insulin PEGylated hydrogel beads

PEGylated hydrogel beads were synthesized according to Example 9. Hydrogel beads as a suspension in 0.1 % acetic acid/0.01% Tween20 were then transferred in to a syringe equipped with a frit and the solvent was discarded. The hydrogel beads were washed five times with water (2 mL/10mg hydrogel beads), five times with NMP (2 mL/10mg hydrogel beads), five times with 2% DIEA in NMP (2 mL/10mg hydrogel beads) and five times with DMSO (2 mL/10mg hydrogel beads). The solvent was each time discarded. Based on amine content of the hydrogel beads, 3 eq of N-maleimido propionic acid NHS-ester were dissolvend in DMSO (750 μL DMSO /10 mg N-maleimido propionic acid NHS-ester) and the solution was drawn into the syringe and allowed to incubate under gentle shaking for 1.5 h at ambient temperature. The solvent was discarded and the hydrogel beads were washed five times with DMSO (2 mL/10mg hydrogel beads) and 0.1% acetic acid/0.01% Tween20 (2 mL/10mg hydrogel beads). The solvent was discarded each time. A fresh solution of 0.1% acetic acid/0.01% Tween20 was drawn into the syringe and the suspension was transferred in to a Falcon tube to give a final concentration of 10 mg/mL. Until further use, the suspension was stored at 4°C.

Linker conjugation of insulin with 6-tritylmercaptohexanoic acid NHS-ester and deprotection of the Trityl protecting group was performed according to the procedure described in WO2011/012719 example 10. Conjugation of the insulin-linker-conjugate to the maleimide functionalized hydrogel beads was performed according to the procedure described in WO2011/012719 example 11dc.

Example **12a** was prepared according to the procedure described in the general procedure in example **12** starting with 10 mg hydrogel **9a** (based on initial dry weight of **3b**) using 4.5 mg deprotected insulin-linker-conjugate to give a PEGylated hydrogel loaded with 3.5 mg insulin-linker-conjugate.

Example **12b** was prepared according to the procedure described in the general procedure in example **12** starting with 10 mg hydrogel **9b** (based on initial dry weight of **3b**) using 4.5 mg deprotected insulin-linker-conjugate to

give a PEGylated hydrogel loaded with 3.5 mg insulin-linker-conjugate.

Example **12c** was prepared according to the procedure described in the general procedure in example **12** starting with 10 mg hydrogel **9c** (based on initial dry weight of **3a**) using 3.9 mg deprotected insulin-linker-conjugate to give a PEGylated hydrogel loaded with 3.6 mg insulin-linker-conjugate.

**Example 13**

**General procedure for the preparation of IL-1RA PEGylated hydrogel beads**

A commercially available IL-1RA solution was buffer exchanged towards PBS-T/5 mM EDTA/pH 6.5.

Maleimide functionalized (PEGylated) hydrogel beads as a suspension in 0.1% HOAc/0.01% Tween20 were transferred into a syringe equipped with a frit. The solvent was discarded and the protein solution drawn into the syringe. The resulting suspension was allowed to incubate at ambient temperature under gentle shaking. The solvent was expelled and the hydrogel was was washed ten times with 1 mL of PBS-T/5 mM EDTA/pH 6.5, the solvent was discarded each time. The hydrogel was washed five times with 10 mM histidine/10 wt% $\alpha,\alpha$-trehalose/0.01% Tween20/pH 5.5, the solvent was discarded each time. The hydrogel was treated ten times x 3 min with each time 1 mL 1 mM $\beta$-mercaptoethanol in 10 mM histidine/10 wt% $\alpha,\alpha$-trehalose/0.01% Tween20/pH 5.5, the solvent was discarded each time. The hydrogel was washed ten times with 2 mL 10 mM histidine/10 wt% $\alpha,\alpha$-trehalose/0.01% Tween20/pH 5.5, the solvent was discarded each time. Subsequently, the hydrogel was washed five times with 2 mL PBS-T pH 7.4, the solvent was discarded each time. 2 mL of fresh buffer were drawn into the syringe and the resulting hydrogel suspension was transferred into a Sarstedt vial. IL-1RA content within the hydrogel was calculated based on initial IL-1RA content correlated to IL-1RA content within the washing solutions, determined via A280 absorption (extinction coefficient of IL-1RA: 15.470 L/(mol x cm), molecular weight 17.260 Da) to give the protein load.

Example **13a** was prepared according to the procedure described in Example **13** using 9.3 mg IL-1RA and 5.2 mg **11a** (based on dry weight of **3c**) resulting in a hydrogel loaded with 8.8 mg IL-1RA.

Example **13b** was prepared according to the procedure described in Example **13** using 9.4 mg IL-1RA and 5.6 mg **11b** (based on dry weight of **3c**) resulting in a hydrogel loaded with 8.9 mg IL-1RA.

Example **13c** was prepared according to the procedure described in Example **13** using 9.4 mg IL-1RA and 5.6 mg **11c** (based on dry weight of **3c**) resulting in a hydrogel loaded with 8.6 mg IL-1RA.

Example **13d** was prepared according to the procedure described in Example **13** using 7.7 mg IL-1RA and 4 mg **11d** (based on dry weight of **3d**) resulting in a hydrogel loaded with 5.3 mg IL-1RA.

Example **13e** was prepared according to the procedure described in Example **13** using 7.7 mg IL-1RA and 4 mg **11e** (based on dry weight of **3d**) resulting in a hydrogel loaded with 4.7 mg IL-1RA.

Example **13f** was prepared according to the procedure described in Example **13** using 7.7 mg IL-1RA and 4 mg **11f** (based on dry weight of **3d**) resulting in a hydrogel loaded with 4.5 mg IL-1RA.

Example **13g** was prepared according to the procedure described in Example **13** using 8.3 mg IL-1RA and 5 mg **11g** (based on dry weight of **3d**) resulting in a hydrogel loaded with 6.3 mg IL-1RA.

Example **13h** was prepared according to the procedure described in Example **13** using 9.3 mg IL-1RA and 5 mg **8a** (based on dry weight of **3e**) resulting in a hydrogel loaded with 3.4 mg IL-1RA.

Example **13i** was prepared according to the procedure described in Example **13** using 9.3 mg IL-1RA and 5 mg **8b** (based on dry weight of **3e**) resulting in a hydrogel loaded with 3.8 mg IL-1RA.

Example **13j** was prepared according to the procedure described in Example **13** using 19 mg IL-1RA and 10 mg **11h** (based on dry weight of **3f**) resulting in a hydrogel loaded with 11.2 mg IL-1RA.

Example **13k** was prepared according to the procedure described in Example **13** using 19 mg IL-1RA and 10 mg **11i** (based on dry weight of **3f**) resulting in a hydrogel loaded with 11.7 mg IL-1RA.

Example **13l** was prepared according to the procedure described in Example **13** using 19 mg IL-1RA and 10 mg **11j** (based on dry weight of **3f**) resulting in a hydrogel loaded with 12.0 mg IL-1RA.

Example **13m** was prepared according to the procedure described in Example **13** using 19 mg IL-1RA and 10 mg **11k** (based on dry weight of **3f**) resulting in a hydrogel loaded with 11.8 mg IL-1RA.

Example **13n** was prepared according to the procedure described in Example **13** using 19 mg IL-1RA and 10 mg **11l** (based on dry weight of **3f**) resulting in a hydrogel loaded with 12.4 mg IL-1RA.

Example **13o** was prepared according to the procedure described in Example **13** using 15.1 mg IL-1RA and 10 mg **11m** (based on dry weight of **3g**) resulting in a hydrogel loaded with 10.7 mg IL-1RA.

Example **13p** was prepared according to the procedure described in Example **13** using 15.1 mg IL-1RA and 10 mg **11n** (based on dry weight of **3g**) resulting in a hydrogel loaded with 11.5 mg IL-1RA.

Example **13q** was prepared according to the procedure described in Example **13** using 15.1 mg IL-1RA and 10 mg **11o** (based on dry weight of **3g**) resulting in a hydrogel loaded with 12.0 mg IL-1RA.

Example **13r** was prepared according to the procedure described in Example **13** using 15.1 mg IL-1RA and 10

mg **11p** (based on dry weight of **3g**) resulting in a hydrogel loaded with 11.9 mg IL-1RA.

Example **13s** was prepared according to the procedure described in Example **13** using 15.1 mg IL-1RA and 10 mg **11q** (based on dry weight of **3g**) resulting in a hydrogel loaded with 10.7 mg IL-1RA.

Example **13t** was prepared according to the procedure described in Example **13** using 11.7 mg IL-1RA and 5 mg **11r** (based on dry weight of **3g**) resulting in a hydrogel loaded with 8.0 mg IL-1RA.

Example **13u** was prepared according to the procedure described in Example **13** using 11.7 mg IL-1RA and 5 mg **11s** (based on dry weight of **3g**) resulting in a hydrogel loaded with 6.7 mg IL-1RA.

Example **13v** was prepared according to the procedure described in Example **13** using 11.7 mg IL-1RA and 5 mg **11t** (based on dry weight of **3g**) resulting in a hydrogel loaded with 11.5 mg IL-1RA.

Example **13w** was prepared according to the procedure described in Example **13** using 11.7 mg IL-1RA and 5 mg **11u** (based on dry weight of **3g**) resulting in a hydrogel loaded with 7.6 mg IL-1RA.

Example **13x** was prepared according to the procedure described in Example **13** using 11.7 mg IL-1RA and 5 mg **11v** (based on dry weight of **3g**) resulting in a hydrogel loaded with 7.9 mg IL-1RA.

Example **13y** was prepared according to the procedure described in Example **13** using 27.9 mg IL-1RA and 15 mg **11w** (based on dry weight of **3h**) resulting in a hydrogel loaded with 20.9 mg IL-1RA.

Example **13z** was prepared according to the procedure described in Example **13** using 27.9 mg IL-1RA and 15 mg **11x** (based on dry weight of **3h**) resulting in a hydrogel loaded with 20.0 mg IL-1RA.

Example **13aa** was prepared according to the procedure described in Example **13** using 27.9 mg IL-1RA and 15 mg **11y** (based on dry weight of **3h**) resulting in a hydrogel loaded with 19.4 mg IL-1RA.

Example **13bb** was prepared according to the procedure described in Example **13** using 27.9 mg IL-1RA and 15 mg **11z** (based on dry weight of **3h**) resulting in a hydrogel loaded with 18.3 mg IL-1RA.

### Example 14

### Blocked hydrogel beads 14

Hydrogel beads were synthesized according to the procedure described in example 1 of WO 2011/012715 A1 and functionalized with maleimide groups according to the procedure described in Example 7. Afterwards, 10 mL of the hydrogel suspension at 67.4 mg/mL were transferred into a 20 mL syringe equipped with a frit. The solvent was expelled and the hydrogel washed 5 times with 10 mL 10 mM histidine/10 wt% α,α-trehalose/0.01% Tween20/pH 5.5. The solvent was expelled and 10 mL 100 mM β-mercaptoethanol in 10 mM histidine/10 wt% α,α-trehalose/0.01% Tween20/pH 5.5 were drawn into the syringe. The resulting suspension was allowed to incubate at ambient temperature under gentle shaking for 1 hour. The solvent was discarded and the hydrogel was washed 10 times with each time 10 mL PBS-T/pH 7.4, the solvent was discarded each time. Finally, fresh PBS-T/pH 7.4 was drawn into the syringe and the suspension transferred into a Falcon tube to give 14.

### Example 15

### Blocked hydrogel beads 15

Hydrogel beads were synthesized according to the procedure described in example **3h** and functionalized with maleimide groups according to the procedure described in example 7. Afterwards, 4 mL of the hydrogel suspension at 10 mg/mL were transferred into a 20 mL syringe equipped with a frit. The solvent was expelled and the hydrogel washed 10 times with 5 mL 10 mM histidine/10 wt% α,α-trehalose/0.01% Tween20/pH 5.5. The solvent was expelled and 5 mL 1 mM β-mercaptoethanol in 10 mM histidine/10 wt% α,α-trehalose/0.01% Tween20/pH 5.5 were drawn into the syringe. The resulting suspension was allowed to incubate at ambient temperature under gentle shaking for 5 min. The solvent was discarded and the hydrogel treated 9 additional times with 5 mL 1 mM β-mercaptoethanol in 10 mM histidine/10 wt% α,α-trehalose/0.01% Tween20/pH 5.5. The solvent was each time discarded. The hydrogel beads were then washed 10 times with each time 5 mL 10 mM histidine/10 wt% α,α-trehalose/0.01% Tween20/pH 5.5, the solvent was discarded each time. The hydrogel beads were then washed ten times with each time 5 mL PBS-T/pH 7.4, the solvent was discarded each time. Finally, fresh PBS-T/pH 7.4 was drawn into the syringe and the suspension transferred into a Falcon tube to give 15.

### Example 16:

### Antibody binding to IL-1ra hydrogel beads

20 μL of hydrogel suspensions (35 volume-%) in PBS-T buffer were mixed with 400 μL first antibody solution

in PBS-T with 1% BSA (Sigma, A3059) and incubated for 1 h at 200 rpm in 1.5 mL Eppendorf tubes. For IL-1ra hydrogel beads a 1:50 dilution of antibody ab124962 (Anti-IL1RA antibody [EPR6483] (ab124962) - Abcam, Cambridge, UK) was used. Hydrogel beads were sedimented through a centrifugation step at 100 g for 1 min in a tabletop centrifuge. The supernatant was removed by pipetting and care was taken not to remove any hydrogel beads. Washing of the beads was accomplished via two rounds of washing steps, which included addition of 1 mL PBS-T buffer, centrifugation at 100 g for 1 min and careful removal of the supernatant by pipetting. 400 μL of the secondary antibody in PBS-T with 1% BSA (Sigma, A3059) were added to the beads and incubated for 1 h at 200 rpm. For IL-1ra hydrogel beads a 1:100 dilution of antibody sc-3750 (bovine anti-rabbit IgG-PE, Santa Cruz Biotechnology Inc., Santa Cruz, CA 95060 USA) was used. The supernatant was removed by pipetting and care was taken not to remove any hydrogel beads. Washing of the beads was accomplished via four rounds of washing steps, which included addition of 1 mL PBS-T buffer, centrifugation at 100 g for 1 min and careful removal of the supernatant by pipetting. The washed beads were resuspended in 200 μL PBS-T and transferred completely into black 96-well plates (black, non-binding, Art. no. 655900, Greiner bio-one GmbH, 72636 Frickenhausen, Germany). The fluorescence intensity was determined with a Tecan Infinite M200 fluorescence plate reader (Excitation 495 nm, Emission 575 nm, Number of flashes 25, Integration time 20 μs, Multiple reads per well 5x5 (Border 250 μm), Optimal gain).

**Data analysis**

Determination of antibody binding to PEG-modified IL-1ra hydrogel beads was achieved in comparison with a standard curve of unmodified IL-1RA hydrogel beads. Unmodified IL-1RA hydrogel beads were always prepared under identical conditions as the PEGylated hydrogel beads except for the addition of PEGylation reagent. Unmodified IL-1RA hydrogel beads were mixed with placebo hydrogel beads (Example **14** or Example **15**) in different ratios. Example **14** was used for Example **16a-16i,** Example **15** was used for Example **16j-16bb.** The plot (percentage of unmodified IL-1ra hydrogel beads versus fluorescence intensity) was fitted in a linear fashion. The percentage of antibody binding to PEGylated IL-1ra hydrogel beads was back-calculated according to the obtained calibration curve.

Example **16a** was prepared according to the procedure described in Example **16** using **13a.**

Example **16b** was prepared according to the procedure described in Example **16** using **13b.**

Example **16c** was prepared according to the procedure described in Example **16** using **13c.**

Example **16d** was prepared according to the procedure described in Example **16** using **13d.**

Example **16e** was prepared according to the procedure described in Example **16** using **13e.**

Example **16f** was prepared according to the procedure described in Example **16** using **13f.**

Example **16g** was prepared according to the procedure described in Example **16** using **13g.**

Example **16h** was prepared according to the procedure described in Example **16** using **13h.**

Example **16i** was prepared according to the procedure described in Example **16** using **13i.**

Example **16j** was prepared according to the procedure described in Example **16** using **13j.**

Example **16k** was prepared according to the procedure described in Example **16** using **13k.**

Example **16l** was prepared according to the procedure described in Example **16** using **13l**.

Example **16m** was prepared according to the procedure described in Example **16** using **13m.**

Example **16n** was prepared according to the procedure described in Example **16** using **13n.**

Example **16o** was prepared according to the procedure described in Example **16** using **13o.**

Example **16p** was prepared according to the procedure described in Example **16** using **13p.**

Example **16q** was prepared according to the procedure described in Example **16** using **13q.**

Example **16s** was prepared according to the procedure described in Example **16** using **13s.**

Example **16t** was prepared according to the procedure described in Example **16** using **13t.**

Example **16u** was prepared according to the procedure described in Example **16** using **13u.**

Example **16v** was prepared according to the procedure described in Example **16** using **13v.**

Example **16w** was prepared according to the procedure described in Example **16** using **13w.**

Example **16x** was prepared according to the procedure described in Example **16** using **13x.**

Example **16y** was prepared according to the procedure described in Example **16** using **13y.**

Example **16z** was prepared according to the procedure described in Example **16** using **13z.**

Example **16aa** was prepared according to the procedure described in Example **16** using **13aa.**

Example **16bb** was prepared according to the procedure described in Example **16** using **13bb.**

**Example 17:**

**Antibody binding to Insulin hydrogel beads**

20 μL of hydrogel suspensions (35 volume-%) in PBS-T buffer were mixed with 400 μL first antibody solution

in PBS-T with 1% BSA (Sigma, A3059) and incubated for 1 h at 200 rpm in 1.5 mL Eppendorf tubes. For Insulin hydrogel beads a 1:100 dilution of antibody ab8302 (Anti-Insulin antibody [7F8] (ab8302) - Abeam, Cambridge, UK) was used. Hydrogel beads were sedimented through a centrifugation step at 100 g for 1 min in a tabletop centrifuge. The supernatant was removed by pipetting and care was taken not to remove any hydrogel beads. Washing of the beads was accomplished via two rounds of washing steps, which included addition of 1 mL PBS-T buffer, centrifugation at 100 g for 1 min and careful removal of the supernatant by pipetting. 400 μL of the secondary antibody in PBS-T with 1% BSA (Sigma, A3059) were added to the beads and incubated for 1 h at 200 rpm. For Insulin hydrogel beads a 1:50 dilution of antibody ab97041 (Goat Anti-Mouse IgG H&L (Phycoerythrin) preadsorbed (ab97041) - Abcam, Cambridge, UK) was used. The supernatant was removed by pipetting and care was taken not to remove any hydrogel beads. Washing of the beads was accomplished via four rounds of washing steps, which included addition of 1 mL PBS-T buffer, centrifugation at 100 g for 1 min and careful removal of the supernatant by pipetting. The washed beads were resuspended in 200 μL PBS-T and transferred completely into black 96-well plates (black, non-binding, Art. no. 655900, Greiner bio-one GmbH, 72636 Frickenhausen, Germany). The fluorescence intensity was determined with a Tecan Infinite M200 fluorescence plate reader (Excitation 495 nm, Emission 575 nm, Number of flashes 25, Integration time 20 μs, Multiple reads per well 5x5 (Border 250 μm), Optimal gain).

**Data analysis**

Determination of antibody binding to PEG-modified Insulin hydrogel beads was achieved in comparison with a standard curve of unmodified Insulin hydrogel beads. Unmodified insulin hydrogel beads were always prepared under identical conditions as the PEGylated hydrogel beads except for the addition of PEGylation reagent.The unmodified Insulin hydrogel beads were mixed with Placebo hydrogel beads (Example **14**) in different ratios. The plot (percentage of unmodified Insulin hydrogel beads versus fluorescence intensity) was fitted in a linear fashion. The percentage of antibody binding to PEGylated Insulin hydrogel beads was back-calculated according to the obtained calibration curve.

Example **17a** was prepared by transferring the insulin loaded hydrogel example **12a** into a 2 mL syringe equipped with a frit. The solvent was discarded and the hydrogel was washed ten times with 1 mL of PBS-T buffer pH 7.4. The buffer was discarded each time. Fresh buffer was drawn into the syringe and the suspension transferred into an Eppendorf tube. Density of the hydrogel suspension was adjusted to 35 volume-% in PBS-T. Analysis of the sample was performed as described in Example **17.**

Example **17b** was prepared by transferring the insulin loaded hydrogel example **12b** into a 2 mL syringe equipped with a frit. The solvent was discarded and the hydrogel was washed ten times with 1 mL of PBS-T buffer pH 7.4. The buffer was discarded each time. Fresh buffer was drawn into the syringe and the suspension transferred into an Eppendorf tube. Density of the hydrogel suspension was adjusted to 35 volume-% in PBS-T. Analysis of the sample was performed as described in Example **17.**

Example **17c** was prepared by transferring the insulin loaded hydrogel example **12c** into a 2 mL syringe equipped with a frit. The solvent was discarded and the hydrogel was washed ten times with 1 mL of PBS-T buffer pH 7.4. The buffer was discarded each time. Fresh buffer was drawn into the syringe and the suspension transferred into an Eppendorf tube. Density of the hydrogel suspension was adjusted to 35 volume-% in PBS-T. Analysis of the sample was performed as described in Example **17.**

| Example No. | Hydrogel | PEGylated Hydrogel | Amine content before PEGylation | Amine content after PEGylation | Final hydrogel formulation | | Percentage of antibody binding [%] |
|---|---|---|---|---|---|---|---|
| | | | | | Test molecule | Example No. | |
| 17a | 3b | 9a | 1.020 | 0.862 | Insulin | 12a | 11 |
| 17b | 3b | 9b | 1.020 | 0.756 | Insulin | 12b | 1.3 |
| 17c | 3a | 9c | 1.003 | 0.932 | Insulin | 12c | 2.9 |
| 16a | 3c | 9d | 0.201 | 0.19 | IL-1RA | 13a | 1.6 |
| 16b | 3c | 9e | 0.201 | 0.126 | IL-1RA | 13b | 4.4 |
| 16c | 3c | 9f | 0.201 | 0.126 | IL-1RA | 13c | 0.8 |
| 16d | 3d | 9g | 0.168 | n.d. | IL-1RA | 13d | 33.5 |

(continued)

| Example No. | Hydrogel | PEGylated Hydrogel | Amine content before PEGylation | Amine content after PEGylation | Final hydrogel formulation | | Percentage of antibody binding [%] |
|---|---|---|---|---|---|---|---|
| | | | | | Test molecule | Example No. | |
| 16e | 3d | 9h | 0.168 | n.d. | IL-1RA | 13e | 2.5 |
| 16f | 3d | 9i | 0.168 | n.d. | IL-1RA | 13f | 2.7 |
| 16g | 3d | 10a | 0.168 | n.d. | IL-1RA | 13g | 17.9 |
| 16h | 3e | 8a | 0.142 | 0.076 | IL-1RA | 13h | 1.5 |
| 16i | 3e | 8b | 0.142 | 0.095 | IL-1RA | 13i | 0.7 |
| 16j | 3f | 9j | 0.152 | 0.125 | IL-1RA | 13j | 22.8 |
| 16k | 3f | 9k | 0.152 | 0.133 | IL-1RA | 13k | 9.9 |
| 161 | 3f | 91 | 0.152 | 0.141 | IL-1RA | 131 | 5.7 |
| 16m | 3f | 9m | 0.152 | 0.131 | IL-1RA | 13m | 10.4 |
| 16n | 3f | 9n | 0.152 | 0.137 | IL-1RA | 13n | 9.3 |
| 16o | 3g | 9o | 0.154 | 0.096 | IL-1RA | 13o | 35.9 |
| 16p | 3g | 9p | 0.154 | 0.098 | IL-1RA | 13p | 11.9 |
| 16q | 3g | 9q | 0.154 | 0.118 | IL-1RA | 13q | 5.4 |
| 16r | 3g | 9r | 0.145 | 0.121 | IL-1RA | 13r | 18.2 |
| 16s | 3g | 9s | 0.145 | 0.116 | IL-1RA | 13s | 13.1 |
| 16t | 3g | 9t | 0.154 | 0.114 | IL-1RA | 13t | 6.7 |
| 16u | 3g | 9u | 0.154 | 0.132 | IL-1RA | 13u | 8.7 |
| 16v | 3g | 9v | 0.154 | 0.119 | IL-1RA | 13v | 7.9 |
| 16w | 3g | 9w | 0.154 | 0.118 | IL-1RA | 13w | 4.6 |
| 16x | 3g | 10b | 0.154 | 0.136 | IL-1RA | 13x | 28.8 |
| 16y | 3h | 9x | 0.159 | 0.108 | IL-1RA | 13y | 18.1 |
| 16z | 3h | 9y | 0.159 | 0.114 | IL-1RA | 13z | 30.2 |
| 16aa | 3h | 9z | 0.159 | 0.085 | IL-1RA | 13aa | 26.1 |
| 16bb | 3h | 9aa | 0.159 | 0.125 | IL-1RA | 13bb | 39.3 |

**Abbreviations**

Boc *tert.*butyloxycarbonyl
BSA bovine serum albumine
DCC dicyclohexylcarbodiimide
DCM dichloromethane
DIPEA diisopropylethylamine
DMAP 4-dimethylaminopyridine
DMSO dimethylsulfoxide
EDTA ethylenediaminetetraacetic acid
Fmoc fluorenylmethyloxycarbonyl

| | |
|---|---|
| Lys | lysine |
| MTBE | methyl *tert*.butyl ether |
| NHS | N-hydroxysuccinimide |
| NMP | N-methyl-2-pyrrolidone |
| PBS | phosphate buffered saline |
| PBS-T | phosphate buffered saline, Tween20 |
| PEG | Polyethyleneglycol |
| PP | polypropylene |
| TSTU | O-(N-Succinimidyl)-1,1,3,3-tetramethyluronium tetrafluoroborate |

## Claims

1. A process for the preparation of a hydrogel suitable as a carrier in a hydrogel-linked prodrug comprising the steps of

(a) providing a hydrogel having groups $A^{x0}$, wherein the groups $A^{x0}$ represent the same or different functional groups;
(b) optionally covalently conjugating a spacer reagent of formula (I)

$$A^{x1}\text{-}SP^2\text{-}A^{x2} \qquad (I),$$

wherein

$SP^2$ is $C_{1-50}$ alkyl, $C_{2-50}$ alkenyl or $C_{2-50}$ alkynyl, which $C_{1-50}$ alkyl, $C_{2-50}$ alkenyl or $C_{2-50}$ alkynyl is optionally interrupted by one or more group(s) selected from the group consisting of -NH-, -N($C_{1-4}$ alkyl)-, -O-, -S-, -C(O)-, -C(O)NH-, -C(O)N($C_{1-4}$ alkyl)-, -O-C(O)-, -S(O)-, $-S(O)_2-$, 4- to 7-membered heterocyclyl, phenyl and naphthyl;
$A^{x1}$ is a functional group for reaction with $A^{x0}$ of the hydrogel; and
$A^{x2}$ is a functional group;

to $A^{x0}$ of the hydrogel from step (a); and
(c) reacting the hydrogel of step (a) or step (b) with a reagent of formula (II)

$$A^{x3}\text{-}Z \qquad (II),$$

wherein

$A^{x3}$ is a functional group; and
Z is an inert linear or branched PEG-based polymer comprising at least 70% PEG, wherein Z has a molecular weight ranging from 10 kDa to 250 kDa, and does not react with $A^{x0}$ or $A^{x2}$ or with other functional groups present;

such that at most 80 mol-% of $A^{x0}$ or $A^{x2}$ react with $A^{x3}$.

2. The process of claim 1, wherein the groups $A^{x0}$ represent the same functional groups.

3. The process of claim 1 or 2, wherein $A^{x0}$ is selected from the group consisting of maleimide, amine $-NH_2$, hydroxyl -OH, thiol, carboxyl -COOH and activated carboxyl $-COY^1$, wherein $Y^1$ is selected from formulas (f-i) to (f-vi):

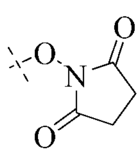 (f-i), 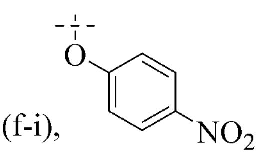 (f-ii), 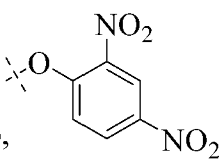 (f-iii),

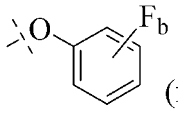 (f-iv), 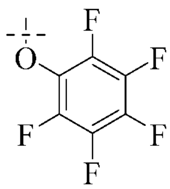 (f-v) and 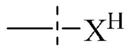 (f-vi),

wherein
the dashed lines indicate attachment to the rest of the molecule,

b is 1, 2, 3 or 4; and
$X^H$ is Cl, Br, I, or F.

**4.** The process of any one of claims 1 to 3, wherein the hydrogel of step (a) is obtainable by a process comprising the steps of:

(a-i) providing a mixture comprising

(a-ia) at least one backbone reagent, wherein the at least one backbone reagent has a molecular weight ranging from 1 to 100 kDa, and comprises at least three functional groups $A^{x0}$, wherein each $A^{x0}$ is a maleimide, amine, $-NH_2$, hydroxyl -OH, carboxyl -COOH or activated carboxyl $-COY^1$, wherein $Y^1$ is selected from formulas (f-i) to (f-vi):

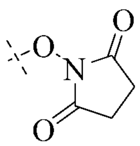 (f-i), 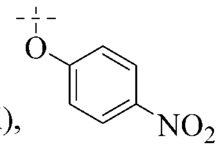 (f-ii), 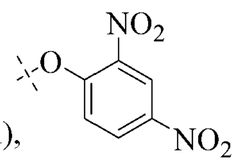 (f-iii),

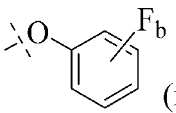 (f-iv), 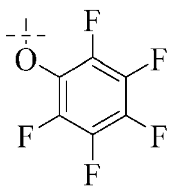 (f-v) and 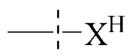 (f-vi),

wherein
the dashed lines indicate attachment to the rest of the molecule,

b is 1, 2, 3 or 4, and
$X^H$ is Cl, Br, I, or F;

(a-ib) at least one crosslinker reagent, wherein the at least one crosslinker reagent has a molecular weight ranging from 0.2 to 40 kDa and comprises at least two functional end groups selected from the group consisting of activated ester groups, activated carbamate groups, activated carbonate groups, activated thiocarbonate groups, amine groups and thiol groups;

in a weight ratio of the at least one backbone reagent to the at least one crosslinker reagent ranging from 1:99 to 99:1 and wherein the molar ratio of $A^{x0}$ to functional end groups is >1;
(a-ii) polymerizing the mixture of step (a-i) to a hydrogel; and
(a-iii) optionally working-up the hydrogel of step (a-ii).

**5.** The process of any one of claims 1 to 4, wherein $A^{x0}$ is an amine and $A^{x1}$ is $ClSO_2$-, $R^1$(C=O)-, I-, Br-, Cl-, SCN-, CN-, O=C=N-, $Y^1$-(C=O)-, $Y^1$-(C=O)-NH-, or $Y^1$-(C=O)-O-,
wherein

$R^1$ is H, $C_{1-6}$ alkyl, $C_{2-6}$ alkenyl, $C_{2-6}$ alkynyl, $C_{3-8}$ cycloalkyl, 4- to 7-membered heterocyclyl, 8- to 11-membered heterobicyclyl, phenyl, naphthyl, indenyl, indanyl, or tetralinyl; and
$Y^1$ is selected from formulas (f-i) to (f-vi):

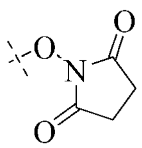 (f-i), 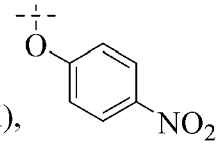 (f-ii), 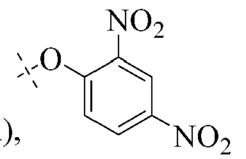 (f-iii),

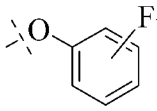 (f-iv), 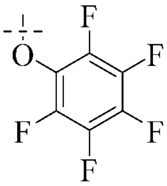 (f-v) and 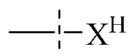 (f-vi),

wherein
the dashed lines indicate attachment to the rest of the molecule,

b is 1, 2, 3 or 4, and
$X^H$ is Cl, Br, I, or F.

**6.** The process of any one of claims 1 to 5, wherein $A^{x2}$ is selected from the group consisting of -maleimide, -SH, $-NH_2$, -SeH, $-N_3$, -C=CH, $-CR^1=CR^{1a}R^{1b}$, -OH, $-(CH=X)-R^1$, $-(C=O)-S-R^1$, -(C=O)-H, $-NH-NH_2$, $-O-NH_2$, -Ar-X°, -Ar-$Sn(R^1)(R^{1a})(R^{1b})$, -Ar-B(OH)(OH), -Br, -I, $Y^1$-(C=O)-, $Y^1$-(C=O)-NH-, $Y^1$-(C=O)-O-,

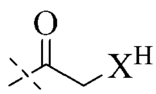 , 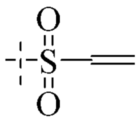 , 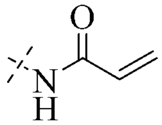 , 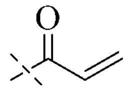 ,

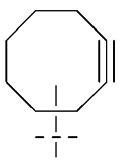 , 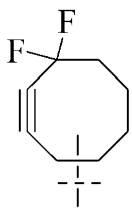 , 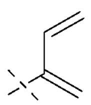 , 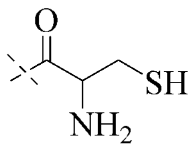 ,

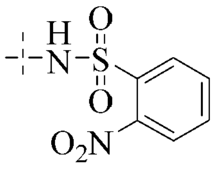 , and 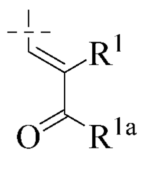 ;

with optional protecting groups; wherein
dashed lines indicate attachment to $SP^2$;

X is O, S, or NH,
$X^0$ is -OH, $-NR^1R^{1a}$, -SH, or -SeH,
$X^H$ is Cl, Br, I or F;

Ar is phenyl, naphthyl, indenyl, indanyl, or tetralinyl;
$R^1$, $R^{1a}$, $R^{1b}$ are independently of each other H, $C_{1-6}$ alkyl, $C_{2-6}$ alkenyl, $C_{2-6}$ alkynyl, $C_{3-8}$ cycloalkyl, 4- to 7-membered heterocyclyl, 8- to 11-membered heterobicyclyl, phenyl, naphthyl, indenyl, indanyl, or tetralinyl; and
$Y^1$ is selected from formulas (f-i) to (f-vi):

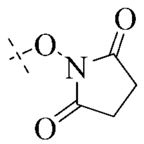 (f-i), 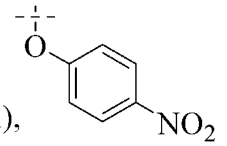 (f-ii), 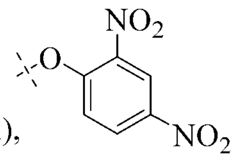 (f-iii),

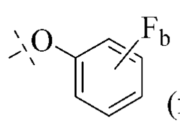 (f-iv), 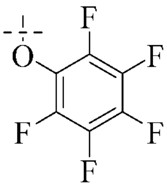 (f-v) and 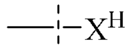 (f-vi),

wherein
the dashed lines indicate attachment to the rest of the molecule,

b is 1, 2, 3 or 4, and
$X^H$ is Cl, Br, I, or F.

**7.** The process of any one of claims 1 to 6, wherein $A^{x3}$ is selected from the group consisting of -SH, $-NH_2$, -SeH, -maleimide, -C=CH, $-N_3$, $-CR^1=CR^{1a}R^{1b}$, $-(C=X)-R^1$, -OH, $-(C=O)-S-R^1$, $-NH-NH_2$, $-O-NH_2$, $-Ar-Sn(R^1)(R^{1a})(R^{1b})$, -Ar-B(OH)(OH), $-Ar-X^0$,

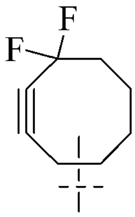, 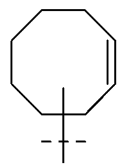, 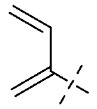, 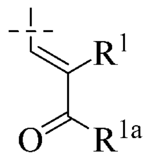, 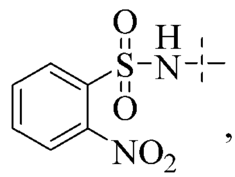,

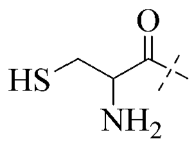, 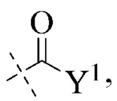, 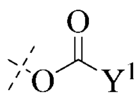, 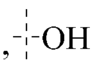, 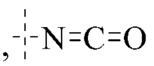 and 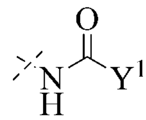;

wherein
dashed lines indicate attachment to Z;

X is O, S, or NH,
$X^0$ is -OH, $-NR^1R^{1a}$, -SH, or -SeH;
$R^1$, $R^{1a}$, $R^{1b}$ are independently of each other H, $C_{1-6}$ alkyl, $C_{2-6}$ alkenyl, $C_{2-6}$ alkynyl, $C_{3-8}$ cycloalkyl, 4- to 7-membered heterocyclyl, 8- to 11-membered heterobicyclyl, phenyl, naphthyl, indenyl, indanyl, or tetralinyl;
Ar is phenyl, naphthyl, indenyl, indanyl, or tetralinyl;
$Y^1$ is an activated carboxylic acid, activated carbonate or activated carbamate, preferably $Y^1$ is selected from formulas (f-i) to (f-vi):

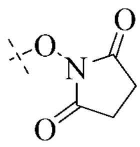 (f-i), 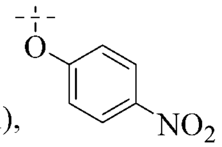 (f-ii), 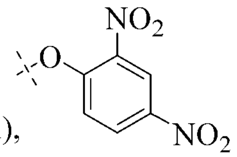 (f-iii),

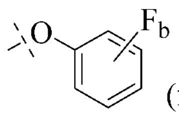 (f-iv), 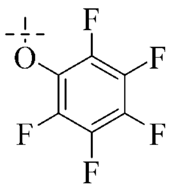 (f-v) and 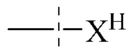 (f-vi),

wherein
the dashed lines indicate attachment to the rest of the molecule,

b is 1, 2, 3 or 4, and
$X^H$ is Cl, Br, I, or F.

**8.** The process of any one of claims 1, 2, 3, 4 or 7, wherein the optional step (b) is omitted, $A^{x0}$ is an amine and $A^{x3}$ is $Y^1$-(C=O)-.

**9.** The process of any one of claims 1 to 8, wherein Z has a molecular weight of 40 kDa.

**10.** The process of any one of claims 1 to 9, wherein Z is an inert linear or branched PEG-based polymer comprising at least 80% PEG.

**11.** The process of any one of claims 1 to 10, wherein in step (c) the reaction rate is monitored and the reaction is interrupted when at most 0.8 chemical equivalents relative to $A^{x0}$ or $A^{x2}$ have reacted.

**12.** The process of any one of claims 1 to 10, wherein no more than 20 mol-% of $A^{x0}$ or $A^{x2}$ react with $A^{x3}$.

**13.** A hydrogel obtainable from the process of any one of claims 1 to 12.

**14.** Use of the hydrogel of claim 13 as a carrier in a hydrogel-linked prodrug.

**15.** A hydrogel-linked prodrug comprising the covalently conjugated hydrogel of claim 13.

**Patentansprüche**

**1.** Ein Verfahren für die Herstellung eines Hydrogels, geeignet als Träger in einem Hydrogel-verbundenen Prodrug, umfassend die Schritte

(a) Bereitstellen eines Hydrogels mit Gruppen $A^{x0}$, wobei die Gruppen $A^{x0}$ die gleichen oder unterschiedliche funktionelle Gruppe darstellen;
(b) optional kovalente Konjugation eines Abstandshalter-Reagenz der Formel (I)

$$A^{x1}\text{-}SP^2\text{-}A^{x2} \qquad (I),$$

wobei

$SP^2$ $C_{1-50}$-Alkyl, $C_{2-50}$-Alkenyl oder $C_{2-50}$-Alkinyl ist, welches $C_{1-50}$-Alkyl, $C_{2-50}$-Alkenyl oder $C_{2-50}$-Alkinyl optional unterbrochen ist durch eine oder mehrere Gruppe(n) ausgewählt aus der Gruppe bestehend aus -NH-, -N($C_{1-4}$-Alkyl)-, -O-, -S-, -C(O)-, -C(O)NH-, -C(O)N($C_{1-4}$-Alkyl)-, -O-C(O)-, -S(O)-, -S(O)$_2$-, 4- bis 7-gliedrigem Heterocyclyl, Phenyl und Naphthyl;
$A^{x1}$ eine funktionelle Gruppe zur Reaktion mit $A^{x0}$ des Hydrogels ist; und

$A^{x2}$ eine funktionelle Gruppe ist;

an $A^{x0}$ des Hydrogels aus Schritt (a); und

(c) Reagieren des Hydrogels aus Schritt (a) oder Schritt (b) mit einem Reagenz der Formel (II)

$$A^{x3}\text{-}Z \qquad \text{(II)},$$

wobei

$A^{x3}$ eine funktionelle Gruppe ist; und

Z ein inertes lineares oder verzweigtes PEG-basiertes Polymer enthaltend mindestens 70% PEG ist, wobei Z ein Molekulargewicht im Bereich von 10 kDa bis 250 kDa hat, und nicht mit $A^{x0}$ oder $A^{x2}$ oder mit anderen anwesenden funktionellen Gruppen reagiert;

so dass maximal 80 mol-% von $A^{x0}$ oder $A^{x2}$ mit $A^{x3}$ reagieren.

**2.** Das Verfahren nach Anspruch 1, wobei die Gruppen $A^{x0}$ die gleichen funktionellen Gruppen darstellen.

**3.** Das Verfahren nach Anspruch 1 oder 2, wobei $A^{x0}$ ausgewählt ist aus der Gruppe bestehend aus Maleimid, Amin $-NH_2$, Hydroxyl -OH, Thiol, Carboxyl -COOH und aktiviertem Carboxyl $-COY^1$, wobei $Y^1$ ausgewählt ist aus den Formeln (f-i) bis (f-vi):

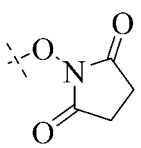 (f-i), 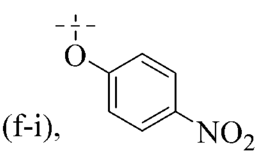 (f-ii), 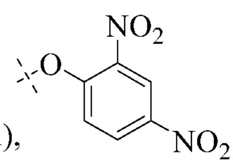 (f-iii),

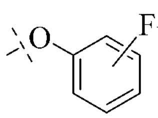 (f-iv), 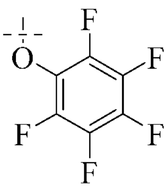 (f-v) und 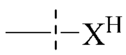 (f-vi),

wobei

die gestrichelten Linien Anknüpfung an den Rest des Moleküls anzeigen,

b 1, 2, 3 oder 4 ist; und

$X^H$ Cl, Br, I oder F ist.

**4.** Das Verfahren nach einem der Ansprüche 1 bis 3, wobei das Hydrogel aus Schritt (a) erhältlich ist durch ein Verfahren enthaltend die Schritte:

(a-i) Bereitstellen einer Mischung enthaltend

(a-ia) mindestens ein Hauptkettenreagenz, wobei das mindestens eine Hauptkettenreagenz ein Molekulargewicht im Bereich von 1 bis 100 kDa hat und mindestens drei funktionelle Gruppen $A^{x0}$ umfasst, wobei jedes $A^{x0}$ ein Maleimid, Amin $-NH_2$, Hydroxyl -OH, Carboxyl -COOH oder aktiviertes Carboxyl $-COY^1$ ist, wobei $Y^1$ ausgewählt ist aus den Formeln (f-i) bis (f-vi):

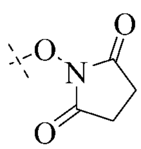 (f-i), 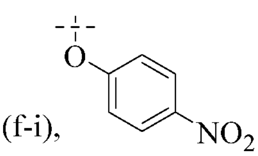 (f-ii), 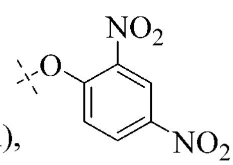 (f-iii),

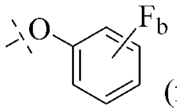 (f-iv), 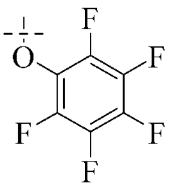 und 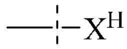 (f-vi),

wobei
die gestrichelten Linien Anknüpfung an den Rest des Moleküls anzeigen,

b 1, 2, 3 oder 4 ist, und
$X^H$ Cl, Br, I oder F ist;

(a-ib) mindestens ein Quervernetzungsreagenz, wobei das mindestens eine Quervernetzungsreagenz ein Molekulargewicht im Bereich von 0,2 bis 40 kDa hat und mindestens zwei funktionale Endgruppen umfasst ausgewählt aus der Gruppe bestehend aus aktivierten Estergruppen, aktivierten Carbamatgruppen, aktivierten Carbonatgruppen, aktivierten Thiocarbonatgruppen, Amingruppen und Thiolgruppen;

in einem Gewichtsverhältnis des mindestens einen Hauptkettenreagenzes zu dem mindestens einem Quervernetzungsreagenz im Bereich von 1:99 bis 99:1, und wobei das molekulare Verhältnis von $A^{x0}$ zu den funktionellen Endgruppen >1 ist;
(a-ii) Polymerisieren der Mischung aus Schritt (a-i) zu einem Hydrogel; und
(a-iii) optional Aufarbeiten des Hydrogels aus Schritt (a-ii).

**5.** Das Verfahren nach einem der Ansprüche 1 bis 4, wobei $A^{x0}$ ein Amin ist und $A^{x1}$ $ClSO_2$-, $R^1$(C=O)-, I-, Br-, Cl-, SCN-, CN-, O=C=N-, $Y^1$-(C=O)-, $Y^1$-(C=O)-NH- oder $Y^1$-(C=O)-O- ist,
wobei

$R^1$ H, $C_{1-6}$-Alkyl, $C_{2-6}$-Alkenyl, $C_{2-6}$-Alkinyl, $C_{3-8}$-Cycloalkyl, 4- bis 7-gliedriges Heterocyclyl, 8- bis 11-gliedriges Heterobicyclyl, Phenyl, Naphthyl, Indenyl, Indanyl oder Tetralinyl ist; und
$Y^1$ ausgewählt ist aus den Formeln (f-i) bis (f-vi):

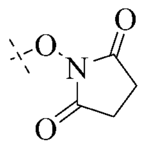 (f-i), 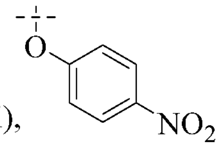 (f-ii), 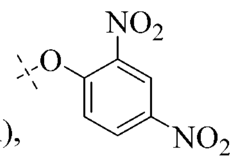 (f-iii),

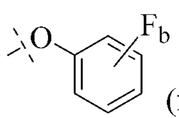 (f-iv), 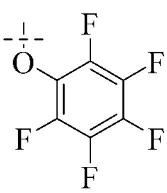 (f-v) und 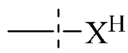 (f-vi),

wobei
die gestrichelten Linien Anknüpfung an den Rest des Moleküls anzeigen,

b 1, 2, 3 oder 4 ist, und
$X^H$ Cl, Br, I oder F ist.

**6.** Das Verfahren nach einem der Ansprüche 1 bis 5, wobei $A^{x2}$ ausgewählt ist aus der Gruppe bestehend aus -Maleimid, -SH, -$NH_2$, -SeH, -$N_3$, -C≡CH, -$CR^1$=$CR^{1a}R^{1b}$, -OH, -(CH=X)-$R^1$, -(C=O)-S-$R^1$, -(C=O)-H, -NH-$NH_2$, -O-$NH_2$, -Ar-$X^0$, -Ar-Sn($R^1$)($R^{1a}$)($R^{1b}$), -Ar-B(OH)(OH), -Br, -I, $Y^1$-(C=O)-, $Y^1$-(C=O)-NH-, $Y^1$-(C=O)-O-,

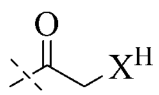, 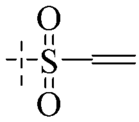, 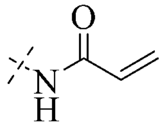, 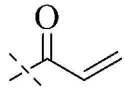,

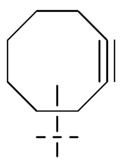, 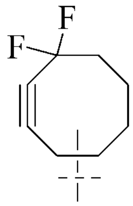, 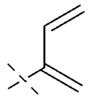, 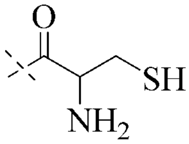,

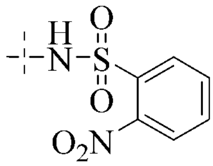 und 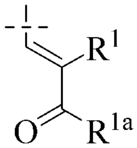 ;

mit optionalen Schutzgruppen;
wobei
gestrichelte Linien Anknüpfung an $SP^2$ anzeigen;

X O, S oder NH ist,
$X^0$ -OH, $-NR^1R^{1a}$, -SH oder -SeH ist,
$X^H$ Cl, Br, I oder F ist;
Ar Phenyl, Naphthyl, Indenyl, Indanyl oder Tetralinyl ist;
$R^1$, $R^{1a}$, $R^{1b}$ unabhängig voneinander H, $C_{1-6}$-Alkyl, $C_{2-6}$-Alkenyl, $C_{2-6}$-Alkinyl, $C_{3-8}$-Cycloalkyl, 4- bis 7-gliedriges Heterocyclyl, 8- bis 11-gliedriges Heterobicyclyl, Phenyl, Naphthyl, Indenyl, Indanyl oder Tetralinyl sind; und
$Y^1$ ausgewählt ist aus den Formeln (f-i) to (f-vi):

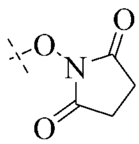 (f-i), 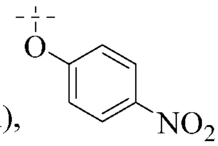 (f-ii), 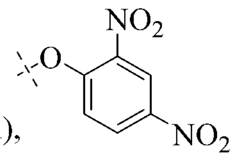 (f-iii),

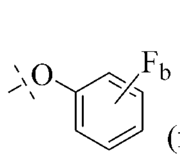 (f-iv), 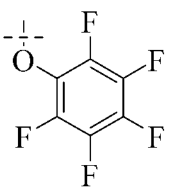 (f-v) und 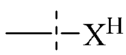 (f-vi),

wobei
die gestrichelten Linien Anknüpfung an den Rest des Moleküls anzeigen,

b 1, 2, 3 oder 4 ist, und
$X^H$ Cl, Br, I oder F ist.

**7.** Das Verfahren nach einem der Ansprüche 1 bis 6, wobei $A^{x3}$ ausgewählt ist aus der Gruppe bestehend aus -SH, $-NH_2$, -SeH, -Maleimide, -C=CH, $-N_3$, $-CR^1=CR^{1a}R^{1b}$, $-(C=X)-R^1$, -OH, $-(C=O)-S-R^1$, $-NH-NH_2$, $-O-NH_2$, -Ar-$Sn(R^1)(R^{1a})(R^{1b})$, -Ar-B(OH)(OH), -Ar-$X^0$,

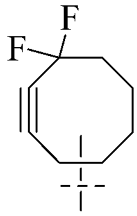, 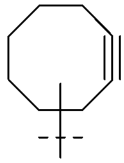, 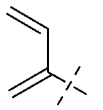, 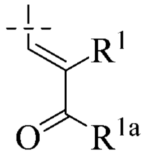, 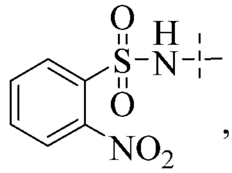,

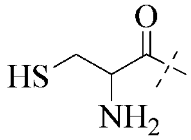, 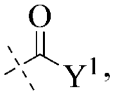, 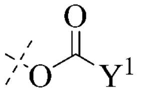, 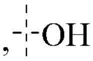, 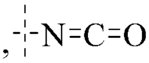 und 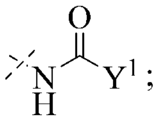;

wobei
gestrichelte Linien Anknüpfung an Z zeigen;

X O, S oder NH ist,
$X^0$ -OH, $-NR^1R^{1a}$, -SH oder -SeH ist;
$R^1$, $R^{1a}$, $R^{1b}$ unabhängig voneinander H, $C_{1-6}$-Alkyl, $C_{2-6}$-Alkenyl, $C_{2-6}$-Alkinyl, $C_{3-8}$-Cycloalkyl, 4- bis 7-gliedriges Heterocyclyl, 8- bis 11-gliedriges Heterobicyclyl, Phenyl, Naphthyl, Indenyl, Indanyl oder Tetralinyl sind;
Ar Phenyl, Naphthyl, Indenyl, Indanyl oder Tetralinyl ist;
$Y^1$ eine aktivierte Carbonsäure, aktiviertes Carbonat oder aktiviertes Carbamat ist, bevorzugt ist $Y^1$ ausgewählt aus den Formeln (f-i) bis (f-vi):

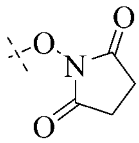 (f-i), 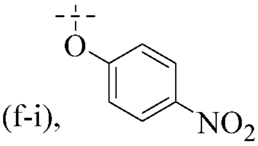 (f-ii), 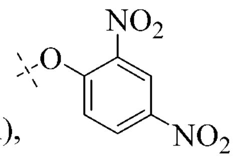 (f-iii),

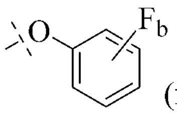 (f-iv), 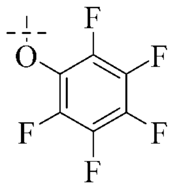 (f-v) und 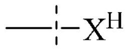 (f-vi),

wobei
die gestrichelten Linien Anknüpfung an den Rest des Moleküls anzeigen,

b 1, 2, 3 oder 4 ist, und
$X^H$ Cl, Br, I oder F ist.

**8.** Das Verfahren nach einem der Ansprüche 1, 2, 3, 4 oder 7, wobei der optionale Schritt (b) ausgelassen wird, $A^{x0}$ ein Amin ist und $A^{x3}$ $Y^1$-(C=O)- ist.

**9.** Das Verfahren nach einem der Ansprüche 1 bis 8, wobei Z ein Molekulargewicht von 40 kDa hat.

**10.** Das Verfahren nach einem der Ansprüche 1 bis 9, wobei Z ein inertes lineares oder verzweigtes PEG-basiertes Polymer enthaltend mindestens 80% PEG ist.

**11.** Das Verfahren nach einem der Ansprüche 1 bis 10, wobei in Schritt (c) die Reaktionsgeschwindigkeit überwacht wird und die Reaktion unterbrochen wird, wenn maximal 0.8 chemische Äquivalente in Bezug auf $A^{x0}$ oder $A^{x2}$ reagiert haben.

**12.** Das Verfahren nach einem der Ansprüche 1 bis 10, wobei nicht mehr als 20 mol-% von $A^{x0}$ oder $A^{x2}$ mit $A^{x3}$ reagieren.

**13.** Ein Hydrogel erhältlich durch das Verfahren nach einem der Ansprüche 1 bis 12.

**14.** Verwendung des Hydrogels nach Anspruch 13 als Träger in einem Träger-verbundenen Prodrug.

**15.** Ein Träger-verbundenes Prodrug enthaltend das kovalent konjugiertes Hydrogel nach Anspruch 13.

**Revendications**

**1.** Procédé pour la préparation d'un hydrogel approprié comme support dans un promédicament lié à un hydrogel comprenant les étapes de

(a) mise à disposition d'un hydrogel possédant des groupes $A^{x0}$, les groupes $A^{x0}$ représentant les mêmes groupes fonctionnels ou des groupes fonctionnels différents ;
(b) éventuellement conjugaison de manière covalente d'un réactif espaceur de formule (I)

$$A^{x1}\text{-}SP^2\text{-}A^{x2} \quad (I),$$

$SP^2$ étant $C_{1-50}$ alkyle, $C_{2-50}$ alcényle ou $C_{2-50}$ alcynyle, lequel $C_{1-50}$ alkyle, $C_{2-50}$ alcényle ou $C_{2-50}$ alcynyle étant éventuellement interrompu par un ou plusieurs groupes choisis dans le groupe constitué par -NH-, -N($C_{1-4}$ alkyle)-, - O-, -S-, -C(O)-, -C(O)NH-, -C(O)N($C_{1-4}$ alkyle)-, -O-C(O)-, -S(O)-, $-S(O)_2-$, hétérocyclyle à 4 à 7 chaînons, phényle et naphtyle ;
$A^{x1}$ étant un groupe fonctionnel pour une réaction avec $A^{x0}$ de l'hydrogel ; et
$A^{x2}$ étant un groupe fonctionnel ;

à $A^{x0}$ de l'hydrogel de l'étape (a) ; et
(c) mise en réaction de l'hydrogel de l'étape (a) ou de l'étape (b) avec un réactif de formule (II)

$$A^{x3}\text{-}Z \quad (II),$$

$A^{x3}$ étant un groupe fonctionnel ; et
Z étant un polymère à base de PEG linéaire ou ramifié inerte comprenant au moins 70 % de PEG, Z possédant un poids moléculaire dans la plage de 10 kDa à 250 kDa, et ne réagissant pas avec $A^{x0}$ ou $A^{x2}$ ou avec d'autres groupes fonctionnels présents ;

de sorte qu'au plus de 80 % en moles de $A^{x0}$ ou $A^{x2}$ réagisse avec $A^{x3}$.

**2.** Procédé selon la revendication 1, les groupes $A^{x0}$ représentant les mêmes groupes fonctionnels.

**3.** Procédé selon la revendication 1 ou 2, $A^{x0}$ étant choisi dans le groupe constitué par maléimide, amine - $NH_2$, hydroxyle -OH, thiol, carboxyle -COOH et carboxyle activé $-COY^1$, $Y^1$ étant choisi parmi les formules (f-i) à (f-vi) :

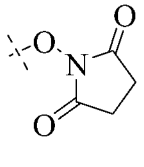 (f-i), 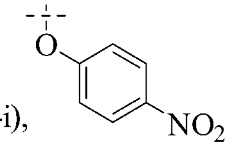 (f-ii), 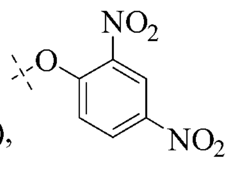 (f-iii),

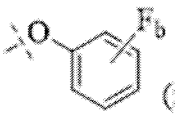 (f-iv), 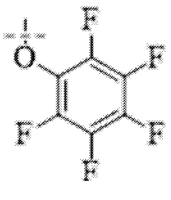 (f-v) et 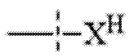 (f-vi),

les lignes en pointillés indiquant la fixation au reste de la molécule,

b étant 1, 2, 3 ou 4 ; et
$X^H$ étant Cl, Br, I, ou F.

**4.** Procédé selon l'une quelconque des revendications 1 à 3, l'hydrogel de l'étape (a) pouvant être obtenu par un procédé comprenant les étapes de :

(a-i) mise à disposition d'un mélange comprenant

(a-ia) au moins un réactif de squelette, l'au moins un réactif de squelette possédant un poids moléculaire dans la plage de 1 à 100 kDa, et comprenant au moins trois groupes fonctionnels $A^{x0}$, chaque $A^{x0}$ étant un(e) maléimide, amine, $-NH_2$, hydroxyle -OH, carboxyle -COOH ou carboxyle activé $-COY^1$, $Y^1$ étant choisi parmi les formules (f-i) à (f-vi) :

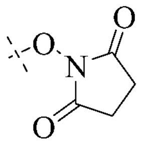 (f-i), 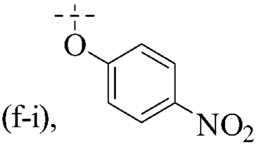 (f-ii), 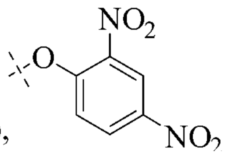 (f-iii),

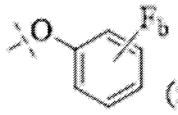 (f-iv), 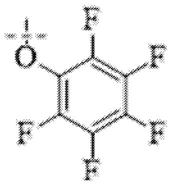 (f-v) et 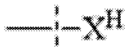 (f-vi),

les lignes en pointillés indiquant la fixation au reste de la molécule,

b étant 1, 2, 3 ou 4, et
$X^H$ étant Cl, Br, I, ou F ;

(a-ib) au moins un réactif d'agent de réticulation, l'au moins un réactif d'agent de réticulation possédant un poids moléculaire dans la plage de 0,2 à 40 kDa et comprenant au moins deux groupes terminaux fonctionnels choisis dans le groupe constitué par des groupes ester activés, des groupes carbamate activés, des groupes carbonate activés, des groupes thiocarbonate activés ; des groupes amine et des groupes thiol ;

en un rapport en poids de l'au moins un réactif de squelette sur l'au moins un réactif d'agent de réticulation dans la plage de 1 : 99 à 99 : 1 et le rapport molaire de $A^{x0}$ sur les groupes terminaux fonctionnels étant > 1 ;
(a-ii) polymérisation du mélange de l'étape (a-i) pour donner un hydrogel ; et
(a-iii) éventuellement traitement de l'hydrogel de l'étape (a-ii).

**5.** Procédé selon l'une quelconque des revendications 1 à 4, $A^{x0}$ étant une amine et $A^{x1}$ étant $ClSO_2$-, $R^1$(C=O)-, I-, Br-, Cl-, SCN-, CN-, O=C=N-, $Y^1$-(C=O)-, $Y^1$-(C=O)-NH-, ou $Y^1$- (C=O) -O-,

$R^1$ étant H, $C_{1-6}$ alkyle, $C_{2-6}$ alcényle, $C_{2-6}$ alcynyle, $C_{3-8}$ cycloalkyle, hétérocyclyle à 4 à 7 chaînons, hétérobicyclyle à 8 à 11 chaînons, phényle, naphtyle, indényle, indanyle, ou tétralinyle ; et
$Y^1$ étant choisi parmi les formules (f-i) à (f-vi) :

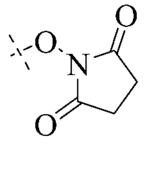 (f-i), 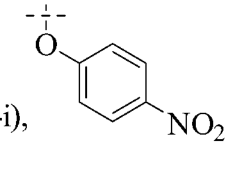 (f-ii), 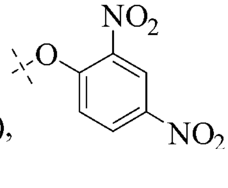 (f-iii),

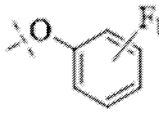 (f-iv), 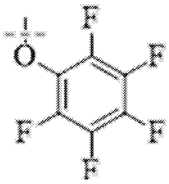 (f-v) et 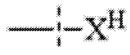 (f-vi),

les lignes en pointillés indiquant la fixation au reste de la molécule,

b étant 1, 2, 3 ou 4, et
$X^H$ étant Cl, Br, I, ou F.

**6.** Procédé selon l'une quelconque des revendications 1 à 5, $A^{x2}$ étant choisi dans le groupe constitué par -maléimide, -SH, $-NH_2$, -SeH, $-N_3$, -C≡CH, $-CR^1=CR^{1a}R^{1b}$, -OH, $-(CH=X)-R^1$, $-(C=O)-S-R^1$, -(C=O)-H, $-NH-NH_2$, $-O-NH_2$, $-Ar-X^0$, $-Ar-Sn(R^1)(R^{1a})(R^{1b})$, -Ar-B(OH) (OH), -Br, -I, $Y^1$-(C=O)-, $Y^1$-(C=O) -NH-, $Y^1$-(C=O)-O-,

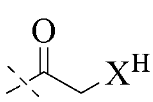, 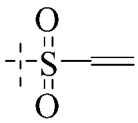, 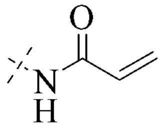, 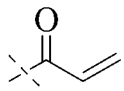,

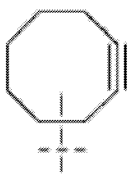, 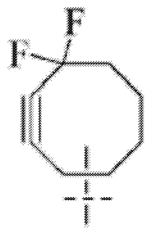, 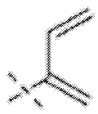, 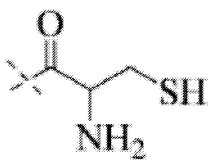,

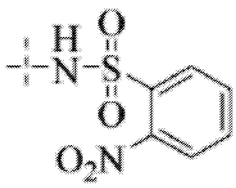, et 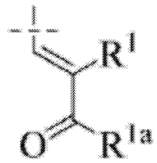 ;

avec des groupes protecteurs éventuels ;

les lignes en pointillés indiquant la fixation à $SP^2$;

X étant O, S, ou NH,
$X^0$ étant -OH, $-NR^1R^{1a}$, -SH, ou -SeH,
$X^H$ étant Cl, Br, I ou F ;

Ar étant phényle, naphtyle, indényle, indanyle, ou tétralinyle ;
$R^1$, $R^{1a}$, $R^{1b}$ étant indépendamment les uns des autres H, $C_{1-6}$ alkyle, $C_{2-6}$ alcényle, $C_{2-6}$ alcynyle, $C_{3-8}$ cycloalkyle, hétérocyclyle à 4 à 7 chaînons, hétérobicyclyle à 8 à 11 chaînons, phényle, naphtyle, indényle, indanyle, ou tétralinyle ; et
$Y^1$ étant choisi parmi les formules (f-i) à (f-vi) :

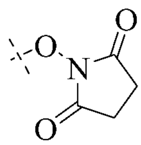 (f-i), 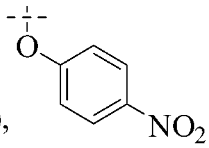 (f-ii), 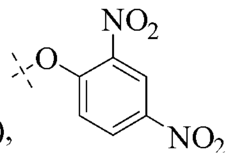 (f-iii),

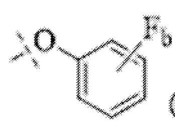 (f-iv), 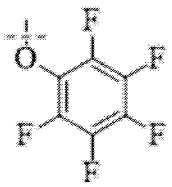 (f-v) et 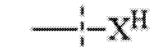 (f-vi),

les lignes en pointillés indiquant la fixation au reste de la molécule,

b étant 1, 2, 3 ou 4, et
$X^H$ étant Cl, Br, I, ou F.

**7.** Procédé selon l'une quelconque des revendications 1 à 6, $A^{x3}$ étant choisi dans le groupe constitué par -SH, $-NH_2$, -SeH, -maléimide, -C≡CH, $-N_3$, $-CR^1=CR^{1a}R^{1b}$, - (C=X) $-R^1$, -OH, -(C=O)-S-$R^1$, $-NH-NH_2$, $-O-NH_2$, -Ar-Sn($R^1$)($R^{1a}$)($R^{1b}$), -Ar-B(OH) (OH), -Ar-$X^0$,

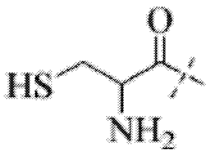, 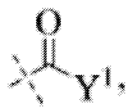, 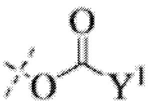, 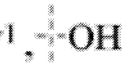, 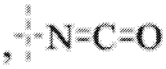 et 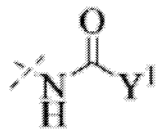;

les lignes en pointillés indiquant la fixation à 2 ;

X étant O, S, ou NH,
$X^0$ étant -OH, $-NR^1R^{1a}$, -SH, ou -SeH;

$R^1$, $R^{1a}$, $R^{1b}$ étant indépendamment les uns des autres H, $C_{1-6}$ alkyle, $C_{2-6}$ alcényle, $C_{2-6}$ alcynyle, $C_{3-8}$ cycloalkyle, hétérocyclyle à 4 à 7 chaînons, hétérobicyclyle à 8 à 11 chaînons, phényle, naphtyle, indényle, indanyle, ou tétralinyle ;
Ar étant phényle, naphtyle, indényle, indanyle, ou tétralinyle ;
$Y^1$ étant un acide carboxylique activé, un carbonate activé ou un carbamate activé, préférablement $Y^1$ étant choisi parmi les formules (f-i) à (f-vi) :

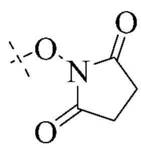 (f-i), 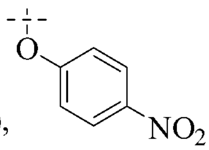 (f-ii), 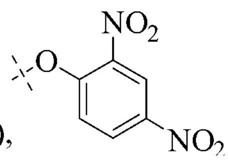 (f-iii),

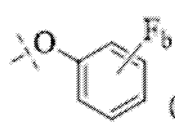 (f-iv), 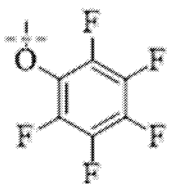 (f-v) et 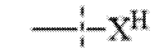 (f-vi),

les lignes en pointillés indiquant la fixation au reste de la molécule,

b étant 1, 2, 3 ou 4, et

$X^H$ étant Cl, Br, I, ou F.

**8.** Procédé selon l'une quelconque des revendications 1, 2, 3, 4 ou 7, l'étape éventuelle (b) étant omise, $A^{x0}$ étant une amine et $A^{x3}$ étant $Y^1$-(C=O)-.

**9.** Procédé selon l'une quelconque des revendications 1 à 8, Z possédant un poids moléculaire de 40 kDa.

**10.** Procédé selon l'une quelconque des revendications 1 à 9, Z étant un polymère à base de PEG linéaire ou ramifié inerte comprenant au moins 80 % de PEG.

**11.** Procédé selon l'une quelconque des revendications 1 à 10, dans lequel dans l'étape (c) la vitesse de réaction est surveillée et la réaction est interrompue lorsqu'au plus 0,8 équivalent chimique par rapport à $A^{x0}$ ou $A^{x2}$ a réagi.

**12.** Procédé selon l'une quelconque des revendications 1 à 10, pas plus de 20 % en moles de $A^{x0}$ ou $A^{x2}$ ne réagissant avec $A^{x3}$.

**13.** Hydrogel pouvant être obtenu à partir d'un procédé selon l'une quelconque des revendications 1 à 12.

**14.** Utilisation de l'hydrogel selon la revendication 13 en tant que support dans un promédicament lié à un hydrogel.

**15.** Promédicament lié à un hydrogel comprenant l'hydrogel selon la revendication 13 conjugué de manière covalente.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2006003014 A2
- WO 2011012715 A1
- WO 2011012719 A


### Non-patent literature cited in the description


- **GUDE et al.** *Letters in Peptide Science,* 2002, vol. 9 (4), 203-206
- **GUDE, M. ; J. RYF et al.** *Letters in Peptide Science,* 2002, vol. 9 (4), 203-206